# EUROPÄISCHE PATENTSCHRIFT

(11) **EP 2 633 320 B1**
(45) Veröffentlichungstag und Bekanntmachung des Hinweises auf die Patenterteilung: **02.05.2018**
(21) Anmeldenummer: 11757569.6
(22) Anmeldetag: 26.08.2011
(51) Int. Cl.: G01N 33/543

(54) **VERFAHREN ZUR BESTIMMUNG EINES ANALYTGEHALTS EINER FLÜSSIGKEITSPROBE MITTELS EINES BIOANALYSATORS**
METHOD FOR DETERMINING AN ANALYTE CONTENT OF A LIQUID SAMPLE BY MEANS OF A BIOANALYZER
PROCEDURE DE DETERMINATION DU CONTENU D'UN ANALYTE DANS UN ÉCHANTILLON LIQUIDE AU MOYEN D'UN BIOANALYZER

(30) Priorität: 30.12.2010 DE 102010064392; 29.10.2010 DE 102010043153
(43) Veröffentlichungstag der Anmeldung: 04.09.2013
(73) Patentinhaber: Endress + Hauser Conducta GmbH+Co. KG, 70839 Gerlingen (DE)
(72) Erfinder: HANKO, Michael, 01324 Dresden (DE); EUBISCH, Angela, 09125 Chemnitz (DE); FIKUS, Axel, 04746 Hartha (DE)
(74) Vertreter: Andres, Angelika Maria
(86) Internationale Anmeldenummer: PCT/EP2011/064686
(87) Internationale Veröffentlichungsnummer: WO 2012/055607

(56) Entgegenhaltungen:
- EP-A1- 1 347 300
- EP-A2- 1 413 876
- WO-A1-01/79848
- WO-A1-99/58963
- WO-A1-2008/107649
- WO-A1-2009/062027
- WO-A2-2010/088219
- US-A- 4 271 140
- CHOI SEOKHEUN ET AL: "Reusable biosensors via in situ electrochemical surface regeneration in microfluidic applications.", BIOSENSORS & BIOELECTRONICS 15 OCT 2009 LNKD- PUBMED:19709871, Bd. 25, Nr. 2, 15. Oktober 2009 (2009-10-15), Seiten 527-531, XP002662020, ISSN: 1873-4235
- SEOKHEUN CHOI ET AL: "A regenerative biosensing surface in microfluidics using electrochemical desorption of short-chain self-assembled monolayer", MICROFLUIDICS AND NANOFLUIDICS, SPRINGER, BERLIN, DE, Bd. 7, Nr. 6, 10. April 2009 (2009-04-10), Seiten 819-827, XP019745301, ISSN: 1613-4990, DOI: 10.1007/S10404-009-0440-7
- CHRISTIE A. CANARIA ET AL.: "Formation and removal of alkylthiolate self-assembled monolayers on gold in aqueous solutions", LAB ON A CHIP, Bd. 6, 2006, Seiten 289-295, XP002662022,
- A.T. TÜDÖS, E.R. LUCAS-VAN-DEN BOS, E.C.A. STIGTER: "Rapid surface plasmon resonance-based inhibition assay of deoxynivalenol", JOURNAL OF AGRICULTURAL AND FOOD CHEMISTRY, Bd. 51, 2003, Seiten 5843-5848, XP002662021,
- SHANKAR BALASUBRAMANIAN; REVZIN ALEXANDER; SIMONIAN ALEKSANDR: "Electrochemical Desorption of Proteins from Gold Electrode Surface", ELECTROANALYSIS, vol. 18, 1 October 2006 (2006-10-01), pages 1885-1892, XP055388019,

## Beschreibung

Die Erfindung betrifft ein Verfahren zur Bestimmung eines Analytgehalts einer Flüssigkeitsprobe mittels eines Bioanalysators.

Biosensoren, welche unterteilt werden können in affinitätsbasierte und metabolische Biosensoren, dienen zum vorzugsweise selektiven und spezifischen Nachweis bzw. zur Konzentrationsmessung eines vorgegebenen, in einer zu untersuchenden Flüssigkeitsprobe enthaltenen Analyten. Die große Gruppe der heterogenen Biosensoren beinhaltet dabei eine Rezeptorschicht, die eine Vielzahl von auf einer Oberfläche immobilisierten Rezeptoren aufweisen kann, z.B. Antikörper, Haptene, DNS, Zellen oder Enzyme, einen Signalwandler (englischer Fachbegriff: Transducer), der ein mit der Menge des an die Rezeptorschicht gebundenen Analyten korrelierendes Messsignal ausgibt, und einer Signalverarbeitungseinrichtung zur Verstärkung und/oder weiteren Verarbeitung des vom Signalwandler ausgegebenen Messsignals.

Der Analyt kann eine nachzuweisende Substanz in einer Flüssigkeitsprobe, beispielsweise ein Protein, ein Peptid, ein Antikörper, ein Enzym oder eine sonstige in biochemischen, biologischen oder medizinischen Systemen nachzuweisende Substanz sein. Beim Analyten kann es sich jedoch auch um Zellen, Zellbestandteile oder DNS oder beispielsweise um Wirkstoffe handeln, welche im Rahmen von Umweltmonitoring oder bei der Überwachung der Wasserqualität nachgewiesen oder bestimmt werden.

Bei affinitätsbasierten Biosensoren kann es gegebenenfalls notwendig sein, dass an die Rezeptoren der Rezeptorschicht neben dem Analyten noch weitere Substanzen anbinden. Eine Substanz, welche vorzugsweise mit hoher Spezifität und Selektivität an einen Rezeptor bindet, wird hier generell als Zielmolekül oder auch als Ligand für diesen Rezeptor bezeichnet. So stellt bei einem gegebenen Biosensor der Analyt ein Zielmolekül für die Rezeptoren dar. Weitere Zielmoleküle können aber auch vom eigentlichen Analyten verschiedene Moleküle sein, die ebenfalls an die Rezeptoren der Rezeptorschicht binden, und beispielsweise mit dem Analyten um Bindungsstellen der Rezeptorschicht konkurrieren (Fachbegriff: Kompetition).

Bei metabolischen Biosensoren kommt es zur katalytischen Umsetzung des Analyten und/oder weiterer Zielmoleküle nach deren vorübergehender Anbindung an die Rezeptorschicht, welche bei metabolischen Biosensoren mindestens ein Enzym, eine Zelle, ein Zellbestandteil oder ein sonstiges katalytisch wirksames Biomolekül enthält, das den Analyten - ggfs. mit weiteren vorhandenen Substanzen - katalytisch umsetzen kann.

Zum Nachweis oder zur Konzentrationsbestimmung eines Analyten in einer Flüssigkeitsprobe wird diese mit der Rezeptorschicht in Kontakt gebracht, so dass in der Flüssigkeitsprobe vorhandene Analytmoleküle und gegebenenfalls weitere Zielmoleküle an die Rezeptorschicht binden oder von dieser umgesetzt werden. Dadurch kommt es zu physikalischen und/oder chemischen Veränderungen, beispielsweise zu einer Veränderung der Schichtdicke, der Brechzahl, der Lichtabsorption oder der elektrischen Ladung. Diese Veränderungen können mittels des Signalwandlers nachgewiesen und quantitativ bestimmt werden. Als Signalwandler zur Erfassung der genannten Veränderungen kommen beispielsweise optoelektrische Sensoren, amperometrische oder potentiometrische Sensoren in Frage. Die an die Rezeptorschicht gebundenen Zielmoleküle können auch mit einem Marker, der beispielsweise lumineszierende oder magnetische Eigenschaften aufweist, direkt oder indirekt markiert sein oder nach der Anbindung der Zielmoleküle an die Rezeptorschicht nachfolgend direkt oder indirekt markiert werden. In diesem Fall ist ein optischer Sensor zur Detektion der Lumineszenz bzw. ein magnetischer Sensor zur Erfassung der magnetischen Eigenschaften als Signalwandler geeignet. Weiterhin eignen sich als Marker auch Enzyme, welche eine nachfolgende chemische Reaktion katalysieren, wobei der Reaktionsverlauf mit entsprechend geeigneten Signalwandlern erfasst werden kann.

Es sind zahlreiche unterschiedliche heterogene biosensorische Verfahren bekannt. Exemplarisch werden nachfolgend die am häufigsten eingesetzten Verfahren aufgeführt und kurz erläutert.
- Direktes Verfahren: Der Analyt bindet an Rezeptoren der Rezeptorschicht, ohne dass ein weiteres Zielmolekül anwesend ist. In einer Modifikation des Verfahrens wird der Analyt vor oder nach der Anbindung an die Rezeptoren mit einer oder mehreren weiteren Substanzen verbunden, was in der Regel dazu dient, eine physikalische Eigenschaft, die der Analyt nicht besitzt, direkt oder indirekt einzubringen und so die Anbindung des Analyten durch Bestimmung dieser eingebrachten physikalischen Eigenschaft detektieren zu können (Marker). Bei dem direkten Verfahren wird somit die Anbindung des Analyten an die Rezeptorschicht direkt oder durch Detektion des an den Analyten angebundenen Markers indirekt detektiert.
- Kompetitive Verfahren: Zu der auf einen Analytgehalt zu untersuchenden Flüssigkeitsprobe wird in der Regel ein weiteres Zielmolekül (Kompetitor) in bekannter Konzentration oder Aktivität hinzugefügt und die so erhaltene Messflüssigkeit auf die Rezeptorschicht aufgebracht. Hierbei kommt es bei der Anbindung an die Rezeptoren der Rezeptorschicht zur Konkurrenz zwischen Analyt und Kompetitor. Der Kompetitor besitzt in der Regel einen Marker, der wiederum direkt oder indirekt eine physikalische Eigenschaft in das System einführt, die detektiert werden kann. Bei kompetitiven Verfahren wird somit die Anbindung des Kompetitors an die Rezeptorschicht direkt oder indirekt durch Detektion des Kompetitor-Markers detektiert. Aus dem Messsignal lässt sich die ursprüngliche Analytkonzentration der Flüssigkeitsprobe ableiten.
- Bindungshemmtest: Zu der auf einen Analytgehalt zu untersuchenden Flüssigkeitsprobe wird ein komplementäres Molekül in bekannter Konzentration oder Aktivität hinzugefügt, welches an den Analyten bindet. Nachfolgend wird die so gebildete Messflüssigkeit über die Rezeptorschicht geleitet, welche Rezeptoren aufweist, die das freie, nicht an den Analyten gebundene komplementäre Molekül binden. Diese Anbindung kann direkt mittels geeigneter Methoden detektiert werden. Alternativ kann das komplementäre Molekül vor oder nach der Anbindung an die Rezeptoren oder vor oder nach der Anbindung an den Analyten direkt oder indirekt mit einem Marker verbunden werden. Bei einem Bindungshemmtest wird also die Anbindung des komplementären Moleküls an den Rezeptor direkt oder der Marker des komplementären Moleküls direkt oder indirekt detektiert. Aus dem Messsignal lässt sich die Analytkonzentration der Flüssigkeitsprobe ableiten.

Zur Präparation der Rezeptorschicht werden die Rezeptoren an festen Trägermaterialien immobilisiert, d.h. an diese angebunden. Dies kann beispielsweise durch unspezifische Adsorption der Rezeptoren an die Oberfläche des Trägermaterials, das so genannte Substrat, erfolgen. Aber auch eine kovalente Anbindung der Rezeptoren oder die Anbindung über mehrere weitere Anbindungsschichten sind etablierte Methoden zur Präparation von Rezeptorschichten. So kann beispielsweise an eine feste Trägeroberfläche Streptavidin kovalent angebunden werden und zur Präparation der Rezeptorschicht Biotin-konjugierte Antikörper unter Ausnutzung der Affinitätswechselwirkung zwischen Biotin und Streptavidin an die Streptavidin-Anbindungsschicht angebunden werden. Eine solche Anbindung der Rezeptorschicht ist beispielsweise aus WO 2009/062027 A1 bekannt. Generell spricht man bei dem gesamten Schichtsystem, welches auf der Oberfläche eines festen Trägermaterials aufgebaut wird, von der Sensormatrix des Biosensors, deren abschließende Schicht die Rezeptorschicht darstellt.

Beispielsweise ist aus US 4,271,140 die Anbindung eines Rezeptors für einen gegebenenfalls in einer Probe enthaltenen Liganden über einen oder mehrere an den Rezeptor anbindende Bindungsliganden und einen zweiten, an den oder die Bindungsliganden anbindenden Rezeptor an ein festes Substrat bekannt.

Die Sensormatrix mit der Rezeptorschicht ist in vielen Anwendungen auf einen in ein Mikrofluidiksystem integrierten Biochip aufgebracht. Diese Biochips sind in der Regel Einweg-Produkte, die nach einer einzigen durchgeführten Bestimmung oder Konzentrationsmessung des Analyten weggeworfen bzw. durch einen neuen Biochip ersetzt werden. Auch bei metabolischen Biosensoren muss der Chip oder die Kartusche, welche die Sensormatrix beinhaltet, in regelmäßigen Abständen ausgetauscht werden. Ursache hierfür ist generell die geringe Stabilität und Robustheit von biologischen Rezeptoren sowie die oftmals hohe Affinität, mit der Zielmoleküle an die Rezeptoren gebunden werden. Somit kommt es bei den Bedingungen, unter denen sich die an die Rezeptoren gebundenen Zielmoleküle von den Rezeptoren ablösen, in der Regel auch zu einer mehr oder weniger großen Zerstörung der Rezeptorschicht. Aus der einschlägigen Fachliteratur sind nur wenige Spezialfälle bekannt, in denen eine solche Regenerierung der Rezeptorschicht, d.h. die nahezu vollständige Entfernung der Zielmoleküle unter Erhalt der im Wesentlichen unveränderten Funktionalität der Rezeptorschicht beschrieben wird. Nach dem Stand der Technik ist dies jedoch für die große Mehrzahl der Biosensoren, speziell für Biosensoren zur Bestimmung von Proteinen, nicht möglich.
Automatisierte oder halbautomatisierte Bioanalysatoren, in denen die Rezeptorschicht tragende Einweg-Chips verwendet werden, sind bekannt. So ist im europäischen Patent EP 1 343 011 B1 beispielsweise eine Vorrichtung zum elektrochemischen Nachweis einer Nukleotidsequenz beschrieben, die eine austauschbare, vorzugsweise als Einweg-Bauteil ausgestaltete Analysekassette zum Einfüllen der Flüssigkeitsprobe aufweist, welche eine Messelektrode mit einer Rezeptorschicht aufweist, an die die Nukleotidsequenz selektiv und spezifisch bindet. Die Analysekassette ist mit einer Recheneinrichtung über eine analoge Schnittstelle verbindbar, wobei ein elektrochemischer Nachweis der Nukleotidsequenz mittels der Recheneinrichtung durchführbar ist.

Zur Anwendung von Biosensoren, insbesondere von affinitätsbasierten Biosensoren, in der Prozessmesstechnik, zum Beispiel zur automatisierten Überwachung eines biotechnologischen Prozesses in einer industriellen Anlage oder zur automatisierten Überwachung von Wasser beispielsweise auf Rückstände von Medikamenten oder auf den Gehalt an endokrin wirksamen Substanzen, ist es dagegen wünschenswert, eine Vielzahl von Messungen nacheinander durchführen zu können, ohne dass für jede Messung ein Austausch oder ein Wechsel eines die Sensormatrix beinhaltenden Chips oder sonstigen Bauteils erforderlich ist. Eine mögliche Anwendung der Biosensorik für derartige Messaufgaben kann ein Bioanalysator sein, dem, insbesondere online, Flüssigkeitsproben eines zu überwachenden Prozessmediums zugeführt werden. Optional können die Flüssigkeitsproben, vorzugsweise automatisiert, mit Reagenzien, beispielsweise mit weiteren Zielmolekülen, Markern etc. zur Durchführung der voran stehend beschriebenen Verfahren vorbehandelt werden. Der Bioanalysator umfasst weiterhin eine Rezeptorschicht, der die Flüssigkeitsprobe bzw. die durch Vorbehandlung erhaltene Messflüssigkeit zugeführt wird, einen Signalwandler, der ein mit der Anzahl der an die Rezeptorschicht gebundenen Analyt-oder Zielmoleküle korreliertes Messsignal ausgibt, und eine Signalverarbeitungseinrichtung, die das Messsignal weiter verarbeitet und insbesondere einen aus diesem abgeleiteten Messwert ausgibt. Die Zuleitung der Proben und ggfs. Reagenzien zur Rezeptorschicht und die Ableitung der Flüssigkeitsprobe bzw. Messflüssigkeit nach erfolgter Messung kann in einem automatisierten oder halb-automatisierten Analysator mittels Pumpvorrichtungen, pneumatischen System oder anderen Flüssigkeitstransporteinrichtungen durchgeführt werden. Um die benötigte Reagenzienmenge und damit auch das nach erfolgter Analyse zu entsorgende Flüssigkeitsvolumen klein zu halten, sollten Zu-und Ableitungen für die Probe und ggfs. Reagenzien einen möglichst geringen Querschnitt, z.B. im Sub-mm-Bereich, aufweisen. Derartige Flüssigkeitsleitungen mit Querschnitten im Sub-mm-Bereich werden im Folgenden auch als Mikrofluidikkanäle bezeichnet. Ein Modul eines Bioanalysators, das solche Mikrofluidikkanäle aufweist, wird auch als Mikrofluidik-Einheit bezeichnet.

Um den Wartungsaufwand eines für solche Zwecke eingesetzten Bioanalysators zu minimieren bzw. um den Automatisierungsgrad zu erhöhen, besteht deshalb ein Bedarf an für eine Vielzahl nacheinander durchführbarer Messungen geeigneten Biosensoren, deren Sensormatrix bzw. deren Rezeptorschicht mit hoher Reproduzierbarkeit *in situ* im Wesentlichen vollständig regenerierbar bzw. erneuerbar ist.

Aus der Literatur sind unterschiedliche Ansätze bekannt, wie Sensormatrizen oder Rezeptorschichten von Biosensoren regeneriert werden können. Häufig werden hierbei durch Veränderungen im Lösungsmittel Effekte bewirkt, die eine Lösung der Bindung zwischen Rezeptor und Zielmolekül bewirken. Häufig wird hierzu der pH-Wert der Lösung verändert. Auch Änderung der lonenstärke, der Lösungsmittelpolarität oder der Zusatz spezifisch wirkender Substanzen werden oftmals eingesetzt, um eine Regenerierung von Rezeptorschichten zu erzielen.

Aus EP 1 347 300 A1 ist beispielsweise ein Verfahren zum Regenerieren einer Sensormatrix für einen SPR Bindungsassay, bei dem ein Maus-Antikörper gegen den humanen IL-6 Rezeptor auf einem Biacore-Chip immobilisiert wird und an diesen der humane IL-6 Rezeptor angebunden wird. Die IL-6 Rezeptoren bilden eine Rezeptorschicht zum Nachweis des an die Rezeptorschicht als Analyt anbindenden Anti-IL-6-Antikörpers. Die Regenerierung der Sensormatrix erfolgt über die Zugabe von saurer Glycinlösung, wobei die Sensormatrix von dem IL-6 Rezeptor und dem daran angebundenen Analyten befreit wird. Der Maus-Antikörper verbleibt dagegen auf dem Substrat.

WO 01/79848 A1 beschreibt eine Immobilisierung von Rezeptormembranfragmenten (RMFs) auf einem Substrat mittels Anbindung der biotinylierten V2-RMFs an kovalent auf einer Oberfläche angebundenem Anti-Biotin oder mittels Anbindung von V2-RMF an auf einem Substrat immobilisiertem Anti-Biotin über eine Zwischenschicht von biotinyliertem Lectin, welches an Saccharid-Komponenten der RMFs anbindet und/oder in die Lipidstruktur der RMFs eingebunden wird. Eine Regenerierung der Sensormatrix erfolgt durch einen Spülschritt mit 25 mM NaOH, der in beiden Ausgestaltungen eine Spaltung der Antibiotin-Biotin-Bindung bewirkt, so dass Anti-Biotin auf der regenerierten Oberfläche verbleibt und neue RMFs angebunden werden können.

Nachteilig an diesen Verfahren ist jedoch, dass, um eine mehrfache oder vielfache Regenerierung der Rezeptorschicht zu ermöglichen, fürjede Rezeptor-Zielmolekül-Wechselwirkung der optimale Kompromiss zwischen optimaler Regenerierungsleistung und minimaler Zerstörung der Rezeptoren in aufwändigen Versuchsreihen ermittelt werden muss. Eine Erneuerung der Rezeptoren bzw. der Rezeptorschicht ist nach diesen Methoden nicht möglich.

Für eine Rezeptorschicht mit Haptenen als Rezeptoren, die besonders klein und robust sind, ist in A.T. Tüdös, E.R. Lucas-van-den Bos, E.C.A. Stigter, "Rapid surface plasmon resonance-based inhibition assay of deoxynivalenol", Journal of Agricultural and Food Chemistry 51 (2003), 5843-5848, ein solches Regenerierungsverfahren beschrieben. Dort sind zwischen 499 und 717 Regenerierungszyklen gezeigt. Für Immunoassays, bei denen die Rezeptoren aus Proteinen bestehen, sind keine Rezeptorschichten bekannt, die sich mit ähnlichem Erfolg vielfach regenerieren lassen.

Aus EP 2 189 793 A1 ist bekannt, dass zur Entfernung der Zielmoleküle von den Rezeptoren der jeweiligen Rezeptorschicht Enzyme eingesetzt werden können, die die an den Rezeptoren angebundenen Zielmoleküle selektiv zersetzen. Hierbei muss jedoch darauf geachtet werden, dass die eingesetzten Enzyme die Rezeptoren nicht gleichermaßen angreifen, was die Anwendbarkeit stark einschränkt. Aufgrund der chemischen Natur einer solchen enzymatischen Zersetzung der angebundenen Zielmoleküle erscheint eine bei mehrfacher Durchführung des Verfahrens im wesentlichen gleich bleibende Rezeptordichte äußerst unwahrscheinlich.

Ein weiterer Ansatz zur Regenerierung der Rezeptorschicht eines Biosensors besteht in der Anbindung der Rezeptoren über Oligonucleotide, also kurze DNS-oder RNS-Moleküle. Hierzu werden Oligonucleotide kovalent an eine Oberfläche gebunden und die Rezeptoren oder Moleküle, die eine entsprechende Anbindungsschicht zur Anbindung von Rezeptoren bilden, mit dem komplementären Oligonucleotid konjugiert. Durch spezifische affine Wechselwirkung zwischen den komplementären Oligonucleotiden kommt es zu einer Immobilisierung der Rezeptoren an der Oberfläche und damit zur Ausbildung einer Rezeptorschicht. Die affine Wechselwirkung zwischen den komplementären Oligonucleotiden kann chemisch oder durch Erhöhung der Temperatur wieder gelöst werden, so dass auch die Rezeptoren wieder von der Oberfläche abgelöst werden. Allerdings ist dieses Verfahren in Gegenwart von Proteinen, z.B. von Antikörpern als Rezeptoren, nicht allgemein durchführbar, da Proteine bei Temperaturerhöhung über 40°C bzw. in Gegenwart der für die Ablösung notwendigen Reagenzien in der Regel denaturieren. Hierbei kann es, speziell bei hohem Proteingehalt, bei der Denaturierung der Proteine zur Bildung von schwer löslichen Proteinaggregaten kommen, die sich ohne aggressive chemische Reinigung nur unvollständig entfernen lassen und die sich durch Verklumpen und Zusetzen beispielsweise von Flüssigkeitsleitungen des Flüssigkeitszufuhr- und -ableitsystems äußerst störend auf die Funktionalität eines automatisiert oder halb-automatisiert betriebenen Bioanalysators auswirken. Gleiches gilt, wenn die an die Rezeptorschicht gebundenen Moleküle untereinander verbrückt oder vernetzt sind. Auch hier müssen aggressive chemische Bedingungen zur Auflösung dieser verbrückten oder vernetzten Moleküle eingesetzt werden. Unter den aggressiven chemischen Bedingungen, die zur Entfernung der Proteinaggregate erforderlich wären, sind im übrigen auch die oberflächengebundenen Oligonucleotide nicht mehr stabil.

Weiterhin sind aus der Literatur erste Ansätze einer elektrochemischen Entfernung von einfach strukturierten biologischen Schichten bekannt. Im Gegensatz zur chemischen Entfernung der Sensormatrix bzw. Rezeptorschicht mit rein chemischen Mitteln, z.B. durch Säuren, Laugen oder Lösungsmittel, wird bei der elektrochemischen Entfernung der Rezeptorschicht ein Stromfluss über das die Rezeptorschicht tragende Substrat bewirkt, der zur oxidativen oder reduktiven Ablösung (Desorption) der Rezeptorschicht bzw. Sensormatrix führt.

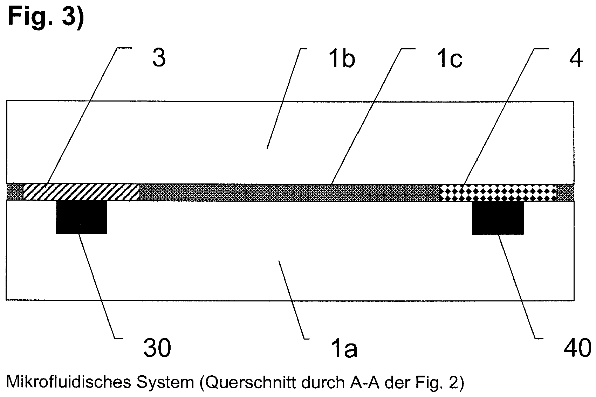

So sind beispielsweise in WO 2010/088219 A2 und in den Artikeln Seokheun Choi, Junseok Chae "Reusable biosensors via in situ electrochemical surface regeneration in microfluidic applications", Biosensors and Bioelectronics 25 (2009) 527-531, Seokheun Choi, Junseok Chae, "A regenerative biosensing surface in microfluidics using electrochemical desorption of short-chain self-assembled monolayer", Microfluidics and Nanofluidics 7 (2009) 819-827 oder Christie A. Canaria et al., "Formation and removal of alkylthiolate self-assembled monolayers on gold in aqueous solutions" Lab on a Chip 6 (2006) 289-295 Ansätze beschrieben, Rezeptoren mittels Alkylthiolen als Anbindungsmoleküle auf Goldoberflächen aufzubringen, um so eine Rezeptorschicht zur Verfügung zu stellen. In diesen Artikeln sind auch Untersuchungen zu der Problematik beschrieben, die als Anbindungsmoleküle verwendeten Alkylthiole wieder abzulösen. Probleme treten dabei insbesondere durch Re-Adsorption bereits abgelöster Alkylthiole und bei elektrochemischer Auflösung der Alkylthiole auftretende Wasserstoffentwicklung auf. Zudem ist bei wiederholter Präparation und Entfernung der Schichten eine stetige Drift in der Oberflächenbelegung zu beobachten, was auf eine nicht vollständige Entfernung hindeutet. In keinem der zitierten Artikel wird darauf eingegangen, ob die Reinigungsleistung bei unterschiedlicher Belegung der Rezeptorschicht gleichermaßen erfolgt. Dies ist jedoch für die Anwendung in einem automatisierten Bioanalysator für die Prozessmesstechnik unerlässlich, da biosensorische Messungen in der Regel gegen eine oder mehrere Referenzmessungen durchgeführt werden, bei denen die Belegung der Rezeptorschicht in der Regel entweder nicht oder nahezu vollständig erfolgt. Speziell bei SPR-Messungen, wie sie in den Artikeln beschrieben sind, ist die Güte der Referenzmessung entscheidend für die Qualität des Messergebnisses. So ist beispielsweise bei Seokheun Choi, Junseok Chae "Reusable biosensors via in situ electrochemical surface regeneration in microfluidic applications", Biosensors and Bioelectronics 25 (2009) 527-531 nicht davon auszugehen, dass die Reinigungsleistung bei Rezeptorschichten, an die viel Fibrinogen angebunden ist (Fig. 3c), die gleiche ist, wie bei Rezeptorschichten, an die nur wenig Fibrinogen angebunden ist.

Aus dem Artikel S. Balasubramanian et al., Electrochemical Desorption of Proteins from Gold Electrode Surface, Electroanalysis 18 (2006) No. 19-20, 1885-1892 ist ein Verfahren bekannt, bei dem ein Substrat mit einer Schicht aus Mercaptoundecansäure modifiziert, die Carboxylgruppen dieser Schicht aktiviert und an die aktivierten Gruppen Rinderserumalbumin (BSA) oder ein OPH-Antikörper als Rezeptor für OPH kovalent angebunden wird. Bei einem reduzierenden Potential von - 1200 mV wird die Mercaptoundecansäure-Schicht und damit die gesamte Sensormatrix wieder abgelöst und das Substrat anschließend abgewaschen.

Für die automatisierte oder halbautomatisierte Anwendung der Biosensorik in der Prozessmesstechnik ist es wichtig, dass nacheinander ausgeführte Konzentrationsbestimmungen miteinander vergleichbare Messergebnisse liefern. Es ist deshalb ein System zur Verfügung zu stellen, das auch nach einer Vielzahl von Regenerierungen bzw. Erneuerungen der Rezeptorschicht, beispielsweise nach 50, 100 oder mehr Regenerierungs- oder Erneuerungszyklen für gleiche Zielmolekülkonzentrationen im wesentlichen gleiche Messsignale, d.h. Messsignale vergleichbarer Intensität, insbesondere bei Bezug dieser Messsignale auf die Referenzmessung, liefert.

Weiterhin ist es für einen automatisiert oder halbautomatisiert arbeitenden Bioanalysator wünschenswert, dass eine Vielzahl unterschiedlicher Rezeptoren in einfacher Weise in einem für den Signalwandler des Bioanalysators zugänglichen Messbereich immobilisiert werden können, um den Analyten in einem direkten Verfahren oder ein anderes Zielmolekül in einem kompetitiven Verfahren oder in einem Bindungshemmtest nachzuweisen. Für die Fertigung solcher Bioanalysatoren wäre es vorteilhaft, für die automatisierte Durchführung einer Vielzahl unterschiedlicher Assays bzw. Analyseverfahren eine im Wesentlichen universelle Grundstruktur, z.B. eine Mikrofluidik-Einheit mit einer bestimmten Flüssigkeitskanal-Struktur zum Durchleiten der Flüssigkeitsprobe und von Reagenzien durch den Messbereich zur Verfügung zu stellen, in der die Vielzahl unterschiedlicher Verfahren durchgeführt werden kann.

Es ist deshalb die Aufgabe der vorliegenden Erfindung, ein für die Anwendung in der Prozessmesstechnik geeignetes Verfahren zur Bestimmung eines Analytgehalts in einer Flüssigkeitsprobe mittels eines Bioanalysators der vorstehend beschriebenen Art bzw. einen zur Durchführung dieses Verfahrens geeigneten Bioanalysator zur Verfügung zu stellen.

Diese Aufgabe wird gelöst durch ein Verfahren zur automatisierten *in situ*-Bestimmung eines Analytgehalts einer Flüssigkeitsprobe mittels eines Bioanalysators gemäß Anspruch 1.

Vorteilhafte Weiterbildungen und Ausgestaltungen sind in den abhängigen Ansprüchen beschrieben.

Das mindestens eine Substrat wird regeneriert , indem die Sensormatrix und gegebenenfalls daran gebundene Moleküle, insbesondere Analytmoleküle, weitere Zielmoleküle oder sonstige an die Sensormatrix gebundene Substanzen, im Wesentlichen vollständig von dem Substrat abgelöst und zumindest teilweise zersetzt werden. Auf diese Weise kann das mindestens eine Substrat vollständig oder im Wesentlichen vollständig, d.h. zumindest so weit freigelegt werden, dass auf dem Substrat nur in so geringem Maße Restbestandteile der Sensormatrix und eventuell an die Sensormatrix gebundener Substanzen verbleiben, dass diese beim Wiederholen der Schritte (i)-(iii), d.h. bei einer erneuten Präparation der Sensormatrix, einem erneuten Durchleiten einer neuen Flüssigkeitsprobe und beim erneuten Regenerieren des Substrats keinen merklichen Einfluss, insbesondere keinen merklichen Einfluss auf die Messgenauigkeit ausüben.

Die Abfolge der Schritte (i)-(iii) wird im Folgenden auch kurz als "Messzyklus" bezeichnet. Indem das Substrat regelmäßig durch Entfernen der Sensormatrix und eventuell daran gebundener Moleküle wieder freigelegt, und danach die Sensormatrix wieder neu präpariert wird, ist ein Wechsel des Substrats oder ein sonstiger Eingriff in einen Bioanalysator, insbesondere in dessen Messkanal, in dem das beschriebene Verfahren durchgeführt wird, zu Wartungszwecken nicht notwendig. Die Analyse der Flüssigkeitsprobe kann daher vollständig automatisiert mit einer Vielzahl aufeinander folgender Messzyklen durchgeführt werden. Indem das Substrat vollständig oder im Wesentlichen vollständig freigelegt wird, ist gewährleistet, dass bei aufeinander folgenden Messzyklen die Sensormatrix eine im Wesentlichen vergleichbare Beschaffenheit hat, was eine sehr gute Vergleichbarkeit von Mess- bzw. Analyseergebnissen, die in verschiedenen Messzyklen gewonnen werden, gewährleistet.

Eine Messung, die insbesondere das Durchleiten der Flüssigkeitsprobe oder der Messflüssigkeit durch den Messkanal und die Bestimmung einer mit der Menge der von den Rezeptoren gebundenen oder umgesetzten Zielmoleküle oder der Menge des von den Rezeptoren gebundenen oder umgesetzten Analyten korrelierenden Messgröße und daraus Ableiten des Analytgehalts der Flüssigkeitsprobe gemäß Schritt (ii) umfasst, kann mindestens innerhalb eines vorgegebenen Messbereichs durchgeführt werden. Der Messbereich ist ein Teil der Oberfläche des Messkanals. Das Substrat ist zumindest teilweise im Messbereich angeordnet, es kann sich aber auch über den Messbereich hinaus innerhalb des Messkanals oder innerhalb weiterer Flüssigkeitskanäle der Mikrofluidikeinrichtung erstrecken.

Mit dem erfindungsgemäßen Verfahren werden die Rezeptoren, an die die Zielmoleküle, insbesondere der Analyt, anbinden, über die erste chemische Spezies an das Substrat gebunden. Die weitere funktionelle Gruppe dient zur Anbindung des Rezeptors . Durch das Anbinden der ersten chemischen Spezies an das Substrat wird eine erste Anbindungsschicht gebildet, an der wiederum durch spezifische Anbindung an die weitere funktionelle Gruppe der Rezeptor direkt oder über ein-oder mehrere weitere Anbindungsschichten angebunden werden kann. Die eine oder mehrere weiteren Anbindungsschichten können aufeinanderfolgend durch Durchleiten jeweils einer weiteren Präparationslösung, welche weitere, an die jeweils zuoberst angeordnete Anbindungsschicht bindende chemische Spezies zur Bildung einer weiteren Anbindungsschicht enthält, gebildet werden. Auf diese Weise wird eine Sensormatrix aus einer oder mehreren Anbindungsschichten und einer abschließenden, an der obersten Anbindungsschicht gebundenen Rezeptorschicht gebildet. Die durch die ersten chemischen Spezies gebildete unterste Anbindungsschicht und jede weitere der Anbindungsschichten sind an Bindungsstellen der die jeweils darunter liegende Schicht bildenden chemischen Spezies, vorzugsweise durch spezifische affine Wechselwirkung, z.B. über eine Biotin-Streptavidin-Bindung oder der Bindung zwischen einem Antikörper und seinem Antigen, gebunden. Auch der Rezeptor ist vorzugsweise über eine spezifische affine Wechselwirkung an der obersten Bindungsschicht gebunden. Die Verwendung von weiteren Anbindungsschichten, d.h. die indirekte Anbindung des Rezeptors an die ersten chemischen Spezies über zweite und evtl. weitere chemische Spezies bietet den Vorteil, dass diese so ausgestaltet werden können, dass an ein gegebenes Anbindungsschichtsystem zahlreiche unterschiedliche Rezeptoren, die jedoch alle über die selbe Funktionalität bezüglich ihrer Anbindung verfügen, als Rezeptorschicht präpariert werden können, ohne dass es hierfür einer Anpassung der Anbindungsschichten oder der Rezeptoren bedarf.

Der Schritt (ii), der das Durchleiten und Analysieren der Flüssigkeitsprobe bzw. der durch Behandeln, z.B. Mischen der Flüssigkeitsprobe mit einem Reagenz, erzeugten Messflüssigkeit umfasst, kann ein-oder mehrmals wiederholt werden, bevor in Schritt (iii) eine Freilegung des Substrats erfolgt.

Durch wiederholte Durchführung der Folge der Verfahrensschritte (i) bis (iii) kann die Rezeptorschicht bzw. die Sensormatrix auf demselben Substrat immer wieder *in situ* d.h. ohne Eingriff in den Messkanal, neu aufgebaut und so wiederholt der Analytgehalt von nacheinander dem Bioanalysator zugeführten Flüssigkeitsproben automatisiert ermittelt werden. Vorzugsweise können die Verfahrensschritte (i) bis (iii) mindestens 50 mal, vorzugsweise mindestens 150 mal, weiter bevorzugt mindestens 300 mal durchgeführt werden.

Unter funktionellen Gruppen werden allgemein Atomgruppen verstanden, die die Stoffeigenschaften und das Reaktionsverhalten der sie tragenden Verbindungen maßgeblich bestimmen. Der Begriff der funktionellen Gruppe umfasst hier und im Folgenden auch Atomgruppen oder Molekülregionen, die bindende, vorzugsweise spezifische, Wechselwirkungen mit anderen chemischen Spezies ausbilden können sowie auch an eine chemische Spezies, insbesondere ein Molekül, gebundene Markierungen, beispielsweise aus Molekülen, Peptiden, Proteinen oder Protein-Tags. Beispielsweise bildet ein an ein Alkylthiol gebundenes, als Bindungspartner für eine Rezeptor-Zielmolekül-Bindung dienendes Biotin-Molekül, eine erste funktionelle Gruppe des Alkylthiols. Die Thiolgruppe des Alkylthiols bildet eine zweite funktionelle Gruppe.

Die an das Substrat bindende funktionelle Gruppe der ersten chemischen Spezies ist vorzugsweise auf das Substratmaterial abgestimmt. Das Substrat kann beispielsweise aus Metall bestehen oder zumindest eine metallische Beschichtung umfassen.

Unter einem Rezeptor wird hier eine funktionelle Gruppe gemäß der voranstehenden Definition verstanden, an welche ein komplementäres Molekül, auch als Zielmolekül bezeichnet, vorzugsweise selektiv und spezifisch, insbesondere aufgrund einer affinen Wechselwirkung, bindet, oder durch welche die Umsetzung anderer chemischer Spezies enzymatisch katalysiert wird. Hierbei muss es sich nicht unbedingt um einen Rezeptor im biologischen Sinne, wie beispielsweise um einen Antikörper, handeln.

Zur Bestimmung des Analytgehalts einer Flüssigkeitsprobe ist eine Vielzahl unterschiedlicher Verfahren denkbar. Beispielsweise kann der Analyt selbst als Zielmolekül direkt an die Rezeptorschicht angebunden werden (direktes Verfahren). Es ist auch möglich, den Analyt und ein vom Analyt verschiedenes Zielmolekül an die Rezeptorschicht zu binden, eine mit der Anzahl der an die Rezeptorschicht gebundenen Zielmoleküle korrelierte Messgröße zu erfassen und daraus auf die Analytkonzentration zu schließen (kompetitive Verfahren). Es ist weiterhin möglich, die Messflüssigkeit vor dem Durchleiten durch den Messkanal mit einer Vorbehandlungslösung zu vermischen, die einen Reaktionspartner des Analyten in einer bekannten Konzentration enthält, und das Gemisch durch den Messkanal zu leiten. In diesem Fall dient der verbleibende, nicht an Analytmoleküle gebundene Reaktionspartner als Zielmolekül, wobei aus der Anzahl der an die Rezeptorfläche gebundenen Zielmoleküle auf die Konzentration des Analyten in der Messlösung geschlossen werden kann (Bindungshemmtest).

Unter der Bestimmung eines Analytgehalts ist ein rein qualitativer Nachweis eines Analyten, eine semi-quantitative Bestimmung eines Anteils des Analyten in einem bestimmten Probenvolumen der Messflüssigkeit oder auch eine quantitative Konzentrationsermittlung oder Aktivitätsbestimmung des Analyten in der Messflüssigkeit zu verstehen.

Vorzugsweise dient die Bestimmung des Analytgehalts gemäß dem hier und im Folgenden beschriebenen Verfahren nicht zur Bestimmung von Desoxyribonucleinsäure (DNA) und Ribonucleinsäure (RNA).

Unter einer chemischen Spezies wird hier und im Folgenden insbesondere ein Molekül, ein Komplex, ein Biomolekül-Aggregat oder ein Mikroorganismus verstanden. Als Moleküle kommen beispielsweise Enzyme, Peptide, Proteine oder DNS in Frage. Beispiele für Komplexe sind Protein-Komplexe, die auch als Protein-Aggregate bezeichnet werden. Mikroorganismen können insbesondere Zellen, wie z.B. Hefezellen, sein.

Das Substrat ist elektrisch leitfähig. Es kann sich dabei beispielsweise um einen elektrisch leitfähigen Oberflächenbereich des Messkanals handeln, der z.B. als ein auf einer Wand des Messkanals aufgebrachter Metallfilm ausgestaltet ist. Das Substrat kann beispielsweise ein chemisch stabiles Metall mit hoher Thiophilie, insbesondere Gold oder Platin umfassen. In diesem Fall kann die an das Substrat bindende funktionelle Gruppe ein an das Substrat, insbesondere kovalent, bindendes, Schwefelatom umfassen. Besonders geeignet sind hierzu Thiol- oder Disulfidgruppen.

Als erste chemische Spezies kommen beispielsweise Thiole, insbesondere Alkylthiole und Polyethylenglykol-Thiole in Frage. Solche Moleküle sind als Anbindungsmoleküle, sog. Linker, für biochemische Moleküle, insbesondere für Rezeptoren eines Biosensors der Fachwelt bekannt (vgl. die eingangs genannten Artikel). Mit einer Thiolgruppen-bindenden Oberfläche als Substrat bilden Alkylthiole kovalente Bindungen zwischen dem Substrat, bspw. einem Gold- oder Platin-Substrat, und dem Schwefelatom der Thiolgruppe aus und bilden auf der Oberfläche häufig selbstorganisierte Monoschichten (engl. Fachausdruck: self-assembled Monolayer, kurz: SAM). Die Ausbildung solcher SAMs ist jedoch für den Aufbau einer Sensormatrix bzw. einer Rezeptorschicht nicht zwingend erforderlich.

Das hier und im Folgenden beschriebene Verfahren erfolgt vorzugsweise automatisiert, wobei eine Steuereinheit, die eine elektronische Datenverarbeitungseinheit, z.B. einen Mikrocomputer, umfasst, das Durchleiten der Flüssigkeiten durch den Messkanal und die Erfassung von Messwerten steuert. Die Mittel zum Durchleiten der Flüssigkeiten können insbesondere Pumpen, z.B. Spritzenpumpen, oder pneumatische Mittel umfassen. Die von einem zur Erfassung der Messgröße geeigneten Signalwandler des Bioanalysators ausgegebenen Messsignale können von der Steuereinheit in ein digitales Signal gewandelt und daraus ein Messwert der Messgröße gebildet werden.

Das Freilegen des Substrats erfolgt durch Erzeugen eines elektrischen Stromflusses zwischen dem Substrat und einer über einen Elektrolyten mit dem Substrat in elektrisch leitfähigem Kontakt stehenden Gegenelektrode, um das Substrat, insbesondere im Wesentlichen unabhängig von der vorhandenen Belegung des Substrats durch die Sensormatrix und gegebenenfalls daran gebundener weitere Substanzen, wieder freizulegen. Zur Entfernung der Sensormatrix wird somit ein elektrischer Stromfluss zwischen dem Substrat und einer Gegenelektrode erzeugt, der eine Freilegung des Substrats, das heißt eine vollständige oder nahezu vollständige Entfernung und/oder zumindest teilweise Zersetzung der Sensormatrix und eventuell daran gebundener Substanzen bewirkt.

Beinhaltet eine der zur Präparation der Schichten eingesetzte Spezies mehr als eine Bindungsstelle für eine Spezies der darunter liegenden Schicht oder für eine Spezies, welche die nachfolgende Schicht bildet, so kann es bei hohen Bedeckungsgraden der einzelnen Schichten auf molekularer Ebene zur Ausbildung von kovalent oder nicht-kovalent verbundenen Verzweigungen bis hin zur Bildung eines Netzwerks kommen. In diesen Fällen ist eine Entfernung der Rezeptorschicht durch Auflösen der Schichtstruktur und ein Freilegen des Substrats mit rein chemischen Mitteln, beispielsweise durch Säuren, Oxidationsmitteln oder organischen Lösungsmittel häufig nicht vollständig oder nur unter Schädigung des Substrats zu erreichen. Es hat sich jedoch gezeigt, dass eine elektrochemische Auflösung der Schichtstruktur durch Erzeugen eines elektrischen Stromflusses zwischen dem beispielsweise als elektrisch leitfähigen Oberflächenbereich des Messkanals gestalteten Substrat und einer über einen Elektrolyten mit dem Substrat in elektrisch leitfähigem Kontakt stehenden Gegenelektrode zu einer weitestgehend vollständigen Ablösung der gesamten Schichtstruktur unter Freilegung des Substrats führt. Insbesondere ist eine weitestgehend vollständige Freilegung der leitfähigen Oberfläche zu erzielen, wenn es durch den erzeugten Stromfluss an der Oberfläche zur elektrolytischen Entstehung von Sauerstoff oder Wasserstoff oder abwechselnd von Sauerstoff und Wasserstoff kommt (elektrochemische Reinigung). Vorteilhaft ist eine Optimierung der elektrochemischen Reinigung dahingehend, dass die Ablösung der Sensormatrix und daran gebundener Moleküle oder sonstiger chemischer Spezies unter mindestens teilweiser Zersetzung der Sensormatrix erfolgt.

Der Schritt des Erzeugens eines elektrischen Stromflusses zwischen dem Substrat und der Gegenelektrode wird so durchgeführt, dass an dem Substrat und/oder der Gegenelektrode durch Elektrolyse Wasserstoff und/oder Sauerstoff gebildet wird. Auf diese Weise wird jeweils die Reinigungswirkung der durch den Stromfluss bewirkten Oxidation bzw. Reduktion der Rezeptorschicht bzw. der Sensormatrix durch den gebildeten Wasserstoff bzw. Sauerstoff noch verstärkt. Im weiter oben zitierten Artikel von Seokhun Choi et al. wird dagegen die oxidative Desorption der Schichten in Gegenwart von chloridhaltigen Lösungen wie beispielsweise einer Phosphat-Kochsalz-Lösung während der Reinigung durchgeführt, was das Anlegen höherer Potentiale und einer damit verbundenen oxidativen Zersetzung der Sensormatrix ausschließt, da andernfalls die dadurch bei positiven Potentialen gebildeten Gold-Clorokomplexe zu einer Zerstörung der Goldoberfläche führen. Wird die Anwesenheit von Chlorid und/oder Cyanid jedoch vermieden, kann die elektrochemische Reinigung sowohl oxidierend als auch reduzierend bei höheren bzw. niedrigeren Potentialen ausgeführt werden, und dadurch die Reinigungsleistung weiter verbessert werden. Insbesondere können abwechselnd oxidierende und reduzierende Bedingungen am Substrat eingestellt werden. Vorzugsweise wird während des Stromflusses der elektrisch leitfähige Kontakt zwischen dem Substrat und der Gegenelektrode durch einen Elektrolyten vermittelt, der Cyanid- und/oder Halogenidionen in Konzentrationen von weniger als 150 mmol/l, bevorzugt weniger als 15 mmol/l, weiter bevorzugt weniger als 1,5 mmol/l, enthält, insbesondere wenn es sich bei dem Substrat um einen Gold- oder Platinfilm handelt.

So kann, indem an dem Substrat durch Elektrolyse Wasserstoff und/oder Sauerstoff gebildet wird, eine sehr hohe und auch über mehr als 50 Messzyklen gleich bleibende Reinigungsleistung erreicht und jeweils nachfolgend die Sensormatrix *in situ* mit hoher Reproduzierbarkeit wieder neu aufgebaut werden. In eigenen Untersuchungen wurde gezeigt, dass sowohl eine verbrückte Proteinschichtstruktur bestehend aus der Schichtfolge Alkylthiol-PEG-Biotin - NeutrAvidin als auch eine hochvernetzte Proteinschichtstruktur bestehend aus der Schichtfolge Alkylthiol-PEG-Biotin - NeutrAvidin - BSA-Biotin - NeutrAvidin - BSA-Biotin - NeutrAvidin, sich hierbei wiederholt und in gleichem Maße entfernen ließ und die jeweils nach dem Entfernen der Schichtstruktur nachfolgend aufgebaute Sensormatrix die gleiche Eigenschaften aufwies wie eine Sensormatrix, die in einem Vergleichsversuch an ein neues, nicht regeneriertes Substrat aufgebaut wurde.

Die Gegenelektrode kann innerhalb des Messkanals, insbesondere dem Substrat gegenüberliegend angeordnet sein. So kann die Gegenelektrode beispielsweise aus einem chemisch sehr inerten, vorzugsweise als Film aufgebrachtem, Material bestehen. Ein solches inertes Elektrodenmaterial bildet beispielsweise eine Schicht aus Bor-dotiertem diamantartigem Kohlenstoff. Solche Elektroden werden zum Beispiel bei der Wasseraufbereitung verwendet, wo diese zur elektrolytischen Generierung von Hydroxylradikalen zur Zersetzung von organischen Materialrückständen eingesetzt werden.

Die Gegenelektrode kann ebenfalls als im Messkanal, insbesondere dem Substrat gegenüberliegendes, weiteres Substrat ausgestaltet sein, an das die an das Substrat bindende funktionelle Gruppe der ersten chemischen Spezies bindet. Beispielsweise können sowohl das Substrat als auch die Gegenelektrode ein Thiolgruppen- oder Disulfidgruppen-bindendes, elektrisch leitfähiges Material umfassen. Das Substrat und die als weiteres Substrat dienende Gegenelektrode können beispielsweise als Gold- oder Platin-Filme auf gegenüberliegenden Wänden des Messkanals gebildet sein. Bei Durchführung des Schritts (i) wird auf diese Weise sowohl auf dem Substrat als auch auf der Gegenelektrode eine Sensormatrix mit einer Vielzahl von Rezeptoren gebildet. Bei Durchführen des Schritts (ii) werden in der Flüssigkeitsprobe oder in der Messflüssigkeit enthaltener Analyt und/oder weitere in der Flüssigkeitsprobe oder in der Messflüssigkeit enthaltene Zielmoleküle vorzugsweise selektiv und spezifisch an die Rezeptoren der auf dem Substrat und der auf dem weiteren Substrat gebildeten Sensormatrix gebunden, oder von diesen chemisch umgesetzt. Eine mit der Menge des von den Rezeptoren beider Sensormatrizen gebundenen oder umgesetzten Analyten oder mit der Menge der von den Rezeptoren beider Sensormatrizen gebundenen Zielmoleküle korrelierende Messgröße kann bestimmt werden. Dies ist besonders vorteilhaft, da sich hierdurch die effektiv zur Verfügung stehende Substratoberfläche vergrößert, was sich auf Grund des entsprechend größeren Messsignals positiv auf die Messgenauigkeit auswirkt. Die Regenerierung gemäß Schritt (iii) dient zur Freilegung beider Substrate.

Zur Steuerung der elektrochemischen oxidativen und/oder reduktiven Entfernung der Sensormatrix kann während des Erzeugens eines elektrischen Stromflusses zwischen dem Substrat und der Gegenelektrode das elektrische Potential des Substrats in Bezug auf ein elektrisches Referenzpotential einer mit dem Substrat über den Elektrolyten, insbesondere mittels eines Stromschlüssels, in elektrolytischem Kontakt stehenden Referenzelektrode eingestellt, insbesondere geregelt werden. Die Regelung kann die Steuereinheit des Bioanalysators übernehmen. Durch Verwendung einer Referenzelektrode zur Einstellung bzw. Regelung des Potentials des Substrats bzw. der Gegenelektrode kann das Auftreten zu großer positiver oder negativer Potentiale an dem als Arbeitselektrode geschalteten Substrat und/oder an der Gegenelektrode vermieden werden, die sonst zum Angriff oder gar zur Zerstörung des Substrats bzw. der Gegenelektrode führen können. Unter elektrolytischem Kontakt ist ein ionenleitender Kontakt über einen Elektrolyten, insbesondere eine ionenhaltige wässrige Lösung zu verstehen. Vorzugsweise handelt es sich um einen diaphragmenlosen Stromschlüssel. In dem oben zitierten Artikel von Seokheun Choi et al. wird dagegen eine reduktive Entfernung einer Rezeptorschicht mittels einer Zwei-Elektrodenschaltung beschrieben. Durch das Fehlen einer Referenzelektrode kann keine Kontrolle der tatsächlich an beiden Elektroden erfolgenden Potentiale durchgeführt werden. Auf diese Weise kann es je nach den im Mikrofluidiksystem herrschenden Bedingungen und nach der Elektrolytkonzentration und - zusammensetzung zu zu großen positiven oder negativen Potentialen an dem Substrat oder der Gegenelektrode kommen, die, wenn sie wiederholt auftreten, zur Zerstörung der Elektrodenfunktionalität führen können. Dies widerspricht jedoch der Zielstellung der hier offenbarten Erfindung.

Die Referenzelektrode kann als elektrisch leitfähige Beschichtung eines mit dem Messkanal verbundenen weiteren Flüssigkeitskanals ausgestaltet sein. Es ist auch möglich, als Referenzelektrode eine stromlos betriebene Pseudo-Referenzelektrode in Form eines Metalldrahts, insbesondere eines Platin- oder Golddrahts zu verwenden, die im Messkanal oder in einem mit dem Messkanal verbundenen weiteren Flüssigkeitskanal angeordnet sein kann. Sollte es im Verlaufe eines Messzyklus zu einer Belegung der Pseudo-Referenzelektrode durch die verwendeten Reagenzien kommen, so wird die Pseudo-Referenzelektrode ebenfalls regeneriert bzw. freigelegt.

In Bezug auf das Referenzpotential der Referenzelektrode wird das Potential des als Arbeitselektrode dienenden Substrats kontinuierlich oder diskontinuierlich, insbesondere linear, zwischen einem Minimal- und einem Maximalwert variiert, um das Substrat durch elektrochemische Reinigung, insbesondere unter Sauerstoff- bzw. Wasserstoffentwicklung, wieder freizulegen. Vorzugsweise wird das Potential mehrfach, insbesondere zwischen 2- und 1000-fach, zwischen dem Maximal- und dem Minimalwert, insbesondere linear, hin und her verfahren. Ebenfalls kann die Verfahrensdauer als auch die Reinigungsdauer in weiten Grenzen variabel und am Reinigungsergebnis orientiert verändert bzw. eingestellt werden. Hierzu kann die Steuereinheit des Bioanalysators entsprechende Eingabemöglichkeiten für eine Bedienperson oder Mittel zum Laden von vorgegebenen Verfahrensparametern zur Verfügung stellen. Optional kann während oder nach dem Erzeugen des Stromflusses oder diesem zwischengeschaltet die an dem Substrat auftretende Stromstärke erfasst werden, um so ein oder mehrere Zyklovoltammogramme zu erhalten, aus denen auf den Zustand des elektrisch leitfähigen Oberflächenabschnitts geschlossen werden kann. Auch alternative Analysenmethoden, beispielsweise optische, voltammetrische oder andere elektrochemische Analysemethoden, können zur Beurteilung des Zustands des Substrats eingesetzt werden.

Alternativ kann auch die Stromstärke des zwischen dem Substrat und der Gegenelektrode erzeugten Stromflusses kontinuierlich oder diskontinuierlich zwischen einem Minimal- und einem Maximalwert variiert werden, um das Substrat durch elektrochemische Reinigung wieder freizulegen. Hierbei darf die sich zwischen dem Substrat und der Referenzelektrode einstellende elektrische Potentialdifferenz einen Minimalwert nicht unterschreiten bzw. einen Maximalwert nicht übersteigen, um das Substrat nicht zu schädigen.

Dient die Gegenelektrode ebenfalls als Substrat, so gelten alle hier für das - als Arbeitselektrode geschaltete - Substrat gemachten Angaben und Bedingungen für diese gleichermaßen.

Vor, während oder nach dem Schritt des Erzeugens eines elektrischen Stromflusses zwischen dem Substrat und der Gegenelektrode können eine oder mehrere Reinigungs- und/oder Hilfsflüssigkeiten durch den Messkanal geleitet werden. Eine vor oder nach dem Erzeugen eines elektrischen Stromflusses zwischen dem Substrat und der Gegenelektrode durchgeleitete Reinigungsflüssigkeit dient vorrangig zur chemischen Reinigung des Substrats. Als Reinigungsflüssigkeit kommen beispielsweise Säuren oder Laugen oder Lösungsmittel in Frage. Als Hilfsflüssigkeiten dienen beispielsweise Elektrolytlösungen, welche die elektrochemischen Vorgänge auf dem elektrisch leitfähigen Substrat vorteilhaft unterstützen (z.B. Na₂SO₄-Lösung oder Zusatz von H₂O₂). Während des Stromflusses durch den Messkanal geleitete Hilfs-oder Reinigungsflüssigkeiten dienen zur Unterstützung der elektrochemischen Reinigung. Als besonders vorteilhaft wurde eine saure, gegebenenfalls zusätzlich Wasserstoffperoxid enthaltende Elektrolytlösung während des Stromflusses eingesetzt. Der verwendete Elektrolyt sollte sich bei den an den Elektroden angelegten elektrischen Potentialen nicht zersetzen oder reagieren. Hier sind entsprechende sogenannte Leitsalze bekannt. Um den stromlosen Zerfall von zugesetzten Peroxiden während der Lagerung zu minimieren, eignen sich hervorragend saure Phosphatpuffer.

Der Elektrolyt und/oder die Hilfsflüssigkeit wird während des Stromflusses kontinuierlich durch den mikrofluidischen Messkanal geleitet. Hierdurch kann eine Verarmung an notwendigen Hilfsreagenzien bzw. eine Anreicherung von reaktiven elektrolytisch erzeugten Substanzen vermieden werden, was sich positiv auf die Lebensdauer der Substratoberfläche auswirkt. Weiterhin werden Abbauprodukte der Sensormatrix und auch die durch Elektrolyse gebildeten Gasblasen aus dem Bereich der Substratoberfläche entfernt, was zu einer rascheren und sicheren Reinigung beiträgt.

Während des Erzeugens eines elektrischen Stromflusses zwischen dem Substrat und der Gegenelektrode kann beispielsweise eine saure Phosphat-Pufferlösung als Hilfsflüssigkeit durch den Messkanal geleitet werden. Vorteilhaft hat sich eine solche Hilfsflüssigkeit zur Unterstützung einer elektrochemischen Reinigung bei einer Variation des Potentials eines Platin-Substrats zwischen negativen Werten im Bereich zwischen -0,5 V und -1,25 V (Reduktion ggfs. mit damit einhergehender Bildung von Wasserstoff) und positiven Werten im Bereich zwischen +1,5 V und +2,25 V (Oxidation ggfs. mit damit einhergehender Bildung von Sauerstoff) in Bezug auf das Potential einer Silber/Silberchloridreferenz erwiesen.

Während des Freilegens oder im Anschluss an das Freilegen des Substrats kann das Substrat auf verbliebene Bestandteile der Sensormatrix überprüft werden, insbesondere mittels einer elektrochemischen Messmethode, z.B. Zyklovoltammetrie oder Impedanzspektroskopie, einer optischen Messmethode, z.B. Ellipsometrie, oder eines Adsorptionstests. Besonders vorteilhaft kann die Messung der Stromstärke während des Freilegens zur Beurteilung und/oder zur Steuerung der Regenerierung bzw. Regenerierungsleistung herangezogen werden. Bei einem Adsorptionstest wird eine Substanz, welche an auf dem Substrat verbliebene Bestandteile der Sensormatrix bindet, durch den Messkanal geleitet und dann die Menge der adsorbierten Substanz ermittelt. Die Überprüfung kann auch zur Steuerung des Freilegens eingesetzt werden.

Die Hilfsflüssigkeit kann beispielsweise auch photochemisch oder elektrochemisch erzeugtes Ozon beinhalten, welches auf Grund seiner stark oxidierenden Wirkung für einen effektiven Abbau der Sensormatrix sorgt. Auch andere, in ihrer Wirkung die Sensormatrix chemisch zersetzende chemische Substanzen oder physikalische Verfahren können verwendet werden. Hierbei kann auf das Erzeugen eines elektrischen Stromflusses und demzufolge auch auf ein oder mehrere elektrisch leitfähige Substrate verzichtet werden.

Allen hier beschriebenen Methoden zur Freilegung des Substrats ist gemeinsam, dass es nicht bloß zu einer Ablösung des an die Rezeptoren gebundenen Analyts bzw. der Analytmoleküle oder der weiteren Zielmoleküle oder sonstiger eventuell an die Rezeptoren gebundener Substanzen von der Rezeptorschicht und ebenfalls nicht bloß zu einer Ablösung der Sensormatrix vom Substrat kommt. Vielmehr kommt es gleichzeitig zu einer Zersetzung der Bestandteile der Sensormatrix und gegebenenfalls hieran gebundener weiterer Moleküle. Hierdurch ist eine wiederholte zuverlässige und sichere Entfernung der Sensormatrix weitgehend unabhängig von der Komplexität der Sensormatrix entsprechend der Aufgabenstellung der Erfindung möglich, insbesondere bei einer Sensormatrix, die Proteine als Bestandteile aufweist und/oder bei der die weiter oben beschriebene Vernetzung der die Sensormatrix bildenden Komponenten und/oder der daran gebundenen Moleküle auftritt. Weiterhin werden alle für einen Messvorgang notwendigen Schritte zum Aufbau der Sensormatrix (Schritt i), zur Bestimmung des Analytgehalts (Schritt ii) und zur Entfernung der Sensormatrix (Schritt iii) *in situ,* d.h. durch sukzessives Durchleiten entsprechender Lösungen durch den Messkanal in einer vorgegebenen Sequenz ohne in sonstiger Weise in den Messkanal einzugreifen, durchgeführt.

Die an das Substrat gebundene Vielzahl von ersten chemischen Spezies bildet eine erste Anbindungsschicht, die weiteren funktionellen Gruppen der ersten chemischen Spezies stellen Bindungsstellen zur nachfolgenden Anbindung der Rezeptoren zur Verfügung.

Die weitere funktionelle Gruppe der ersten chemischen Spezies kann beispielsweise Biotin umfassen. Der Rezeptor kann in diesem Fall direkt oder indirekt über ein an die weitere funktionelle Gruppe der ersten chemischen Spezies gebundenes, Biotin-bindende zweite chemische Spezies mit mindestens einer Biotin-Bindungsstelle an die weitere funktionelle Gruppe der ersten chemischen Spezies gebunden werden.

Die Biotin-bindende zweite chemische Spezies kann ein Biotin-bindendes Molekül, z.B. Avidin, ein Avidin-Derivat oder ein Avidin-ähnliches Molkeül, insbesondere Streptavidin oder NeutrAvidin, umfassen.

Umgekehrt kann auch ein Biotin-bindendes Molekül, z.B. Streptavidin, NeutrAvidin oder Avidin bzw. ein Avidin-Derivat als funktionelle Gruppe der ersten chemischen Spezies dienen. Der Rezeptor wird dann mittels einer an den Rezeptor direkt oder über mehrere Bindungsspezies, z.B. Bindungsmolekülen, gebundenen Biotin-Gruppe angebunden.

Der an die weitere funktionelle Gruppe der ersten chemischen Spezies gebundene Rezeptor kann über mindestens eine nicht-kovalente Bindung an die erste chemische Spezies direkt oder indirekt gebunden sein.

Die zur Anbindung der Rezeptoren zur Verfügung gestellten Bindungsstellen der obersten Anbindungsschicht können ausgewählt sein aus einer Gruppe umfassend wenige, jeweils zur Anbindung einer Vielzahl unterschiedlicher Rezeptoren geeignete, Bindungsstellen. Diese Bindungsstellen sind daher möglichst universell einsetzbar. Beispielsweise können die Bindungsstellen solche zur Anbindung von Biotin sein und in diesem Fall Streptavidin oder NeutrAvidin oder ein Avidin-ähnliches Molekül umfassen. Die Biotin-Streptavidin-Bindung kann als universelle Schnittstelle zur Anbindung einer Vielzahl unterschiedlicher Rezeptoren dienen, da die Biotinylierung bzw. die Anbindung von Streptavidin an die Anbindungsmoleküle mit gängigen Techniken der Biotechnologie einfach und universell möglich ist. Speziell Biotin-funktionalisierte Antikörper sind beispielsweise für die Durchführung von Immunoassays weit verbreitet.

Andere geeignete Bindungsstellen zum Aufbau einer möglichst universellen Anbindungsschicht können Immunglobulin-bindende Bindungsstellen sein. Vorteilhafterweise können diese durch Anbindung eines biotinylierten, Immunglobulin-bindenden Moleküls an eine Bindungsstelle zur Anbindung von Biotin, wie diese oben beschrieben wurde, zur Verfügung gestellt werden.

Die weitere funktionelle Gruppe der ersten chemischen Spezies dient zur Anbindung eines Rezeptors und die durch die erste chemische Spezies gebildete Schicht bildet eine Anbindungsschicht, auf der optional eine oder mehrere weitere Anbindungsschichten gebildet werden können, wobei als abschließende Schicht eine Rezeptorschicht, die durch an die darunter liegenden Bindungsstellen der Anbindungsschicht gebundenen Rezeptoren gebildet wird, aufgebracht wird. Die die einzelnen Schichten bildenden chemischen Spezies können mit den die jeweils darüber oder darunterliegenden Schichten bildenden chemischen Spezies durch eine affine Wechselwirkung, insbesondere eine nicht-kovalente Bindung, verbunden sein.

Vorzugsweise weist jede Schicht der Sensormatrix bezüglich der jeweils darunter liegenden Schicht einen, bezogen auf die jeweiligen Bedingungen, vollständigen Bedeckungsgrad, mindestens jedoch einen Bedeckungsgrad von mehr als 80 % auf. Auf diese Weise wird gewährleistet, dass Messungen mit nacheinander neu präparierten Sensormatrizen untereinander vergleichbare Messergebnisse liefern, da in diesem Fall immer die im Wesentlichen gleiche Anzahl an Bindungsstellen für die Rezeptoren vorhanden ist, so dass jede neu präparierte Sensormatrix eine vergleichbare maximale Anzahl von Zielmolekülen binden kann. Dies gewährleistet eine hohe Reproduzierbarkeit der Messsignale des Bioanalysators, trotz der ständigen Neupräparation der Sensormatrix.

Im Zusammenhang mit der vorliegenden Erfindung ist der Begriff der "Schicht" einer Sensormatrix nicht notwendigerweise als morphologische Beschreibung zu verstehen. Vielmehr wird mit dem Begriff "Schicht" die Vielzahl von im gleichen Anbindungsschritt und auf die gleiche Art und Weise, bevorzugt über nicht-kovalente, weiter bevorzugt über affine Wechselwirkungen, an chemische Spezies der darunter liegenden Schicht oder an das Substrat gebundene chemische Spezies verstanden. Eine Schicht muss nicht nur aus einer Vielzahl gleicher chemischer Spezies, z.B. gleicher Moleküle, insbesondere gleicher Proteine, gebildet sein, sondern kann auch mehrere unterschiedliche chemische Spezies umfassen. Entscheidend ist die Anbindung im gleichen Anbindungsschritt, z.B. durch Durchleiten einer die die Schicht bildenden chemischen Spezies umfassenden Präparationslösung, über die im Wesentlichen gleichen physikalischen Wechselwirkungskräfte.

Zur Erreichung einer hohen Reproduzierbarkeit und einer geringen Exemplarstreuung ist es insbesondere von Vorteil, wenn die mittlere Oberflächendichte und vorteilhafterweise auch die mittlere Orientierung der Rezeptoren der Rezeptorschicht bei wiederholter oder mehrfacher Präparation der Sensormatrix möglichst konstant bleiben. Dies kann dadurch erreicht werden, dass die Oberflächendichte der einzelnen Schichten bei der Präparation der Sensormatrix möglichst hoch und/oder möglichst nahe dem jeweiligen Gleichgewichtszustand aus Anbindung und Ablösung der die jeweilige Schicht bildenden Substanz oder Substanzen an die Bindungsstellen der darunter liegenden Schicht gewählt wird.

Unter einem Bedeckungsgrad von mehr als 80 % wird derjenige Bedeckungsgrad einer Oberfläche verstanden, bei der die Bedeckung der Oberfläche größer als 80 % der Bedeckung im Gleichgewichtszustand bei den jeweils zur Anbindung verwendeten Bedingungen, welche sich im Wesentlichen aus der Konzentration, der Durchflussrate, der Anbindungsdauer und der Geometrie des fluidischen Messkanals zusammensetzen, der anzubindenden Spezies in der Präparationslösung ist. Bei einer vorgegebenen Geometrie des Messkanals können die Durchflussrate und Dauer des Durchleitens der jeweiligen Lösung durch den Messkanal derart gewählt und aufeinander und auf die Konzentration der Lösung abgestimmt werden, dass der Bedeckungsgrad von mehr als 80% erreicht wird. Die genannten Parameter können in Vorversuchen ermittelt werden, und für die Durchführung des beschriebenen Verfahrens zur Ermittlung eines Analytgehalts fest vorgegeben werden. Der Bedeckungsgrad lässt sich durch Messungen ermitteln, wobei der Bedeckungsgrad der jeweiligen Spezies entweder direkt gemessen werden kann, beispielsweise durch Ellipsometrie oder mittels einer Quarz-Mikrowaage, oder indirekt ermittelt werden kann, indem ein mit der jeweiligen Spezies spezifisch bindendes Molekül der jeweiligen Schicht zugeführt wird, und die Anzahl der angebundenen Moleküle ermittelt wird, beispielsweise durch optische Verfahren.

Zur reproduzierbaren Einstellung einer bestimmten Rezeptordichte der Rezeptorschicht kann bei jeder Schicht der Sensormatrix die jeweilige eine Anbindungsschicht bildende Spezies, im Folgenden als "Anbindungsspezies" oder auch als Anbindungsmolekül bezeichnet, zusammen mit einer jeweils weiteren chemischen Spezies anbinden, die an die gleichen Bindungsstellen der darunter liegenden Schicht wie die Anbindungsspezies bindet, aber keine weitere funktionelle Gruppe aufweist, an die nachfolgend durch den Messkanal geleitete chemische Spezies binden können. Aus der Konzentration der Anbindungsspezies, dem Konzentrationsverhältnis und dem Größenverhältnis von Anbindungsspezies und der oder den weiteren chemischen Spezies sowie den jeweiligen Gleichgewichtsverhältnissen der Anbindung und Ablösung ergibt sich die jeweilige Oberflächendichte der Anbindungsspezies. Analog kann so auch die Rezeptordichte der Rezeptorschicht eingestellt werden. So kann beispielsweise im Falle einer Sensormatrix mit der Schichtfolge Gold-Substrat/Alkylthiol-PEG-Biotin/NeutrAvidin/Biotin-konjugierter Antikörper durch Zumischen von Biotin-Polyethylenglykol zu dem Biotin-konjugierten Antikörper die Oberflächendichte der Rezeptoren der Rezeptorschicht verringert werden. In diesem Fall wird die Schicht aus Rezeptoren und Polyethylenglykol ebenfalls als Rezeptorschicht bezeichnet. In gleicher Weise kann auch die Dichte der Anbindungsspezies in den Anbindungsschichten eingestellt werden.

Die mit der Menge der von den Rezeptoren gebundenen oder umgesetzten Zielmoleküle oder mit der Menge des von den Rezeptoren gebundenen oder umgesetzten Analyten korrelierende Messgröße kann eine optische Messgröße sein, welche insbesondere anhand einer Lumineszenzmessung, einer Reflexionsmessung oder einer Absorptionsmessung ermittelt wird. Für prozesstaugliche Bioanalysatoren sind SPR-Messungen auf Grund von großen Störeinflüssen hervorgerufen vor allem durch Temperatur- und Elektrolytschwankungen weniger geeignet. Zur Ermittlung der optischen Messgröße kann beispielsweise an das Zielmolekül eine funktionelle Gruppe gebunden sein oder nachträglich gebunden werden, welche Lumineszenz-Eigenschaften, d.h. Fluoreszenz- oder Phosphoreszenz-Eigenschaften zeigt, oder die eine in einem nachfolgenden Schritt zugeführte Substratlösung unter Erzeugung von Licht (Chemolumineszenz) oder unter Farbveränderung umsetzt.

Wenn die Messgröße eine direkt oder indirekt vom Zielmolekül hervorgerufene chemische Lumineszenz oder die Lumineszenz eines Lumineszenz-Markers des Zielmoleküls ist, ist es vorteilhaft, wenn das Substrat bzw. der das Substrat beinhaltende Messbereich Licht im Wellenlängenbereich zwischen 300 nm und 900 nm zu mindestens 10%, vorzugsweise mindestens 50%, oder sogar mindestens 70% transmittiert. Generell ist es für die Anwendung optischer Messverfahren in dem Bioanalysator vorteilhaft, wenn die Messstrahlung von dem Substrat bzw. dem das Substrat beinhaltenden Messbereich zu mindestens 10%, vorzugsweise mindestens 50%, oder sogar mindestens 70% transmittiert wird. Beispielsweise kann das Substrat als Metallfilm in Gestalt einer Gitterstruktur auf einer Wand des Messkanals aufgebracht sein. Eine Transmission kann in diesem Fall durch Zwischenräume der Gitterstruktur erfolgen.

In einer Ausgestaltung kann der Analyt ein Peptid oder ein Protein sein, wobei das weitere Zielmolekül neben dem Analyten ein weiteres, vom Analyt verschiedenes Molekül, insbesondere ein vom Analyt abgeleitetes Peptid oder Protein ist, welches einen Tag, vorzugsweise einen Protein-Tag, aufweist, der zur direkten oder indirekten Erzeugung der Messgröße dient, oder der die Messgröße beeinflusst. Das vom Analyt verschiedene Zielmolekül kann beispielsweise ein Kompetitor sein, der aufgrund einer Affinitäts-Wechselwirkung wie der Analyt an den Rezeptor bindet (vgl. kompetitives Verfahren). Alternativ kann das vom Analyt verschiedene Zielmolekül ein vorzugsweise selektiv und spezifisch an die Rezeptorschicht anbindendes Molekül, insbesondere ein Peptid oder Protein, sein, das einen Komplex mit den Analytmolekülen bildet, so dass die Anzahl der an die Rezeptorschicht angebundenen Zielmoleküle ein Maß für die Konzentration des Analyten ist (vgl. Bindungshemmtest).

Ein solches einen Protein-Tag aufweisendes Protein oder Peptid als weiteres Zielmolekül kann als rekombinantes Protein oder Peptid einschließlich des Protein-Tags hergestellt werden. Vorteilhaft hierbei ist, dass keine nachfolgende chemische Kupplung des Proteins oder Peptids mit dem als zur nachfolgenden direkten oder indirekten Markierung durch einen Marker verwendeten Protein-Tag erfolgen muss. Handelt es sich bei dem den Protein-Tag aufweisenden Protein oder Peptid um das gleiche Grundprotein oder Grundpeptid wie der Analyt, so besteht ein weiterer Vorteil darin, dass die affine Wechselwirkung von Analyt und von vom Analyt verschiedenem Molekül mit den Rezeptoren in der Regel oftmals in einer ähnlichen Größenordnung liegen.

Zur Bestimmung der mit der Menge der an den Rezeptoren gebundenen Analytmolekülen oder weiteren Zielmoleküle korrelierenden Messgröße kann ein Marker direkt oder indirekt an den an die Sensormatrix angebundenen Analyten oder an ein weiteres Zielmolekül angebunden werden. Der Marker dient direkt oder indirekt zur Einbringung einer die Messgröße beeinflussenden Eigenschaft. Er kann insbesondere an ein einen Protein-Tag aufweisendes Zielmolekül über den Protein-Tag angebunden werden. Dies kann beispielsweise durch Durchleiten einer Lösung des Markers oder einer den Marker als funktionelle Gruppe tragenden chemischen Spezies durch den Messkanal bewirkt werden, wobei der Marker bzw. die den Marker tragende chemische Spezies eine an den Analyten, das weitere Zielmolekül oder an den Protein-Tag eines Zielmoleküls bindende funktionelle Gruppe aufweist. Der Marker bindet im letzteren Fall spezifisch an die an der Rezeptorschicht angebundenen, einen Protein-Tag aufweisenden Zielmoleküle, und nicht an gleichfalls an der Rezeptorschicht angebundene Analytmoleküle. Alternativ ist es auch möglich, die Zielmoleküle in einem vorgelagerten Verfahrensschritt mit dem Marker zu markieren, so dass bereits mit dem Marker markierte Zielmoleküle durch den Messkanal geleitet werden. Die Verwendung von Proteingetaggten Zielmolekülen, vorzugsweise als Kompetitor, beinhaltet gegenüber bekannten Verfahren in der Proteinanalytik den großen Vorteil, dass für die Bestimmung unterschiedlicher Analyte lediglich ein Marker, welcher direkt oder indirekt an den Protein-Tag des Zielmoleküls bindet, notwendig ist, oder der Protein-Tag selbst den Marker darstellt. So bietet beispielsweise die Verwendung eines Grün-Fluoreszierenden Proteins (GFP) als als Marker wirkender Protein-Tag den Vorteil, dass dieses als Messgröße seine Fluoreszenz einbringt, die direkt mittels eines optischen Signalwandlers bestimmt und/oder aber durch nachfolgende Anbindung eines beispielsweise Enzym-konjugierten, gegen GFP gerichteten Antikörpers, indirekt bestimmt werden kann.

Als direkt angebundene Marker eignen sich neben GFP auch andere fluoreszierende Substanzen, wie z.B. fluoreszierende Nanoteilchen. Indirekt angebundene Marker können vorteilhafterweise über affine Wechselwirkung an ein Zielmolekül bzw. den Analyten angebunden werden. In diesem Fall eigenen sich besonders gut die beschriebenen Protein-Tags als universelle Anbindungsstruktur zur nachfolgenden direkten oder indirekten Anbindung des Markers. Als Marker kommen neben chemischen Spezies, die selbst, beispielsweise aufgrund bestimmter Fluoreszenz-oder Absorptionseigenschaften, detektierbar sind, auch solche chemischen Spezies in Frage, die in Gegenwart anderer chemischer Spezies eine durch den Signalwandler, insbesondere optisch, detektierbare Eigenschaft des Messsystems beeinflussen. Ein Beispiel hierfür sind Marker, die chemische Reaktionen eingehen, durch welche ein detektierbares optisches Signal entsteht, insbesondere durch Chemolumineszenz oder eine Farbänderung. Beispielsweise kommt hierfür Peroxidase als Marker in Frage, die in Gegenwart einer Lösung von Luminol/H₂O₂ eine durch die Peroxidase katalysierte Chemolumineszenz bewirkt.

Ein Bioanalysator, der erfindungsgemäß zur Durchführung des Verfahrens nach einer der voranstehend beschriebenen Ausgestaltungen verwendet wird, umfasst eine Mikrofluidikeinrichtung mit mindestens einem Messkanal und Mittel zum Durchleiten von Flüssigkeit durch den Messkanal, wobei der Messkanal mindestens ein Substrat umfasst, auf dem mindestens innerhalb eines Messbereichs mindestens zeitweise eine Sensormatrix mit einer Vielzahl von Rezeptoren angeordnet ist, welche in einer Flüssigkeitsprobe oder einer durch Behandeln der Flüssigkeitsprobe mit mindestens einem Reagenz erhaltenen Messflüssigkeit vorhandene Zielmoleküle oder Analyte vorzugsweise selektiv und spezifisch binden oder chemisch umsetzen,
wobei die Sensormatrix gebildet ist durch eine oder mehrere an das Substrat angebundene Anbindungsschichten und eine abschließende, an der obersten Anbindungsschicht gebundene Rezeptorschicht, wobei eine erste Anbindungsschicht durch eine Vielzahl von eine erste, an das Substrat gebundene funktionelle Gruppe aufweisenden ersten chemischen Spezies gebildet ist, und wobei die ersten chemischen Spezies eine weitere funktionelle Gruppen aufweisen, an welche die Rezeptoren durch spezifische Anbindung direkt oder indirekt über eine oder mehrere weitere Anbindungsschichten angebunden sind, so dass die Sensormatrix aus einer oder mehreren Anbindungsschichten und einer abschließenden, an der obersten Anbindungsschicht gebundenen Rezeptorschicht gebildet wird; wobei der Bioanalysator mindestens einen Signalwandler umfasst, welcher ein von einer Menge von an die Rezeptoren gebundenem oder von diesen umgesetztem Analyten oder ein von einer Menge von an die Rezeptoren gebundenen oder umgesetzten Zielmolekülen abhängiges Messsignal ausgibt, und
wobei das Substrat elektrisch leitfähig ist und innerhalb der Mikrofluidikeinrichtung eine zumindest während des Freilegens mit dem Substrat in leitendem Kontakt stehende, insbesondere innerhalb des Messkanals dem ersten Substrat gegenüberliegend oder koaxial zum Substrat ausgerichtet angeordnete und bevorzugt als weiteres Substrat dienende, Gegenelektrode angeordnet ist, und wobei der Bioanalysator weiterhin Mittel zum Erzeugen eines Stromflusses zwischen dem Substrat und der Gegenelektrode umfasst.

Die Menge des gebundenen Analyten bzw. der gebundenen Zielmoleküle entspricht der Anzahl der an die Rezeptoren gebundenen Analytmoleküle bzw. Zielmoleküle.

Innerhalb der Mikrofluidikeinrichtung ist die Gegenelektrode angeordnet , welche insbesondere innerhalb des Messkanals dem ersten Substrat gegenüberliegend angeordnet ist und bevorzugt als weiteres Substrat dient. Dient die Gegenelektrode als weiteres Substrat, dann ist auch auf diesem weiteren Substrat mindestens zeitweise eine Sensormatrix mit einer Vielzahl von Rezeptoren angeordnet. Die Präparation der Sensormatrix und das Freilegen des Substrats und der als weiteres Substrat dienenden Gegenelektrode kann, wie voranstehend im Zusammenhang mit dem Verfahren zur automatisierten *in situ* Bestimmung eines Analytgehalts einer Flüssigkeitsprobe beschrieben, erfolgen.

Das Substrat und die Gegenelektrode können bezüglich der Flussrichtung des den Messkanal durchströmenden Elektrolyten derart angeordnet sein, dass aufgrund eines Stromflusses zwischen dem Substrat und der Gegenelektrode im Elektrolyten entstehende Gasblasen so geleitet werden, dass sie den elektrischen Stromfluss nicht wesentlich beeinflussen. Die Gegenelektrode kann als elektrisch leitfähige Beschichtung eines Kanals der Mikrofluidikeinrichtung bzw. als Film ausgestaltet sein, der insbesondere vom Messkanal verschieden, aber mit diesem verbunden sein kann. Sie kann bevorzugt innerhalb des Messkanals, beispielsweise dem Substrat gegenüberliegend oder koaxial zu diesem angeordnet, angeordnet sein. Der Bioanalysator umfasst weiterhin eine innerhalb der Mikrofluidikeinrichtung gebildete Referenzelektrode, welche über einen, insbesondere diaphragmenlosen, Stromschlüssel in elektrisch leitfähiger Verbindung mit mindestens einem Substrat steht. Das Substrat, die Gegenelektrode und gegebenenfalls die Referenzelektrode können als elektrisch leitfähige, konnektierbare Filme, insbesondere als Metallbeschichtungen auf der Wand des jeweiligen Kanals der Mikrofluidikeinrichtung, in der sie angeordnet sind, ausgestaltet sein.

Die Mikrofluidikeinrichtung kann ein erstes Bauteil umfassen, welches eine Fläche aufweist, in der der Messkanal und gegebenenfalls weitere, insbesondere über Verzweigungen, mit dem Messkanal verbundene Kanäle zum Flüssigkeitstransport als Vertiefungen ausgebildet sind,
wobei mindestens ein zweites Bauteil mit dem ersten Bauteil verbunden ist und die Vertiefungen überdeckt und flüssigkeitsdicht zur Umgebung hin abschließt. Die so gebildeten Flüssigkeitsleitungen verlaufen zwischen einem oder mehreren Flüssigkeitsreservoiren aus denen Flüssigkeiten in die Mikrofluidikeinrichtung eingeleitet werden können, und Abfallbehältern, in die Flüssigkeiten aus der Mikrofluidik-Einheit ausgeleitet werden können. Das erste und zweite Bauteil können insbesondere aus einem elektrisch isolierenden, vorzugsweise transparenten Material, z.B. aus Glas oder einem Kunststoff, bestehen. Das Substrat, die Gegenelektrode und gegebenenfalls die Referenzelektrode können als elektrisch leitfähige, konnektierbare Filme, insbesondere als Metallbeschichtungen, auf dem zweiten Bauteil gebildet sein.

Die Mikrofluidikeinrichtung kann in einer alternativen Ausgestaltung ein erstes Bauteil, insbesondere eine planparallele erste Platte, und ein von dem ersten Bauteil beabstandet angeordnetes zweites Bauteil, insbesondere eine planparallele zweite Platte, umfassen, wobei das erste und das zweite Bauteil über ein oder mehrere zwischen dem ersten und dem zweiten Bauteil angeordnete Abstandselemente, insbesondere eine oder mehrere, Zwischenplatten, miteinander verbunden sind, wobei das oder die Abstandselemente Zwischenräume und/oder Durchbrüche aufweisen, welche eine zwischen dem ersten und dem zweiten Bauteil verlaufende Kanalstruktur, mit dem Messkanal und gegebenenfalls weiteren, mit dem Messkanal verbundenen Kanälen zum Flüssigkeitstransport bilden. Das erste und zweite Bauteil bestehen insbesondere aus einem elektrisch isolierenden, vorzugsweise transparenten Material, z.B. aus Glas oder einem Kunststoff. Die eine oder mehreren Zwischenplatten können beispielsweise elastisch sein, sie können beispielsweise als Silikon-Matten ausgestaltet sein. Das erste und das zweite Bauteil können beispielsweise als Glasplatten ausgebildet sein. Vorteilhaft ist hier die Verwendung von Objektträgern. Das Substrat, die Gegenelektrode und gegebenenfalls die Referenzelektrode können in dieser Ausgestaltung als elektrisch leitfähige, konnektierbare Filme, insbesondere als Metallbeschichtungen, auf dem ersten und/oder dem zweiten Bauteil gebildet sein.

In einer weiteren Ausgestaltung können als Substrat, als Gegenelektrode und gegebenenfalls als Bezugselektrode auch elektrisch leitfähige, mikrofluidische Röhrchen verwendet werden. So kann als Substrat beispielsweise ein Gold-Röhrchen verwendet werden, als Gegenelektrode ein Platin-Röhrchen und als Referenzelektrode ein im Innern mit Silberchlorid ausgekleidetes Silber-Röhrchen. Vorteile bei dieser Art der Ausgestaltung liegen in der einfachen elektrischen und mikrofluidischen Konnektierbarkeit dieser Röhrchen. Eine vorteilhafte Variante hierbei besteht aus einem Messkanal, dessen Messbereich aus einem, mindestens im Inneren elektrisch leitfähigen Röhrchen, beispielsweise aus Platin, in dessen Innern koaxial und ohne direkten Kontakt zu dem Röhrchen ein Draht eingebracht ist, der zumindest auf der Oberfläche elektrisch leitend beschichtet ist, z.B. ein Platin-Draht. Das Röhrchen kann hierbei als Substrat und der Draht als Gegenelektrode und gegebenenfalls als weiteres Substrat dienen. Auch die umgekehrte Funktionalität ist möglich. Die koaxiale Anordnung von Substrat und Gegenelektrode erlaubt durch den kleinen Abstand zwischen Substrat und Gegenelektrode hohe Stromdichten, was eine lange Verwendung auch bei geringfügiger Abtragung der Oberfläche des Substrats und/oder der Gegenelektrode bei der elektrischen Regenerierung bzw. Reinigung ermöglicht.
Für optische Messungen ist der Messkanal im Bereich des Messbereiches vorzugsweise so ausgestaltet, dass es Licht im Wellenlängenbereich zwischen 300 nm und 900 nm zu mindestens 10% transmittiert. Dies ist beispielsweise der Fall bei einer Ausgestaltung des Substrats als dünner Platinschicht, deren Dicke zwischen 2 und 30 nm beträgt oder bei einem strukturiert, beispielsweise als Linien-oder Gitterstruktur, auf ein transparentes Material aufgebrachten, opaken Substrat.

In einer bevorzugten Ausgestaltung ist die Mikrofluidik-Einheit bzw. sind gegebenenfalls mehrere Mikrofluidik-Einheiten zusammen mit Behältern zur Bevorratung von Reagenzien bzw. Reagenzienlösungen und zur Aufnahme von Flüssigkeitsabfällen in einer auswechselbaren Einheit des Bioanalysators, einer sogenannten Wechselkassette, angeordnet. Die Behälter sind innerhalb der Wechselkassette mittels mikrofluidischer Verbindungssysteme mit der oder den Mikrofluidik-Einheiten zur Zuleitung der Reagenzien in die Mikrofluidik-Einheit oder-Einheiten bzw. zur Ableitung von verbrauchten Flüssigkeiten in die Behälter verbunden. Der Transport der Flüssigkeiten durch eine Mikrofluidik-Einheit erfolgt bevorzugt indirekt, z.B. pneumatisch, so dass neben einer Leitung zur Zuführung der zu analysierenden Flüssigkeitsprobe keine weiteren flüssigkeitsführenden Leitungen an die Wechselkassette anzuschließen sind. Dies ermöglicht einen einfachen und anwenderfreundlichen Betrieb des Bioanalysators. Nach dem Einsetzen einer solchen, mit den benötigten Reagenzien bzw. Reagenzienlösungen befüllten Wechselkassette in den Bioanalysator werden in einem initialen Schritt die fluidischen Leitungen und Kanäle mit Flüssigkeit gefüllt. Hiernach können *in situ* und in direkter Abfolge wiederholt die Schritte i bis iii zur Durchführung von Messungen mit Hilfe des Bioanalysators durchgeführt werden. Flüssigkeitsproben zur Analyse können einem zu überwachenden Prozessmedium mittels eines automatischen Probennahmesystem entnommen, und der Wechselkassette bzw. einer in der Wechselkassette enthaltenen Mikrofluidik-Einheit über einen Anschluss der Wechselkassette an das Probennahmesystem zugeführt werden. Auf diese Weise können mit Hilfe dieses on-line Bioanalysators voll automatisiert Messungen durchgeführt werden. Nach Verbrauch der bevorrateten und für den Messbetrieb notwendigen Reagenzien bzw. Reagenzienlösungen oder nach vollständiger Befüllung der Behälter zur Aufnahme von Flüssigkeitsabfällen ist die Wechselkassette durch eine neue auszutauschen oder aufzubereiten.

Die Erfindung wird im Folgenden anhand der in den Zeichnungen dargestellten Ausführungsbeispiele im Detail beschrieben. Es zeigen:
- Fig. 1: a) eine erste schematische Darstellung einer Rezeptorschicht mit angebundenen Analyt- und Zielmolekülen;
b) eine zweite schematische Darstellung einer Rezeptorschicht mit angebundenen Analyt- und Zielmolekülen;
- Fig. 2: eine schematische Darstellung einer Mikrofluidik-Einheit eines Bioanalysators in Aufsicht;
- Fig. 3: eine schematische Längsschnitt-Darstellung der Mikrofluidik-Einheit aus Fig. 2;
- Fig. 4: a) eine schematische Darstellung der Mikrofluidik-Einheit aus Fig. 2 vor einer elektrochemischen Reinigung in Aufsicht;
b) eine schematische Darstellung der Mikrofluidik-Einheit aus Fig. 2 während der elektrochemischen Reinigung in Aufsicht;
- Fig. 5: eine schematische Darstellung einer zweiten Ausgestaltung einer Mikrofluidik-Einheit eines Bioanalysators, bestehend aus einem Verbund eines ersten Bauteils, einer den Kanal bildenden durchbrochenen Zwischenplatte und eines zweiten Bauteils:
a) eine schematische Darstellung des ersten Bauteils in Aufsicht
b) eine schematische Darstellung einer Kanäle bildenden, durchbrochenen Zwischenplatte in Aufsicht
c) eine schematische Darstellung eines zweiten Bauteils in Aufsicht;
- Fig. 6: eine schematische Darstellung einer dritten Ausgestaltung einer Mikrofluidik-Einheit eines Bioanalysators:
a) eine schematische Längsschnitt-Darstellung entlang der Linie C-C in Fig. 6 b)
b) eine schematische Querschnitt-Darstellung entlang der Linie B-B in Fig. 6 a).

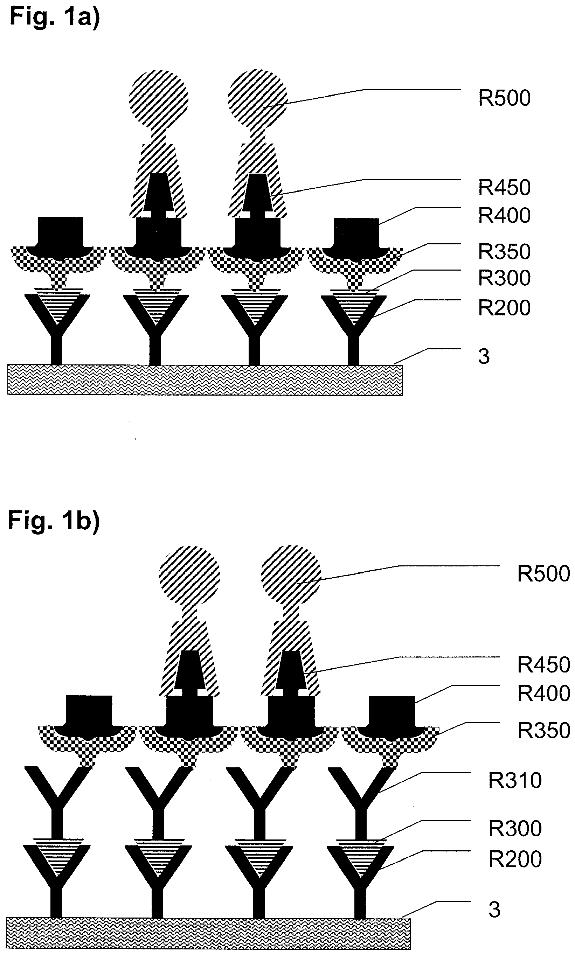

In Fig. 1 a) ist eine schematische Darstellung einer regenerierbaren Sensormatrix eines Bioanalysators mit Rezeptoren R350 und an diese gebundenen Analytmolekülen R400 und ebenfalls an die Rezeptoren R350 gebundenen weiteren Zielmolekülen R450 gezeigt. Als Substrat 3 für die Sensormatrix dient eine Thiolgruppen bindende elektrisch leitfähige Oberfläche. Diese kann beispielsweise aus einer Gold- oder Platinoberfläche, die beispielsweise durch eine Beschichtung, einen Film oder ein massives Metallstück, z.B. ein Röhrchen, gebildet ist, bestehen. Platin und Gold verfügen über eine hohe Thiophilie und haben weiterhin den Vorteil, dass sie chemisch stabil sind, und beim weiter unten im Detail beschriebenen elektrochemischen Auflösen der Rezeptorschicht und der Präparation einer neuen Rezeptorschicht chemisch nicht verändert werden, insbesondere nicht oxidieren.

Auf dem Substrat 3 ist eine Schicht von als Anbindungsmoleküle R200 für die Rezeptoren R350 dienenden ersten chemischen Spezies angeordnet. Als Anbindungsmoleküle R200 zur Bildung einer Anbindungsschicht auf dem Substrat 3 können, wenn es sich bei diesem um eine thiophile Oberfläche wie beispielsweise Gold- oder Platinoberfläche handelt, beispielsweise eine weitere funktionelle Gruppe aufweisende Alkylthiole dienen, deren Thiolgruppen mit der Gold- oder Platinoberfläche eine kovalente Bindung ausbilden. Ein weiteres Beispiel für Anbindungsmoleküle R200 ist α-Mercapto-ω-Biotin-Polyethylenglykolen (kurz auch als Thiol-PEG-Biotin bezeichnet). Dieses kann aus wässriger Lösung über eine Metall-Schwefel-Bindung an das Substrat 3 angebunden werden. Durch optionalen Zusatz von Mercapto-Polyethylenglykol, welches nur eine an das Substrat 3 bindende Thiolgruppe, nicht aber eine weitere funktionelle Gruppe zur Anbindung des Rezeptors R350 aufweist, kann zum einen die Oberflächendichte an Biotin-Gruppen eingestellt werden. Zum anderen verhindert das eingebrachte Polyethylenglykol die unspezifische Adsorption von nachfolgend zugegebenen Proteinen an die Substratoberfläche.

Die Anbindungsmoleküle R200 weisen weiterhin mindestens eine weitere funktionelle Gruppe auf, die als Bindungsstelle für die spezifische Anbindung eines als funktionelle Gruppe an den Rezeptor R350 gebundenen komplementären Moleküls dient. Im vorliegenden Beispiel ist die weitere funktionelle Gruppe der Anbindungsmoleküle R200 durch eine Biotin-Gruppe der Alkylthiole gebildet. Dient α-Mercapto-ω-Biotin-Polyethylenglykol als Anbindungsmolekül R200, dient ebenfalls dessen Biotin-Gruppe als funktionelle Gruppe zur spezifischen direkten oder indirekten Anbindung des Rezeptors R350. Der Rezeptor R350 ist im vorliegenden Beispiel an ein an die Anbindungsmoleküle R200 vorzugsweise selektiv und spezifisch bindendes Molekül R300 gebunden, das im vorliegenden Beispiel ein Biotin-bindendes Molekül, z.B. Streptavidin ist. Die Anbindungsmoleküle R200 und das an die weitere funktionelle Gruppe der Anbindungsmoleküle R200 vorzugsweise selektiv und spezifisch bindende Molekül R300, über das der Rezeptor R350 an die Anbindungsmoleküle R200 gebunden wird, bildet eine universelle Schnittstelle zur Bindung einer großen Auswahl von Rezeptoren R350 an die gleichen Anbindungsmoleküle R200 und damit zur (indirekten) Bindung einer Vielzahl unterschiedlicher Rezeptoren an das Substrat 3. Die Biotin-Streptavidin-Verbindung zur Bindung des Rezeptors R350 an das Anbindungsmolekül R200 in dem hier gezeigten Beispiel ist als universelle Schnittstelle gut geeignet, da die Biotinylierung bzw. die Markierung von Molekülen mit Streptavidin eine in der Biotechnologie gängige Technik ist. Speziell Biotin-funktionalisierte Antikörper sind beispielsweise für die Durchführung von Immunoassays weit verbreitet.

An die Rezeptoren R350 sind im in Fig. 1 a) gezeigten Beispiel Analytmoleküle R400 und mit den Analytmolekülen R400 um die Bindung an die Rezeptoren R350 kompetierende und vom Bioanalysator zu detektierende weitere Zielmoleküle R450 gebunden. Die weiteren Zielmoleküle R450 sind wie der Analyt R400 in der Lage, vorzugsweise selektiv und spezifisch durch affine Wechselwirkung an die Rezeptoren R350 zu binden. Bei dem Analyten R400 kann es sich beispielsweise um ein Protein handeln. Die Proteinmoleküle binden entsprechend jeweils vorzugsweise selektiv und spezifisch an die Rezeptoren R350. In diesem Fall sind die weiteren Zielmoleküle R450 vorzugsweise ebenfalls Proteinmoleküle, die ebenfalls vorzugsweise selektiv und spezifisch, insbesondere durch affine Wechselwirkung, an die Rezeptoren R350 binden. Insbesondere können die weiteren Zielmoleküle R450 markierte oder nachfolgend markierbare Proteinmoleküle sein. Die weiteren Zielmoleküle R450 sind im vorliegenden Beispiel mit einem Protein-Tag versehen. Ein Protein-Tag ist eine an das eigentliche Protein gebundene Aminosäuresequenz. Protein-Tags dienen beispielsweise in der Affinitätschromatographie zur Aufreinigung eines in einem biotechnologischen Verfahren hergestellten Zielproteins dazu, durch spezifische Wechselwirkung des an das Zielproteine gebundenen Protein-Tags mit einem auf die Chromatographie-Säule aufgebrachten Bindungspartner das Zielprotein von den übrigen Verfahrensprodukten zu trennen. Der Protein-Tag dient im vorliegenden Beispiel dazu, ein einen Marker tragendes Molekül R500 direkt oder indirekt anzubinden. Der Marker besitzt physikalische oder chemische Eigenschaften, anhand derer der Signalwandler des Bioanalysators ein Messsignal erzeugen kann, das mit der Anzahl der an die Rezeptoren R350 gebundenen weiteren Zielmoleküle R450 und indirekt mit der Anzahl der an die Rezeptoren R350 gebundenen Analytmolekülen korreliert ist. Die Marker tragenden Moleküle R500 umfassen dazu eine funktionelle Gruppe, die aufgrund einer affinen Wechselwirkung spezifisch an die Zielmoleküle R450, bevorzugt an deren Protein-Tag, bindet.

Handelt es sich beispielsweise bei dem in einer Flüssigkeitsprobe vorliegenden Analyten R400 um das Protein Pfs, so kann als Rezeptor R350 ein entsprechender Antikörper (Anti-Pfs) dienen. Zur Bestimmung des Analytgehalts der Flüssigkeitsprobe in einem kompetitiven Verfahren wird die Flüssigkeitsprobe mit einem als weiteres Zielmolekül R450 dienenden Kompetitor, im vorliegenden Fall z.B. rekombinant hergestelltes, mit einem Hämagglutinin-Tag (HA-Tag) versehenes Pfs (HA-Pfs) zugegeben und so eine für die Analyse durch den Bioanalysator vorgesehene Messflüssigkeit erhalten. Das Marker tragende Molekül R500 kann beispielsweise ein Antikörper gegen den HA-Tag sein, welcher mit einer Peroxidase konjugiert ist. Eine mittels Zugabe einer Lösung von Luminol/H₂O₂ katalysiert durch den Marker Peroxidase bewirkte Chemolumineszenz wird durch einen optischen Signalwandler, der insbesondere eine Photodiode umfasst, in ein elektrisches Messsignal gewandelt, dessen Signalstärke von der Chemolumineszenz-Intensität abhängt. Diese hängt wiederum mit der Anzahl der an die Zielmoleküle R450 gebundenen Marker tragenden Moleküle R500 ab. Aus dem mittels des Signalwandlers erzeugten Messsignal kann mithin auf die Anzahl der an der Rezeptorschicht gebundenen Zielmoleküle R450 und entsprechend auf die Konzentration der Analytmoleküle R400 in der Messflüssigkeit geschlossen werden. Das Messsignal kann durch eine Messschaltung und/oder eine elektronische Datenverarbeitungseinheit des Bioanalysators verarbeitet und daraus die Konzentration des Analyten in der Messflüssigkeit bzw. der Flüssigkeitsprobe abgeleitet werden.

Um zu demonstrieren, dass mit einer solchen universellen Anbindungsstruktur aus Anbindungsmolekülen R200 und daran über ein an die Anbindungsmoleküle R200 vorzugsweise selektiv und spezifisch bindende, an die Rezeptoren R350 gebundene Molekülen R300 auch vollständig andere Immunoassays realisiert werden können, ist nachfolgend ein weiteres Beispiel mit Bezug auf Fig. 1 a) angegeben, bei dem Carbamazepin in einer Flüssigkeitsprobe nachgewiesen werden soll. Wie im vorigen Beispiel wird die universelle Schnittstelle zur Anbindung der Rezeptoren aus an dem Substrat 3 über eine Vielzahl von Anbindungsmolekülen R200 immobilisierte Biotin-Gruppen und daran bindende Neutravidin-oder Streptavidin-Moleküle R300 gebildet. Hieran werden biotinylierte Antikörper R350 gegen den Wirkstoff Carbamazepin angebunden. Nachfolgend wird über die so gebildete Sensormatrix eine Messflüssigkeit aus einer mit einem Peroxidase-konjugierten Carbamazepin-Derivat versetzten Flüssigkeitsprobe geleitet. Das in der Flüssigkeitsprobe ursprünglich enthaltene Carbamazepin bindet hierbei als Analyt R400 an die Rezeptoren und konkurriert hierbei mit der in bekannter Konzentration vorliegenden Peroxidase-konjugierten Carbamazepin-Molekülen, welche als vorab hergestelltes Konjugat sowohl die Funktionalität der Zielmoleküle R450 als auch des Marker tragenden Moleküls R500 beinhalten. Alternativ ist es auch möglich, als Kompetitor an den HA-Tag kovalent gebundenes Carbamazepin einzusetzen. Dies hat gegenüber der Verwendung eines Peroxidase-konjugierten Carbamazepin-Derivats den Vorteil, dass der HA-Tag ein wesentlich geringeres Molekulargewicht als die Peroxidase besitzt, so dass das Größenverhältnis zwischen Analyt und Kompetitor möglichst gering wird. Weisen Analyt und Kompetitor sehr unterschiedliche Größen auf, kann dies zu stark unterschiedlichen Bindungsaffinitäten von Analyt und Kompetitor führen, was häufig unerwünscht ist. Speziell bei niedermolekularen Analyten wie Carbameazepin führt eine Konjugation mit einem so hochmolekularen Analyten, wie es das Enzym Peroxidase darstellt, zu deutlich veränderten Bindungsaffinitäten, was sich negativ auf die untere Nachweis- und Bestimmungsgrenze auswirkt bzw. Messfehler vergrößert.

Eine weitere universelle Schichtstruktur zur Bindung von Rezeptoren R350 ist in Fig. 1 b) dargestellt. Hier kommt der in Fig. 1 a) dargestellte Schichtaufbau aus einem Substrat 3, einer Anbindungsschicht aus einer Vielzahl von Anbindungsmolekülen R200 und an eine funktionelle Gruppe der Anbindungsmoleküle R200 vorzugsweise selektiv und spezifisch bindenden Molekülen R300 zur Anbindung der Rezeptoren R350 ebenfalls zum Einsatz, wird jedoch um eine weitere Anbindungsschicht erweitert, an die auch nicht-biotinylierte Rezeptoren R350 in vielfältiger Art und Weise angebunden werden können. Diese zusätzliche Anbindungsschicht besteht in dem hier gegebenen Beispiel aus einem biotinylierten Immunoglobulin-bindenden Molekül R310, z.B. einem biotinylierten Antikörper gegen Immunoglobuline vom Typ IgG (Biotin-Anti-IgG) oder aus einem biotinylierten Protein A/G. Hieran können nachfolgend zahlreiche unterschiedliche, bislang in einmal verwendbaren immunologischen Testverfahren verwendete Antikörper als Rezeptoren angebunden werden, ohne dass hierbei die darunterliegenden Schichten oder das Verfahren zu deren Aufbau verändert werden müssten.

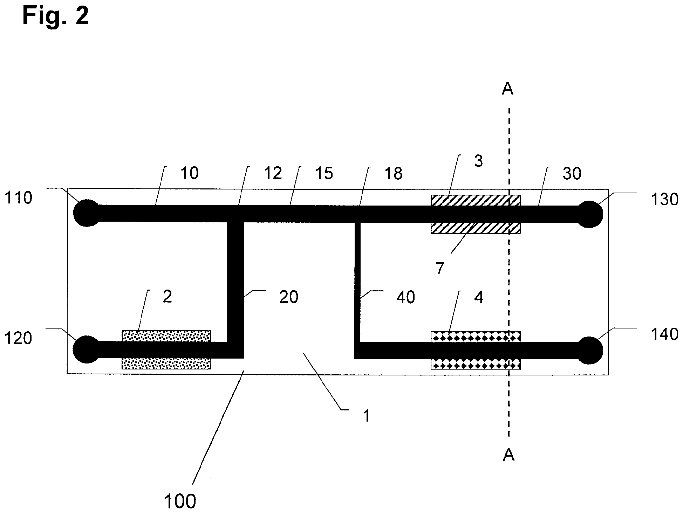

Fig. 2 zeigt eine Mikrofluidikeinrichtung 100 eines Bioanalysators zum Nachweis bzw. zur Bestimmung einer Konzentration eines Analyten in einer Flüssigkeitsprobe in Aufsicht. Die Mikrofluidikeinrichtung 100 weist einen Grundkörper 1a, beispielsweise aus einem transparenten Kunststoff, auf, der an einer Basisfläche eine durch Vertiefungen in dem Grundkörper 1a gebildete offene Kanalstruktur mit Kanalabschnitten 10, 15, 20, 30, 40 und Verzweigungen 12, 18 besitzt. Auf dem Grundkörper 1a liegt eine die Kanalabschnitte 10, 15, 20, 30, 40 dicht gegeneinander und gegen die Umgebung abschließende Deckplatte 1b auf, die beispielsweise ebenfalls aus einem transparenten Kunststoff, gebildet ist. Dies ist in der in Fig. 3 gezeigten Querschnittdarstellung entlang der Linie A-A zu sehen. Der Grundkörper 1a und die Deckplatte 1b sind durch eine formkraft- oder stoffschlüssige Verbindung 1c, beispielsweise eine Verklebung oder eine Verpressung, miteinander fest verbunden. Die durch die Deckplatte 1b dicht verschlossenen Kanalabschnitte 10, 15, 20, 30, 40 bilden Flüssigkeitsleitungen, durch die zur Durchführung einer Messung die zu analysierende Flüssigkeitsprobe und gegebenenfalls weitere Reagenzien geleitet werden.

Der Grundkörper 1a weist außerdem mit Flüssigkeitszuleitungen verbindbare Anschlüsse 110, 120 und mit Flüssigkeitsableitungen verbindbare Anschlüsse 130, 140 auf, durch die Flüssigkeiten der Kanalstruktur zugeführt bzw. wieder aus dieser abgeleitet werden können. Die Zu- und Ableitungen verbinden die Mikrofluidikeinrichtung 100 mit beabstandet angeordneten Flüssigkeitsreservoiren, in denen Flüssigkeiten zur Durchführung einer Messung bzw. einer Analyse aufgenommen sind. Über die Zu- und Ableitungen sind sowohl die zu untersuchende Flüssigkeitsprobe als auch gegebenenfalls der Flüssigkeitsprobe zuzusetzende, in den Flüssigkeitsreservoiren aufgenommene Reagenzien der Mikrofluidikeinrichung 100 zuführbar. Die Zuführung und das Durchleiten der Flüssigkeiten durch die Kanalstruktur der Mikrofluidikeinrichtung 100 kann beispielsweise mittels Pumpen oder pneumatisch erfolgen.

Alternativ kann die Kanalstruktur, wie sie Fig. 2 zeigt, auch als Grundstruktur eines mikrofluidischen Systems angesehen werden, die unabhängig von der konkreten Ausgestaltung der Flüssigkeitsleitungen ist. So kann die gezeigte Struktur beispielsweise auch aus mikrofluidischen Schläuchen und/oder Röhrchen bestehen. Die Flüssigkeitsleitungen weisen vorzugsweise einen Querschnitt zwischen 10⁷ und 1 µm², bevorzugt zwischen 10⁶ und 10² µm², weiter bevorzugt zwischen 10⁵ und 10⁴ µm² auf.

Ein durch einen Kanalabschnitt der Mikrofluidikeinrichtung 100 gebildeter Messkanal 30 weist das bereits anhand von Fig. 1 beschriebene, als elektrisch leitfähiger Oberflächenbereich des Messkanals gestaltete Substrat 3 auf. Das Substrat 3 kann beispielsweise als ein auf der Deckplatte 1b aufgebrachter Metallfilm, beispielsweise als Gold- oder Platinfilm, ausgestaltet sein. Der mit im Messkanal 30 strömender Flüssigkeit in Berührung kommende Bereich des Substrats dient als Messbereich 7, in dem die Sensormatrix aufgebaut wird und gegebenenfalls in einer durch den Messkanal 30 geleiteten Flüssigkeitsprobe oder Messflüssigkeit enthaltener Analyt an die Sensormatrix angebunden und mittels eines, insbesondere optischen, Signalwandlers detektiert wird.

Ein weiterer Kanalabschnitt 40, der mit dem Messkanal 30 über eine Verzweigung 18 verbunden ist, weist einen als Gegenelektrode 4 dienenden weiteren elektrisch leitfähigen Oberflächenbereich auf, der beispielsweise als auf der Deckplatte aufgebrachter Metallfilm gebildet ist. Sowohl das Substrat 3 als auch die Gegenelektrode 4 sind über nicht weiter dargestellte elektrische Anschlüsse mit einer Spannungsquelle, einem Potentiostaten oder einem Galvanostaten, verbunden. Sind die Kanalabschnitte 30 und 40 von einem Elektrolyten durchflossen, stehen das Substrat 3 und die Gegenelektrode 4 über den Elektrolyten in elektrisch leitfähigem Kontakt. Durch Anlegen einer Potentialdifferenz zwischen Substrat 3 und Gegenelektrode 4 kann ein Stromfluss durch den Elektrolyten bewirkt werden.

Im hier gezeigten Beispiel ist in einem weiteren Kanalabschnitt 20 ein dritter elektrisch leitfähiger Oberflächenbereich angeordnet, der optional als Referenzelektrode 2 dienen kann. Die Referenzelektrode 2 ist ebenfalls als Metallschicht, insbesondere als Silberschicht zur Ausbildung einer Ag/AgCl-Referenzelektrode auf der Deckplatte 1b der Mikrofluidik-Einheit 100 ausgebildet, die mit einem elektrischen Anschluss, beispielsweise zum Anschluss an einen Potentiostaten oder Galvanostaten, verbunden sein kann.

Ein Verfahren zur Präparation der Sensormatrix und zum Nachweis bzw. zur Bestimmung einer Konzentration eines Analyten in einer Messlösung unter Verwendung der in Fig. 2 dargestellten Mikrofluidikeinrichtung und einer Sensormatrix mit Rezeptoren gemäß Fig. 1 wird im Folgenden beschrieben.

Zunächst wird eine Sensormatrix auf dem Substrat 3 präpariert, die eine Rezeptorschicht mit Rezeptoren R350 aufweist an die die zu bestimmenden Zielmoleküle R450 vorzugsweise selektiv und spezifisch binden. In einem ersten Schritt wird dazu eine erste Präparationslösung von eine Thiolgruppe und eine weitere funktionelle Gruppe aufweisenden Anbindungsmolekülen R200, z.B. von Alkylthiolen mit einer weiteren funktionellen Gruppe, durch den Messkanal 30 geleitet. Die Anbindungsmoleküle R200 werden kovalent über ihre Thiolgruppe an das Substrat 3 gebunden und bilden dort idealerweise eine Monoschicht aus. Die Präparationslösung wird bei vorzugsweise konstanter Durchflussrate über einen vorgegebenen ersten Zeitraum durch den Messkanal 30 geleitet, bis der Bedeckungsgrad der entstehenden Anbindungsschicht, wie er weiter oben definiert ist, sicher mehr als 80% beträgt. Der dazu erforderliche Zeitraum kann in Vorexperimenten bestimmt werden. Er hängt insbesondere von der Durchflussrate, der Art der Anbindungsmoleküle R200, der Konzentration der Präparationslösung, der Anbindungsdauer und von weiteren Umgebungsvariablen, beispielsweise der Temperatur, ab.

Wenn die Bildung der durch die Anbindungsmoleküle R200 gebildeten ersten Anbindungsschicht der Sensormatrix beendet ist, wird, nach einem oder mehreren Spülschritten (im Folgenden nicht explizit aufgeführt), in einem zweiten Schritt eine zweite Präparationslösung durch den Messkanal 30 geleitet, die die eigentlichen Rezeptoren R350 enthält, die an eine an die erste Anbindungsschicht vorzugsweise selektiv und spezifisch bindende funktionelle Gruppe R300 gebunden sind. Beispielsweise können die die erste Anbindungsschicht bildenden Anbindungsmoleküle R200 eine Biotin-Gruppe aufweisen, an die eine an den Rezeptor R350 gebundene Biotin-bindende Gruppe R300, beispielsweise eine Streptavidin-Gruppe, spezifisch bindet. Das Durchleiten der zweiten Präparationslösung wird über einen Zeitraum betrieben, der so bemessen ist, dass der Bedeckungsgrad der durch an die Anbindungsmoleküle R200 angebundenen Rezeptoren gebildete Rezeptorschicht einen wie weiter oben definierten Bedeckungsgrad von mindestens 80% aufweist. Der dazu erforderliche Zeitraum kann in Vorexperimenten bestimmt werden.

Die Präparation der Sensormatrix benötigt im vorliegenden Beispiel lediglich zwei Schritte, nämlich Bindung der Anbindungsmoleküle R200 an das Substrat 3 und die anschließende Bindung der Rezeptoren R350 über ihre funktionelle Gruppe R300 an die Anbindungsmoleküle R200. Es ist jedoch auch möglich, dass der Rezeptor R350 nicht an eine unmittelbar an die erste Anbindungsschicht spezifisch bindende funktionelle Gruppe gebunden ist, sondern erst über ein oder mehrere weitere Anbindungsmoleküle, und damit über ein oder mehrere weitere Anbindungsschichten an die erste Anbindungsschicht gebunden ist. Das weitere oder die weiteren Anbindungsmoleküle werden in einem oder mehreren weiteren Schritten durch Durchleiten einer entsprechenden Präparationslösung, die das jeweils anzubindende Bindungsmolekül enthält, an funktionelle Gruppen der jeweils zuletzt aufgebrachten Schicht angebunden. Dabei ergibt sich eine Schichtstruktur der Sensormatrix mit einer oder mehreren Anbindungsschichten und einer abschließenden Rezeptorschicht. Vorzugsweise weisen alle Schichten der Sensormatrix einen Bedeckungsgrad von mindestens 80% auf, wie bereits weiter oben erläutert.

Sind die Rezeptoren R350 an der Oberfläche aufgebracht, ist die Präparation der Sensormatrix beendet und der Nachweis eines Analyten oder die Bestimmung einer Konzentration des Analyten in einer Flüssigkeitsprobe oder in einer durch Behandlung der Flüssigkeitsprobe mit einem oder mehreren weiteren Reagenzien erhaltenen Messflüssigkeit kann durchgeführt werden.

Die einzelnen Verfahrensschritte zum Bestimmen einer Konzentration des Analyten hängen von dem jeweils durchzuführenden Nachweis ab. Beispielsweise kann der Analyt an den Rezeptor gebunden werden und die Anbindung direkt oder durch Anbindung eines weiteren Rezeptors an den an einem ersten Rezeptor immobilisierten Analyten (Sandwich-Testverfahren) nachgewiesen werden. Es kann statt des Analyten auch ein kompetitiv an den Rezeptor anbindendes Zielmolekül nachgewiesen werden, wie weiter oben bereits beschrieben. Die den Analyten enthaltende Messlösung kann auch zuvor mit einer weiteren Lösung (Reagenz) vermischt werden, die einen an den Analyten bindendes Zielmolekül enthält, so dass ein Analyt-Zielmolekül-Komplex gebildet wird. Das Gemisch wird dann durch den Messkanal geleitet und das verbleibende freie Zielmolekül an die Rezeptorschicht angebunden und nachgewiesen (Bindungshemmtest). Dem Fachmann ist eine Vielzahl weiterer Verfahren zum Nachweis oder zur Konzentrationsbestimmung eines Analyten durch Anbindung von Zielmolekülen an eine Rezeptorschicht bekannt, wie eingangs kurz umrissen. All diese Verfahren können durch entsprechendes Durchleiten einer Messlösung bzw. einer mit einer oder mehreren Reagenzienlösungen vermischten Messlösung durch den Messkanal 30 in dem Bioanalysator mit der Mikrofluidik-Einheit 100 oder der weiter unten beschriebenen Ausgestaltungen abgebildet werden.

Im vorliegenden Beispiel wird ein kompetitives Testverfahren durchgeführt. Dabei wird ein Gemisch der den Analyten R400 enthaltenden Messflüssigkeit mit einer Lösung, die als Kompetitor dienende weitere Zielmoleküle R450 bekannter Konzentration enthält, durch den Messkanal 30 geleitet. Die Zielmoleküle R450 kompetieren mit den Analytmolekülen R400 um die Bindung an die Rezeptoren R350. Wie zuvor beschrieben, sind die Zielmoleküle R450 mit einem Protein-Tag markierte Proteine, die bis auf den Tag im Wesentlichen identisch mit dem Analyten R400 sind.

Nach Bindung der Zielmoleküle, also des Analyten R400 und des weiteren Zielmoleküls R450 an die Rezeptorschicht wird in einem weiteren Schritt eine Lösung durch den Messkanal 30 geleitet, die Marker tragende Moleküle R500 enthält, die spezifisch an eine funktionelle Gruppe des Zielmoleküls R450, im vorliegenden Beispiel an den Protein-Tag des Zielmoleküls R450, binden. Bei dem Marker tragenden Molekül handelt es sich im vorliegenden Beispiel um ein Anti-HA-Antikörper-Peroxidase-Konjugat. Alternativ kann das den Marker tragende Molekül auch indirekt angebunden werden. So kann an die weiteren, den Protein-Tag beinhaltenden Zielmoleküle R450 beispielsweise in einem ersten Schritt ein biotinylierter Anti-HA-Antikörper angebunden werden, an den nachfolgend mit einem Fluoreszenzfarbstoff markiertes Streptavidin als Marker tragendes Molekül angebunden wird.

Handelt es sich bei dem Marker um eine Peroxidase, so wird in weiteren Schritt eine Luminol und H₂O₂ enthaltende Lösung durch den Messkanal 30 geleitet, was zur Emission von Lumineszenzstrahlung führt. Die emittierte Strahlung wird mittels eines (nicht dargestellten) photoelektrischen Signalwandlers, z.B. einer Photodiode, erfasst und in ein elektrisches Messsignal umgewandelt. Das Messsignal wird vom Signalwandler an eine Messschaltung ausgegeben, die eine Verstärkung und Verarbeitung des Messsignals durchführt, und das verarbeitete Messsignal an eine Datenverarbeitungseinrichtung, beispielsweise einen Mikrocomputer, ausgibt. Die Datenverarbeitungseinrichtung leitet anhand eines hinterlegten Auswertungsprogramms aus dem Messsignal eine Konzentration des Analyten in der Messflüssigkeit ab und gibt diese an eine Anzeigeeinrichtung und/oder über eine Datenleitung an eine übergeordnete Einheit aus. Die Datenverarbeitungseinrichtung kann Bestandteil einer Steuereinheit sein, die die Funktion des Bioanalysators steuert, Messsignale verarbeitet, speichert und ausgibt und Eingabebefehle einer Bedienperson oder einer übergeordneten Einheit zur Bedienung und/oder Steuerung des Biosanalysators verarbeitet und ausführt.

Je nachdem, welches Testverfahren zum Nachweis bzw. zur Konzentrationsbestimmung des Analyten in der Flüssigkeitsprobe bzw. Messflüssigkeit verwendet wird, können unterschiedliche Signalwandler zur Erzeugung eines mit der Anzahl der an die Rezeptorschicht gebundenen Zielmoleküle zum Einsatz kommen. Beispielsweise kann anstelle einer Chemolumineszenz zur Erkennung der Zielmoleküle auch eine Fluoreszenz angeregt werden. In diesem Fall wird Strahlung zur Anregung der Fluoreszenz in die Mikrofluidik-Einheit im Bereich der Rezeptorschicht eingestrahlt und emittierte Fluoreszenzstrahlung mittels eines photoelektrischen Signalwandlers, z.B. einer Photodiode erfasst. Zur Erkennung der Zielmoleküle kann auch eine Absorptionsmessung durchgeführt werden. In all diesen Fällen ist es vorteilhaft, die Messstrahlung, d.h. beispielsweise die Lumineszenz- oder Fluoreszenzstrahlung, entweder durch den transparenten Grundkörper 1a der Mikrofluidik-Einheit 100 oder die Deckplatte 1b der Mikrofluidik-Einheit 100 zu erfassen und ggfs. benötigte Anregungsstrahlung (für Fluoreszenz-oder Absorptionsmessungen) entsprechend durch den transparenten Grundkörper oder die Deckplatte einzustrahlen. Für den Fall, dass Strahlung durch die Deckplatte 1b eingestrahlt oder erfasst wird, ist es vorteilhaft, wenn das Substrat 3, auf dem die Sensormatrix aufgebracht ist, für Strahlung der verwendeten Wellenlänge, die in der Regel zwischen 300 und 900 nm liegt, transparent ist. Dies wird dadurch erreicht, dass die das Substrat 3 bildende leitfähige Beschichtung, beispielsweise die Gold-oder Platinbeschichtung, hinreichend dünn ausgebildet ist. Bevorzugt ist hier eine Schichtdicke von weniger als 100 nm, vorzugsweise zwischen 10 und 50 nm. Eine Gold- oder Platinschicht dieser Dicke ist für optische Messungen mit Messstrahlung im Wellenlängenbereich zwischen 300 und 900 nm hinreichend transparent. Um die erforderliche optische Transparenz im Bereich des Messbereichs zu erreichen, kann alternativ das Substrat auch als strukturierte Beschichtung aufgebracht werden, z.B. in Form einer Linien- oder Gitterstruktur mit unbeschichteten, und damit transparenten Zwischenräumen. Eine weitere Variante bei der Verwendung eines Enzyms als Marker und eines chromophoren Enzymsubstrats besteht darin, dem eigentlichen Messbereich nachfolgend einen transparenten Bereich nachzuschalten, beispielsweise vor dem Ausgang 130 im Bereich des Messkanals 30, in dem die Absorption der durch den Kanal fließenden Flüssigkeit gemessen werden kann. Neben Peroxidasen sind auch Phosphatasen und weitere Enzyme als Marker in der Bioanalytik weit verbreitet. Für diese stehen auch zahlreiche unterschiedliche Enzym-Substrate zur Verfügung.

Nach Beendigung der Messung ist für die Durchführung einer erneuten Messung eine Regenerierung der Rezeptorschicht möglich. Da das Ablösen der Analytmoleküle R400 bzw. der Zielmoleküle R450 oder das Ablösen der über die Bindungsgruppen R300 an die Anbindungsmoleküle R200 gebunden Rezeptoren R350 nicht vollständig in ausreichend reproduzierbarer Weise möglich ist, wird hierzu die gesamte Sensormatrix einschließlich der daran gebundenen Analytmoleküle R400 bzw. Zielmoleküle R450 einschließlich aller daran gebundenen weiteren Moleküle abgelöst, und so das Substrat 3 wieder freigelegt. Experimente haben gezeigt, dass die Anbindungsmoleküle R200 und die über die Bindungsgruppen R300 angebundenen Rezeptoren R350 ein auf molekularer Ebene kovalent oder nicht-kovalent verbundenes Netzwerk bilden, das sich durch eine rein chemische Reinigung, bei der eine Reinigungsflüssigkeit wie beispielsweise eine Säure, eine oxidierende Säure, eine Lauge oder ein organisches Lösungsmittel, durch den Messkanal 30 geleitet wird, auch bei erhöhter Temperatur nur schwer bzw. in langwierigen Reinigungsverfahren vollständig auflösen lässt.

Zur vollständigen Entfernung der Sensormatrix und der daran gebundenen Moleküle hat sich ein elektrochemisches Reinigungsverfahren als zweckmäßig erwiesen, bei dem eine Potentialdifferenz zwischen dem Substrat 3 und der Gegenelektrode 4 angelegt wird, während eine Elektrolytflüssigkeit sowohl durch den Messkanal 30 als auch durch den weiteren Kanalabschnitt 40 geleitet wird. Ein aufgrund der Potentialdifferenz bewirkter Stromfluss zwischen dem Substrat 3 und der Gegenelektrode 4 durch die Elektrolytflüssigkeit führt zur Auflösung der, gegebenenfalls auf molekularer Ebene kovalent oder nicht-kovalent vernetzten, Sensormatrix einschließlich der daran gebundenen weiteren Moleküle.
In der hier anhand der Fig. 2 beschriebenen Ausgestaltung sind das Substrat 3, die Gegenelektrode 4 und die Referenzelektrode 2 als Drei-Elektrodenschaltung beschaltet, wobei mittels einer potentiostatischen Schaltung eine Potentialdifferenz zwischen der Referenzelektrode 2 und dem Substrat 3 eingestellt und mittels der Gegenelektrode 4 geregelt wird.

Zur Freilegung des Substrats 3 kann beispielsweise für einen vorgegebenen Zeitraum eine konstante Potentialdifferenz angelegt werden. Es hat sich als besonders schonend für die das Substrat 3 bildende Metallbeschichtung herausgestellt, wenn die zwischen dem Substrat 3 und der Referenzelektrode angelegte Potentialdifferenz zwischen einem Maximalwert und einem Minimalwert kontinuierlich variiert wird, z.B. durch einen linearen Potentialsweep, wobei der Maximalwert und der Minimalwert vorzugweise unterschiedliche Vorzeichen aufweisen, so dass abwechselnd ein oxidierendes und ein reduzierendes Potential an dem Substrat 3 anliegt. Das, insbesondere lineare, Verfahren des Potentials des Substrats 3 bezüglich der Referenzelektrode von einem Ausgangswert zum Maximalwert, danach zum Minimalwert und dann zurück zum Ausgangswert (oder umgekehrt) wird als ein "Zyklus" bezeichnet. Vorzugsweise werden zum Freilegen des Substrats 3 mehrere, beispielsweise 2 bis 100 oder sogar bis zu 1000 Zyklen durchlaufen.

Die konkret zu wählenden Bedingungen der elektrochemischen Reinigung hängen unter anderem von der jeweiligen Zusammensetzung des Elektrolyten und der Sensormatrix, von der Geometrie der Mikrofluidikeinrichtung und von der Anordnung und Materialwahl des Substrats 3 bzw. der Gegenelektrode 4 ab. Sie sind in der Regel in Vorversuchen zu ermitteln. Dabei ist das Reinigungsverfahren dahingehend zu optimieren, dass einerseits eine vollständige Entfernung der Sensormatrix gewährleistet ist, andererseits aber die Elektroden nicht angegriffen werden. Es hat sich in ersten Experimenten gezeigt, dass im Allgemeinen eine besonders gute Reinigungsleistung erzielt wird, wenn das Potential des Substrats zwischen negativen Werten im Bereich zwischen -0,5 V und -1,25 V und positiven Werten im Bereich zwischen + 1,5 V und +2,25 V gegenüber einer Ag/AgCl-Referenzelektrode linear variiert wird, wobei bei Erreichen des Maximal- und Minimalwerts optional das Potential jeweils für einen Zeitraum von einigen Sekunden festgehalten wird. Auf diese Weise wird die Sensormatrix abwechselnd oxidativen und reduktiven Bedingungen und entstehendem Wasserstoff bzw. Sauerstoff ausgesetzt, so dass sich unterschiedliche Bestandteile der Sensormatrix oxidativ bzw. reduktiv lösen und insbesondere zersetzen. Eine rein oxidative Reinigung ist ebenfalls möglich, es hat sich jedoch gezeigt, dass es hierbei eher zur Beschädigung des Substrats kommt. Besonders vorteilhaft an den angegebenen Potentialbereichen ist, dass es beim Maximalwert bzw. Minimalwert in Anwesenheit eines wässrigen Elektrolyten zur Sauerstoff- bzw. Wasserstoffentwicklung kommt. Der gebildete Sauerstoff bzw. Wasserstoff trägt dabei unterstützend zur Auflösung und Zersetzung der Sensormatrix-Bestandteile bei.

Es sind zahlreiche unterschiedliche Potential- bzw. Stromverlaufsfunktionen verwendbar, so beispielsweise Dreieck-, Sägezahn-, Sinus-, Rechteck oder unterschiedliche Puls- und Stufenfunktionen. Besonders bevorzugt sind hierbei Funktionen, bei denen der Stromfluss in jeder Periode, also bei jedem Potentialsweep, für einen gewissen zeitlichen Anteil, weiter bevorzugt zwischen 10 und 95% bezogen auf die Dauer einer Periode, näherungsweise Null beträgt.

In Experimenten mit Substraten aus Platin hat sich gezeigt, das für eine oxidative Reinigung das elektrische Potential des Substrats vorzugsweise zumindest zeitweise auf mehr als +0,6 V, bevorzugt auf mehr als +1,0 V, gegenüber dem Potential einer Silber/Silberchlorid-Referenzelektrode eingestellt wird. Für eine reduktive Reinigung wird das elektrische Potential des Substrats vorzugsweise zumindest zeitweise auf weniger als -0,2 V, bevorzugt auf weniger als -0,4 V, gegenüber dem Potential einer Silber/Silberchlorid-Referenzelektrode eingestellt. Aufgrund der geringen Überspannung für die Entstehung von Wasserstoff auf Platin wird unter diesen Bedingungen ausreichend Wasserstoff für eine reduktive Reinigung erzeugt. Bevorzugt erfolgt der zeitliche Wechsel zwischen oxidativer und reduktiver Reinigung durch ein cyclisches oder periodisches Verfahren des Potentials. In einer besonders einfachen Variante wird für die Einstellung des elektrischen Potentials eine einstell- bzw. verfahrbare Spannungsquelle verwendet, deren Ausgänge nicht auf das Massepotential bezogen sind.

Bevorzugt beträgt die während eines Potentialsweeps bzw. während eines Zyklus, auftretende maximale Stromdichte im Betrag mehr als 2 mA/cm², weiter bevorzugt mehr als 20 mA/cm² bezogen auf die Oberfläche des Substrats innerhalb der mikrofluidischen Kanäle, insbesondere auf den Messbereich des Substrats._Bevorzugt beträgt die während einer Freilegung des Substrats geflossene Ladung des positiven Stromflusses mehr als +0,2 C/cm², weiter bevorzugt mehr als +2 C/cm² und die Ladung des negativen Stromflusses weniger als -0,2 C/cm², weiter bevorzugt weniger als -2 C/cm², jeweils bezogen auf die Substratoberfläche innerhalb der mikrofluidischen Kanäle bzw. den Messbereich. Weiter bevorzugt sind die positive und negative, bei elektrolytischen Freilegung durch das Substrat geflossenen Ladungen betragsmäßig im Wesentlichen gleich, bzw. unterscheiden sich in ihren Beträgen um weniger als 25%.

Der bei einem linearen Potentialsweep am Substrat 3 auftretende Strom kann optional erfasst und als Zyklovoltammogramm ausgewertet werden. Die so gemessenen Zyklovoltammogramme können insbesondere Aufschluss darüber geben, ob noch Reste der Sensormatrix am elektrisch leitfähigen Substrat 3 gebunden sind. Somit kann der erfolgreiche Abschluss der Reinigungsphase anhand der erfassten Zyklovoltammogramme ermittelt werden. Auch andere voltammetrische Analyseverfahren wie beispielsweise die Square-Wave-Voltammetrie oder Differential-Pulse-Voltammetrie sind hierfür gut geeignet.

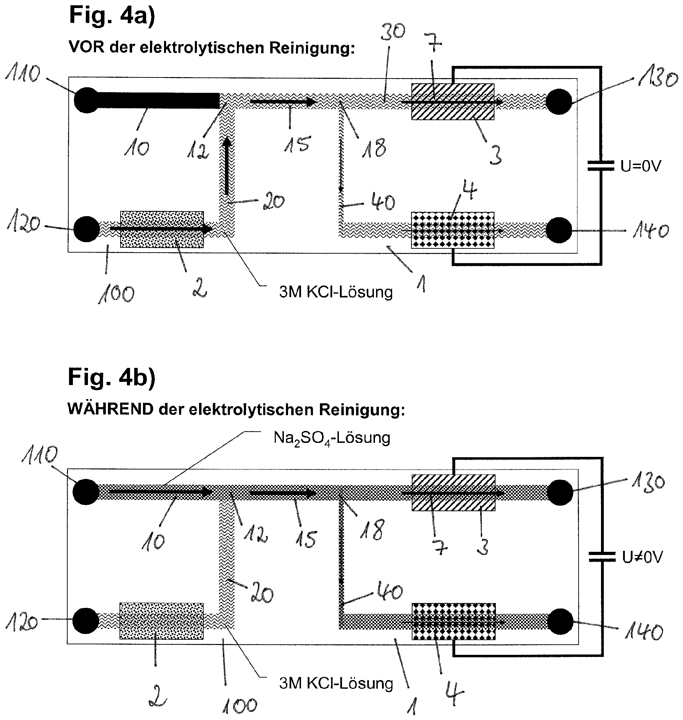

Anhand der Fig. 4 a) und 4 b) wird im Folgenden ein Beispiel für ein derartiges elektrochemisches Reinigungsverfahren zur Freilegung des Substrats 3 mittels der Mikrofluidikeinrichtung 100 im Detail beschrieben. Vor Anlegen einer Potentialdifferenz zwischen dem Substrat 3 und der Gegenelektrode 4 wird zunächst über den Anschluss 120 an eine Flüssigkeitszuleitung eine 3 M wässrige KCI-Lösung in den Kanalabschnitt 20, dann weiter über die Verzweigung 12 in den Messkanal 30 und über die Verzweigung 18 zwischen dem Messkanal 30 und dem weiteren Kanalabschnitt 40 in den weiteren Kanalabschnitt 40 geleitet, in dem die Gegenelektrode 4 angeordnet ist. Die Leitung der Flüssigkeit durch die genannten Kanalabschnitte erfolgt, indem zwischen dem Zulaufanschluss 120 und den Ableitungsanschlüssen 130, 140 eine Druckdifferenz angelegt wird, während zwischen dem Zulaufanschluss 110 und den Ableitungsanschlüssen 130, 140 keine Druckdifferenz anliegt.

Sobald der Kanalabschnitt 20, in dem die Referenzelektrode 2 angeordnet ist, für die elektrochemische Reinigung mit der KCI-Lösung gefüllt ist, wird über die Zuleitung 110 durch den Messkanal 30 und über die Verzweigungsstelle 18 auch in den Kanalabschnitt 40 mit der Gegenelektrode 4 ein Reinigungselektrolyt für die elektrochemische Reinigung, beispielsweise eine 100 mM Na₂SO₄-Lösung, geleitet. Als Reinigungselektrolyt hat sich weiterhin auch eine saure Phosphatpufferlösung (pH=2,1) mit 100 mmol/l Phosphatgehalt, der gegebenenfalls 0,25 Vol.-% Wasserstoffperoxid zugesetzt sind, als geeignet erwiesen. Generell sollte die Zusammensetzung der Reinigungslösung so gewählt werden, dass ein möglichst hoher Stromfluss bei geringen Potentialen an Substrat und Gegenelektrode ermöglicht wird. Auf diese Weise ist eine hohe Reinigungsleistung bei nur geringer bis keiner Schädigung der Elektroden möglich.

Das Durchleiten des Reinigungselektrolyten auf diesem Strömungsweg wird durch Anlegen einer Druckdifferenz zwischen dem Zuleitungsanschluss 110 und den beiden Ableitungsanschlüssen 130, 140 bewirkt. Dadurch, dass der an dem zweiten Zuleitungsanschluss 120 anliegende Druck so eingestellt wird, dass zwischen diesem und den Ableitungsanschlüssen 130, 140 keine Druckdifferenz anliegt, wird verhindert, dass die Na₂SO₄-Lösung auch in den Kanalabschnitt 20 mit der Referenzelektrode 2 strömt. An der Verzweigungsstelle bildet sich daher ein Übergang zwischen der zuvor eingeleiteten KCI-Lösung und der Na₂SO₄-Lösung aus, über den ein Ladungsträgeraustausch möglich ist. Auf diese Weise wird ein diaphragmenloser Stromschlüssel der Referenzelektrode gebildet, deren Referenzelektrolyt, die KCl-Lösung, über die Verzweigungsstelle in leitfähigem Kontakt mit der als Reinigungselektrolyten dienenden Na₂SO₄-Lösung steht, ohne dass ein poröses Diaphragma, ein Spalt, eine Fritte oder sonst ein den Referenzelektrolyten von dem Reinigungselektrolyten körperlich trennendes Bauteil notwendig ist.

Nachdem der Messkanal 30 und der Gegenelektrode 4 aufweisende Kanalabschnitt 40 vollständig mit dem Reinigungselektrolyten befüllt sind, wird eine Potentialdifferenz zwischen dem Substrat 3 und der Gegenelektrode 4 angelegt. Dies kann beispielsweise durch Anlegen eines bestimmten Potentials gegenüber der Referenzelektrode 2 an das Substrat 3 erfolgen. Der Flüssigkeitsübergang zwischen dem im Kanalabschnitt 20 enthaltenen KCl-Elektrolyten und dem im Kanalabschnitt 10, 15 aufgenommenen Reinigungselektrolyten an der Verzweigungsstelle 12 zwischen dem Kanalabschnitt 10 und dem Kanalabschnitt 20 bildet einen diaphragmenlosen Stromschlüssel, über den die Referenzelektrode 2 mit dem Substrat 3 verbunden ist. Durch die potentiostatische Schaltung wird der Strom zwischen dem Substrat 3 und der Gegenelektrode 4 so eingestellt, dass das gewünschte Potential erreicht wird. Entsprechend entstehen auch eine Potentialdifferenz und ein Stromfluss zwischen dem Substrat 3 und der Gegenelektrode 4. Wie voranstehend beschrieben, wird das an dem Substrat 3 anliegende Potential gegenüber der Referenzelektrode 2 beispielsweise linear zwischen einem Maximal- und Minimalwert mehrfach verfahren. Dieses Vorgehen hat sich als besonders schonend für den das Substrat 3 bildenden Metallfilm erwiesen.

Bei der elektrochemischen Reinigung an dem Substrat 3 oder an der Gegenelektrode 4 gegebenenfalls, beispielsweise aufgrund von Wasserstoff- oder Sauerstoffentwicklung an einer der Elektroden, auftretende Gasblasen, werden in Strömungsrichtung (vgl. Pfeile der Fig. 4 b)) der Elektrolytflüssigkeit von der Verzweigungsstelle 18 weg über die Ableitungen 130, 140 aus dem Kanalsystem der Mikrofluidikeinrichtung abgeleitet. Gleichermaßen werden auch von dem Substrat 3 gelöste Moleküle, Molekülteile oder Molekülaggregate aus dem Messkanal 30 in Strömungsrichtung abgeleitet, so das eine Re-Adsorption vermieden wird.

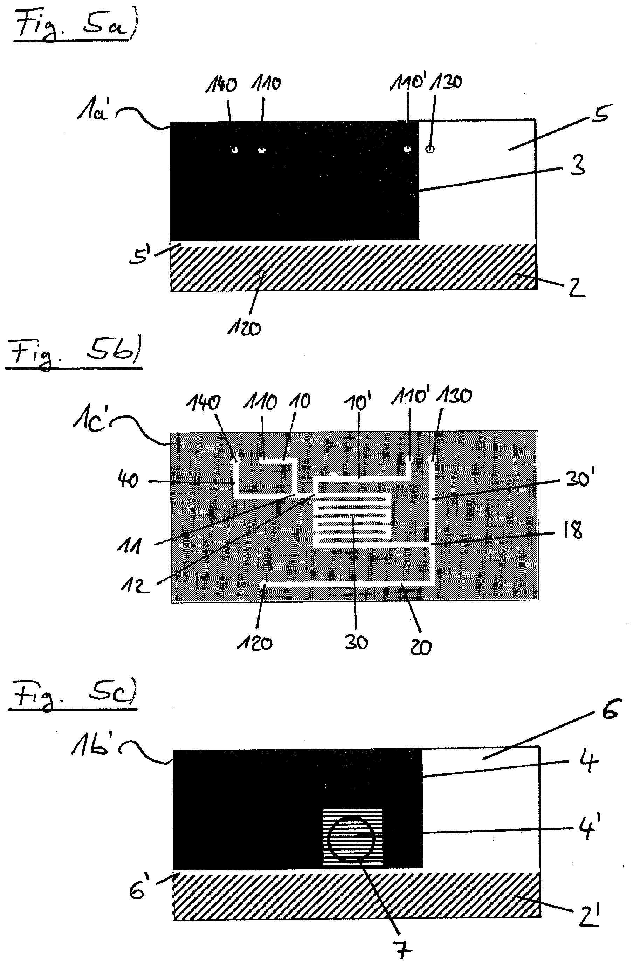

Auch zahlreiche alternative Ausgestaltungen der Mikrofluidik-Einheit des Bioanalysators sind möglich. Fig. 5 a) bis c) veranschaulicht eine bevorzugte Ausgestaltung einer Mikrofluidik-Einheit. Gleiche Baugruppen sind mit gleichen Bezugszeichen wie in dem vorigen Beispiel bezeichnet. Die Mikrofluidik-Einheit weist ein erstes Bauteil 1a' (Fig. 5 a)), ein zweites Bauteil 1b' (Fig. 5 c)) und eine zwischen dem ersten Bauteil 1a' und dem zweiten Bauteil 1b' angeordnete Zwischenplatte 1c' (Fig. 5 b)) auf, die in der Fig. 5 jeweils in Aufsicht gezeigt sind.

Das in Fig. 5 a) gezeigte erste Bauteil 1a' kann aus einem transparenten Material, beispielsweise aus Glas oder einem Kunststoff bestehen. In Aufsicht sind die als Filme ausgestaltete Referenzelektrode 2 und das Substrat 3 zu sehen. Als Referenzelektrode 2 dient z.B. eine Silber/Silberchlorid-Referenzelektrode, welche hergestellt wurde aus einem, auf dem Bauteil 1a' gegebenenfalls über eine oder mehrere Zwischenschichten, insbesondere Haftvermittler, aufgebrachten Silberfilm, welcher oberflächlich chloridiert wurde, beispielsweise durch Behandlung mit einer Eisen(III)-chlorid-Lösung. Das Substrat 3 ist ebenfalls als Film auf dem ersten Bauteil 1a', gegebenenfalls über eine oder mehrere Zwischenschichten, aufgebracht. Hierbei kann es sich beispielsweise um eine 50 bis 500 nm dicke Platinschicht handeln, welche z.B. mittels Sputterns aufgebracht wurde, und welche als thiophiles Substrat für eine Anbindung einer Sensormatrix mittels der Ausbildung einer Platin-Schwefel-Bindung zwischen einer ersten, eine erste Anbindungsschicht bildenden chemischen Spezies dient. Das Substrat 3 und die Referenzelektrode 2 sind über nicht näher gezeigte Anschlüsse elektrisch kontaktierbar mit z.B. einer Messschaltung oder einer Spannungsquelle, z.B. einen Potentiostaten oder Galvanostaten.

Die Bereiche 5 und 5' des Bauteils 1a' sind nicht mit einem elektrisch leitfähigen Material beschichtet. Gegebenenfalls können die Bereiche 5 und 5', gegebenenfalls auch nur in den an die Elektroden angrenzenden Bereichen, mit einem chemisch stabilen und elektrisch isolierenden Material wie beispielsweise Siliziumnitrid Si₃N₄ beschichtet sein. Bevorzugt ist eine solche Beschichtung etwa 10 bis 100 µm überlappend auf die Elektrodenflächen aufgebracht und kann so wirkungsvoll ein Unterätzen der Elektroden, also beispielsweise eine Auflösung eines Haftvermittlers wie Titandioxid, bei der elektrochemischen Freilegung des Substrats 3 verhindern. Weiterhin sind mit 110, 110' und 120 die Zuführungsanschlüsse der Mikrofluidik-Einheit und mit 130 und 140 die Ableitungsanschlüsse der Mikrofluidik-Einheit dargestellt. Diese sind in dem gezeigten Beispiel als Bohrungen ausgeführt, wobei auf der nicht in Aufsicht gezeigten Fläche beispielsweise handelsübliche Konnektoren zum Anschluss mikrofluidischer Schlauchleitungen z.B. durch Verklebung aufgebracht sein können.

Fig. 5 c) zeigt das zweite Bauteil 1b' der Mikrofluidik-Einheit in Aufsicht. Das zweite Bauteil 1b' kann wie das erste Bauteil 1a' aus einem transparenten Material, beispielsweise Glas oder Kunststoff, gebildet sein, wobei Elektroden 2', 4 und 4' sowie elektrisch isolierende Bereiche 6, 6' als Filme, gegebenenfalls über, insbesondere haftvermittelnde, Zwischenschichten auf dem zweiten Bauteil 1b' aufgebracht sind. Die Referenzelektrode 2' ist gleich ausgestaltet wie die auf dem ersten Bauteil 1a' aufgebrachte Referenzelektrode 2. Die Gegenelektrode weist einen ersten Gegenelektrodenbereich 4 und einen zweiten Gegenelektrodenbereich 4' auf. Der erste Gegenelektrodenbereich 4 ist aus einer als Film vollflächig aufgebrachten, elektrisch leitfähigen, chemisch inerten Substanz, z.B. aus Bor-dotiertem Diamant oder aus Platin gebildet. Im Bereich 4', der in der zusammengesetzten Mikrofluidik-Einheit im Bereich des mäanderförmig ausgestalteten Messkanals 30 aufliegt, ist die Gegenelektrode unterbrochen ausgeführt. Im gezeigten Beispiel handelt es sich um eine seitlich mit dem Gegenelektrodenbereich 4 verbundene Linienstruktur, z.B. aus Linien mit etwa 10 µm Breite, welche in einem Abstand von etwa 20 µm parallel angeordnet sind. Ebenso wären hier andere Abmessungen und Geometrien, beispielsweise eine Gitterstruktur, verwendbar. Bei Verwendung eines optisch nicht transparenten Elektrodenmaterials wird hierdurch im Messbereich 7 des Messkanals, also in dem Bereich, der für die Gewinnung eines Messsignals mit Hilfe eines entsprechenden Signalwandlers genutzt wird, eine teilweise optische Transparenz geschaffen. So ist in einer bevorzugten Ausgestaltung der Mikrofluidik-Einheit eines Bioanalysators, welcher mit einem indirekten Verfahren mit Hilfe einer Enzym-Markierung betrieben wird, beispielsweise ein optischer Signalwandler unterhalb eines Bereichs oder eines Teilbereichs der Gegenelektrode 4' angeordnet, dessen Erfassungsbereich durch den in Fig. 5 c) durch die Kreisfläche angedeuteten Messbereich 7 gebildet ist. Der Signalwandler kann beispielsweise eine Photodiode aufweisen, welche durch den Enzym-Marker katalytisch erzeugtes Chemolumineszenzlicht erfasst und in ein proportionales Stromsignal umwandelt. Die Gegenelektrode 4, 4' und die Referenzelektrode 2' weisen nicht näher dargestellte Anschlüsse zur elektrischen Kontaktierung auf. Im zusammengebauten Zustand der Mikrofluidik-Einheit können die auf dem ersten Bauteil 1a' gebildete Referenzelektrode 2 und die auf dem zweiten Bauteil 1b' gebildete Referenzelektrode 2' elektrisch leitfähig miteinander verbunden und/oder gemeinsam von der Messschaltung bspw. eines Potentiostaten oder Galvanostaten kontaktiert sein.

Die in Fig. 5b gezeigte durchbrochene Zwischenplatte 1c' besteht beispielsweise aus einer elastischen, 125 µm dicken Matte aus Polydimethylsiloxan (PDMS). Zur Bildung der Mikrofluidik-Einheit werden die in Fig. 5a) sichtbare Basisfläche des ersten Bauteils 1a' und die in Fig. 5 c) sichtbare Basisfläche des zweiten Bauteils 1b' einander zugewandt, beabstandet und einander gegenüberliegend angeordnet, während die Zwischenplatte 1c' mit den Bauteilen 1a' und 1b' fluchtend als Abstandselement zwischen den beiden Bauteilen 1a' und 1b' zu liegen kommt. Ist die Zwischenplatte 1c' aus einem elastischen Material gebildet, können das erste 1a' und zweite Bauteil 1b' durch beidseitiges Verpressen mit der dazwischen angeordneten Zwischenplatte 1c' flüssigkeitsdicht verbunden und so die Mikrofluidik-Einheit mit einer gegenüber der Umgebung abgeschlossenen Kanalstruktur gebildet werden. Die Breite der Kanäle beträgt in dem gezeigten Beispiel 500 µm. In einer alternativen Ausgestaltung wird die Kanal bildende, durchbrochene Zwischenplatte in einem photolithographischen Prozess aus einem photosensitiven Material, beispielsweise aus einem Photolack, hergestellt. Bevorzugt ist in diesem Fall die Zwischenplatte 1c' fest mit dem ersten und zweiten Bauteil 1a' bzw. 1b' verbunden.

Die so gebildete Kanalstruktur wird im Folgenden beschrieben: Über die Anschlüsse 110 und 110' können die für den Aufbau der Sensormatrix (Schritt i), die Durchführung der Messung (Schritt ii) und die Freilegung des Substrats (Schritt iii) benötigten Reagenzien bzw. Reagenzienlösungen aus Bevorratungsgefäßen bzw. Probezuleitungs- und ggfs. Mischsystemen (nicht dargestellt) zugeführt werden. Optional kann die Mikrofluidik-Einheit auch noch weitere Anschlüsse aufweisen (nicht dargestellt). In einer bevorzugten Variante werden die Reagenzien bzw. Reagenzienlösungen, die miteinander wechselwirken können, über zwei unterschiedliche Zuleitungen der Mikrofluidik-Einheit zugeführt. Hierdurch wird verhindert, dass solche Komponenten bereits im Zuleitungssystem miteinander wechselwirken, z.B. dass sich ein nachfolgendes Reagenz mit einem zuvor durch die Zuleitung gespülten anderen Reagenz, welches durch unspezifische Adsorption an den Wandungen des Zuleitungssystems verblieben ist, wechselwirkt. Bei einem dauerhaften Betrieb eines erfindungsgemäßen Bioanalysators kann es durch solche Wechselwirkungen von Komponenten und Reagenzien in den Zuleitungssystemen zum einen zu größeren Ablagerungen kommen und zum anderen zu einer anfänglichen Abnahme der Konzentration von Reagenzien beim Durchspülen durch die Mikrofluidik-Einheit, speziell durch den Messkanal 30, führen, was sich, speziell bei Analysen, die eine niedrige Nachweisgrenze erfordern, negativ auf die Reproduzierbarkeit der Messergebnisse auswirkt bzw. die Spülzeiten und damit den Reagenzienverbrauch stark erhöhen kann.

Der von dem Anschluss 110' kommende Kanalabschnitt 10' trifft an der Kanalverzweigung 12 auf den von dem Anschluss 110 kommenden Kanalabschnitt 10. An der Kanalverzweigung 11 kann die Flüssigkeit über den Kanalabschnitt 40 in den Ableitungsanschluss 140 in ein diesem nachgeschalteten Abfall-Behältnis (nicht dargestellt) geleitet werden, was einem Bypass des Messbereichs entspricht. Hierbei muss durch die externe Beschaltung der Mikrofluidik-Einheit mit Flüssigkeitsantriebssystemen und Ventilen der Fluss entsprechend gelenkt werden. Ein Vorteil des durch den Kanalabschnitt 140 gebildeten Bypasses besteht darin, dass die Reagenzien bzw. Reagenzienlösungen zuerst hierüber an der eigentlichen Messeinheit vorbei gelenkt werden können, bis die anfänglich möglicherweise enthaltenen Gasblasen aus den Zuleitungssystemen entfernt sind und bis der beim Wechsel eines ersten Reagenz bzw. einer ersten Reagenzienlösung auf ein zweites, nachfolgendes Reagenz bzw. Reagenzienlösung sich ausbildender Konzentrationsgradient des zweiten Reagenz bzw. Reagenzienlösung das Kanalsystem mindestens die Kanalverzweigung 12 passiert hat. Durch diese anfängliche Durchleitung der Reagenzien bzw. Reagenzienlösungen kann gewährleistet werden, dass beim Durchleiten der Reagenzien bzw. Reagenzienlösungen durch den Messkanal 30, der durch den Messbereich führt, diese von Anfang an in dergleichen Konzentration und im Wesentlichen frei von Gasblasen durchgeleitet werden, was sich positiv auf die Reproduzierbarkeit der Messergebnisse bei aufeinander folgender Durchführung der oben beschriebenen Verfahrensschritte i bis iii mit dem automatisierten Bioanalysator auswirkt.

Der Messkanal 30 ist im vorliegenden Beispiel mäanderförmig ausgeführt, wobei bevorzugt alle Kanäle der Mikrofluidik-Einheit den gleichen Querschnitt aufweisen. Hierdurch können Gasblasen, wenn sie von außen in die Mikrofluidik-Einheit eingeleitet werden und vor allem die Gasblasen, welche bei einer elektrochemischen Freilegung des Substrates entstehen, sehr einfach und mit verminderter Gefahr, dass diese sich in Bereichen mit größerem Kanalquerschnitt an die Kanalwandungen anhaften, wieder aus der Mikrofluidik-Einheit ausgeleitet werden. Der Messkanal 30 trifft an der Verzweigung 18 auf einen weiteren Kanalabschnitt 20, durch den über den Zuleitungsanschluss 120 Reagenzien für die Sicherstellung eines stabilen Referenzpotentials der Referenzelektroden 2 und 2', beispielsweise eine 3-molare Kaliumchlorid-Lösung, zugeführt werden können. Die Flüssigkeiten, die durch den Messkanal 30 und durch den Kanalabschnitt 20 geleitet werden, fließen der Kanalverzweigung 18 nachfolgend, wiederum durch die äußere Beschaltung mit Flüssigkeitsantriebssystemen und Ventilen gesteuert, durch den Kanalabschnitt 30' und den Ableitungsanschluss 130 in ein nachgeschaltetes Abfall-Behältnis (nicht dargestellt). Hierdurch wird ein Eindringen von Chlorid-Ionen in den Bereich des Messkanals 30 wirkungsvoll vermieden.
Wie im weiter oben angeführten Beispiel stehen die Referenzelektroden 2 bzw. 2' über einen diaphragmenlosen Stromschlüssel mit dem Substrat 3 und der Gegenelektrode 4 und 4' in elektrischem Kontakt. Auch das Entstehen von Gasblasen während der elektrochemischen Freilegung des Substrats unterbricht diesen elektrischen Kontakt zwischen der Referenzelektrode 2, 2' mit dem als Arbeitselektrode geschalteten Substrat 3 und der Gegenelektrode 4,4' nur kurzzeitig, wenn sich eine Gasblase am Ende des Messkanals 30 kurz vor der Kanalverzweigung 18 befindet. Da die Gasblasen jedoch aufgrund des, bevorzugt mindestens zeitweise angelegten, Volumenstroms durch den Messkanal 30 und des, ebenfalls bevorzugt mindestens zeitweise angelegten, Volumenstroms durch den Kanalabschnitt 20 rasch in den Kanalabschnitt 30' geleitet werden, ist diese Unterbrechung des elektrischen Kontakts zwischen Referenz- und Arbeits- bzw. Gegenelektrode nur von kurzer Dauer und kann darüber hinaus, beispielsweise mit elektronischen Hilfsmitteln, detektiert und kompensiert werden.
Die Mikrofluidik-Einheit gemäß Fig. 5 a) bis c) verdeutlicht die geometrischen Zusammenhänge zwischen Messkanal, Messbereich, Substrat und Gegenelektrode. Der Messkanal 30 ist der Bereich des mikrofluidischen Systems, in welchem die Sensormatrix des Bioanalysators beim Durchleiten der zu deren Aufbau benötigten Reagenzien in immer gleicher und reproduzierbarer Weise auf dem darin angeordneten Substrat erfolgt. In der Mikrofluidik-Einheit gemäß Fig. 5 a) bis c) ist dies der Kanalbereich 30 bis zum Ende des Substrats kurz vor der Kanalverzweigung 18. Auch in dem Kanalbereich zwischen den Kanalverzweigungen 11 und 12 und im Kanalbereich 40 des Bypasses wird durch das Vorspülen der Reagenzien bzw. Reagenzienlösungen durch diese Kanalabschnitte eine Sensormatrix aufgebaut. Diese kann jedoch in ihrer Zusammensetzung und Dichte variieren oder nach der Durchleitung von Gasblasen sogar teilweise oder vollständig ohne Funktion sein. Das Substrat und die Gegenelektrode bedecken zusätzlich neben dem Messkanal in dem in Fig. 5 gezeigten Beispiel auch die Kanalbereiche 10, 10' und 40 sowie die Kanalverzweigungen 11 und 12. Auch hier sind andere Geometrien möglich. Voraussetzung für eine erfindungsgemäße Funktionsweise der Mikrofluidik-Einheit ist, dass das Substrat mindestens im Messbereich zumindest teilweise vorliegt. Der das Substrat beinhaltende Messkanal, auf welchem die vollständige Sensormatrix in reproduzierbarer Weise aufgebaut wird, kann sich aber auch zusätzlich außerhalb des vom Signalwandler erfassten Bereichs 7 erstrecken. Ein Beispiel hierfür ist der Ersatz der Photodiode in dem in Fig. 5 a) bis c) gezeigten Beispiel der Mikrofluidik-Einheit, mit dem ein durch die Enzym-Markierung verursachtes Chemilumineszenzsignal erfasst werden kann, durch eine optische Vorrichtung zur Messung der Transmission, welche beispielsweise beidseitig der Mikrofluidik-Einheit angeordnet sein kann und die Transmission am Ende des Kanalabschnitts 30 kurz vor der Kanalverzweigung 18 bestimmt. Bei der Verwendung eines chromophoren an Stelle eines chemilumineszenten Enzym-Substrats dient in diesem Fall der gesamte von Substrat bedeckte Bereich des Kanalabschnitts 30 und nicht mehr nur der Bereich im Erfassungsbereich der Photodiode als Messbereich, da dieser gesamte Bereich für die Gewinnung des Messsignals, in diesem Fall einer Transmissionsänderung der durch den Messkanal 30 geleiteten Enzym-Substratlösung, mit Hilfe eines dem Messkanal nachgeschalteten Signalwandlers genutzt wird.

Das als Arbeitselektrode geschaltete Substrat 3 und die Gegenelektrode 4, 4' sind bezüglich der mikrofluidischen Kanäle so angeordnet, dass die Kanalabschnitte 10, 10', 30 und 40 sowie die Kanalverzweigungen 11 und 12 von diesen flüssigkeitsdicht abgedeckt werden. Hierdurch wird gewährleistet, dass in diesen Bereichen eine vollständige Freilegung der Substratoberfläche durch elektrochemische Regenerierung bzw. Reinigung erfolgt. Durch den näherungsweise über alle Kanalbereiche gleichbleibenden Abstand zwischen dem Substrat und der Gegenelektrode erfolgt die Freilegung der Substratoberfläche ebenfalls weitgehend über alle Kanalbereiche gleichermaßen und ohne wesentliche Unterschiede in der Verteilung der die Substratoberfläche durchfließenden elektrischen Stromdichte. Dies ist von Vorteil, da hierdurch eine gegebenenfalls unter den Regenerierungs- bzw. Reinigungsbedingungen auftretende oberflächliche Ablösung des Substrats sich gleichermaßen auf alle Bereiche, besonders auf die Bereiche im Messkanal, auswirkt und somit eine häufige Regenerierung und damit eine häufige *in situ*-Durchführung von Messungen mittels des Bioanalysators bei gleichbleibend hoher Qualität der Messergebnisse ermöglicht. Wie bereits weiter oben beschrieben, kann die Gegenelektrode 4 und 4' auch als weiteres Substrat zum Aufbau einer Sensormatrix dienen. Hierbei besteht die Gegenelektrode vorzugsweise aus dem gleichen Material wie das Substrat 3, in dem gezeigten Beispiel also aus Platin. Auf diesem weiteren Substrat laufen dann während der Durchführung der Schritte i bis iii vorzugsweise die im Wesentlichen gleichen Vorgänge ab wie auf dem im mikrofluidischen Kanal gegenüberliegenden Bereich des Substrats 3.

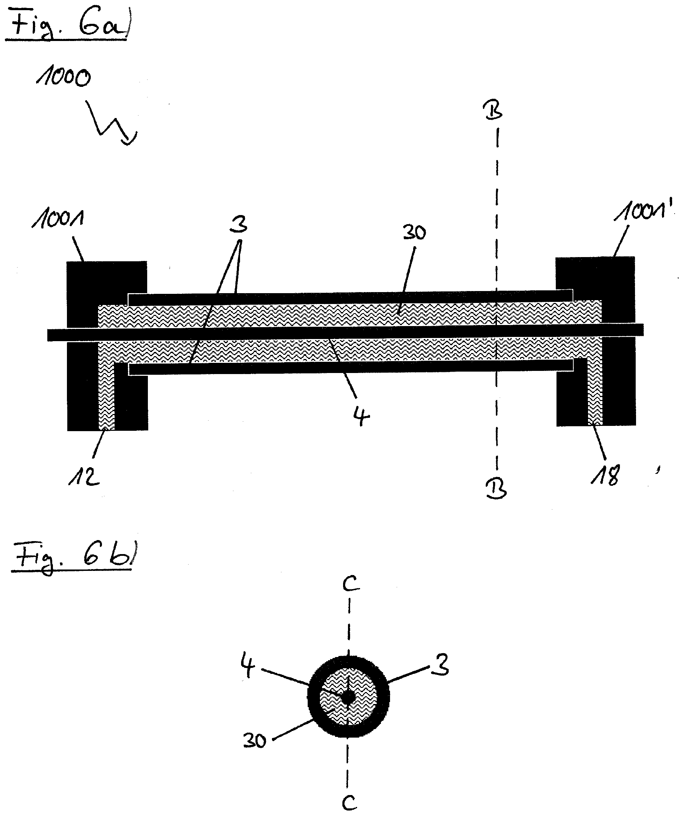

In Fig. 6 ist eine weitere Ausgestaltung einer Mikrofluidik-Einheit 1000 gezeigt. Hierbei sind als Substrat 3 und als Gegenelektrode 4 elektrisch leitfähige, mikrofluidische Röhrchen bzw. Drähte verwendet. So besteht das Substrat 3 beispielsweise aus einem Gold-Röhrchen und die Gegenelektrode 4 aus einem koaxial zum Substrat 3 angeordneten Gold-Draht. Vorteile bei dieser Art der Ausgestaltung liegen in der einfachen elektrischen und mikrofluidischen Konnektierbarkeit dieser Röhrchen mittels entsprechender Halterungen 1001, 1001'. Das Röhrchen 3 dient hierbei als Substrat und der Draht 4 als Gegenelektrode und gleichfalls als weiteres Substrat. Auch die umgekehrte Funktionalität ist möglich. Die koaxiale Anordnung von Substrat 3 und Gegenelektrode 4 erlaubt durch den kleinen Abstand zwischen Substrat und Gegenelektrode hohe Stromdichten, was eine lange Verwendung auch bei geringfügiger Abtragung der Oberfläche des Substrats und/oder der Gegenelektrode bei der elektrischen Regenerierung bzw. Reinigung ermöglicht. Durch die entsprechende Konnektierung mittels mikrofluidischer Schläuche und Röhrchen lassen sich auch komplexere Mikrofluidik-Einheiten, welche nicht oder nur teilweise als Mikrofluidik-Chip aufgebaut sind, realisieren.

Nach einigen Zyklen, in denen das Potential des Substrats 3 zwischen dem Maximal- und dem Minimalwert verfahren wird, ist das Substrat 3 im Wesentlichen vollständig freigelegt. Danach kann eine erneute Präparation einer Sensormatrix in der weiter oben beschriebenen Weise durchgeführt werden, um für eine erneute Messung zur Verfügung zu stehen. Auf diese Weise können mindestens 50, oder sogar 100 bis 200 Messungen mit jeweils neu präparierter Sensormatrix durchgeführt werden.

Da die Sensormatrix regelmäßig vollständig neu präpariert wird, ist es auch möglich, aufeinanderfolgende Messungen verschiedener Analyte durchzuführen, wobei dann jeweils eine Sensormatrix mit bestimmten Rezeptoren präpariert wird, die speziell für den jeweils zu bestimmenden Analyten geeignet sind. Beispielsweise können in aufeinanderfolgenden Präparationen unterschiedliche, jeweils an einen aktuell nachzuweisenden Analyten bzw. an entsprechende für den Analytnachweis verwendete Zielmoleküle vorzugsweise selektiv und spezifisch bindenden Rezeptoren an die Anbindungsmoleküle R200 gebunden werden.

In einem alternativen und bevorzugten Aufbau der Mikrofluidikeinrichtung können die Gegenelektrode und das Substrat innerhalb ein und desselben Kanals einander gegenüberliegend angeordnet sein. Dabei ist die Referenzelektrode in einem durch eine Verzweigung mit dem Messkanal verbundenen Kanalabschnitt angeordnet, so dass sie, wie in Fig. 4b gezeigt, während der elektrochemischen Reinigung über einen diaphragmenlosen Stromschlüssel mit dem Substrat leitfähig verbunden ist. Wenn das Substrat und die Gegenelektrode einander gegenüberliegend im Messkanal angeordnet sind, wird bei dem oben beschriebenen Verfahren zur Präparation der Sensormatrix sowohl auf dem Substrat als auch auf der Gegenelektrode eine Sensormatrix aufgebaut, so dass beide zur Generierung des Messsignals genutzt werden können. Dabei kann beispielsweise das Substrat transparent für Messstrahlung sein, so dass beispielsweise an der Gegenelektrode und dem Substrat erzeugte Lumineszenzstrahlung durch das Substrat transmittiert wird und durch eine hinter dem Substrat angeordnete Photodiode als Messsignal erfasst werden kann.

Eine wichtige Anforderung an einen zur Überwachung einer (quantitativen) Analytkonzentration in einem industriellen Prozess verwendeten Bioanalysator ist die Reproduzierbarkeit der vom Bioanalysator bei aufeinanderfolgenden Messungen mit dazwischen jeweils regenerierter Sensormatrix ausgegebenen Messsignale. Insbesondere muss gewährleistet sein, dass der Bioanalysator bei aufeinanderfolgenden Messungen, zwischen denen jeweils eine Neupräparation der Sensormatrix durchgeführt wurde, bei gleich bleibender Analytkonzentration auch jeweils Messsignale dergleichen Signalstärke ausgibt.

Um dies mit dem hier beschriebenen Verfahren zu gewährleisten, wird die Präparation der Sensormatrix so durchgeführt, dass der Bedeckungsgrad der Schicht von Anbindungsmolekülen R200 auf dem leitfähigen Oberflächenabschnitt 3 mindestens 80%, wie oben definiert, beträgt.

Optimalerweise wird auch beim zweiten und gegebenenfalls weiteren Präparationsschritten, bei dem bzw. bei denen die Rezeptoren an die Anbindungsschicht angebunden werden, die Zeitspannen bei denen die zweite und gegebenenfalls weitere Präparationslösungen durch den Messkanal geleitet werden, so gewählt, dass jeweils ein Bedeckungsgrad von 80%, wie oben definiert, erreicht wird.

Der in Fig. 1 a) und b) gezeigte Schichtaufbau der Sensormatrix und gegebenenfalls die direkte oder indirekte Anbindung von Marker tragenden Molekülen an den Analyt oder an mit dem Analyten um Bindungsplätze der Sensormatrix konkurrierende Zielmolekülen erlaubt es, in einfacher Weise für einmalig verwendbare Assays aus dem Laborgebrauch bekannte Verfahren auf ein wiederholt und automatisiert mittels eines Bioanalysators durchführbares Verfahren zu übertragen. Als Beispiel hierfür wird hier die Übertragung eines Immunoassays für den einmaligen Laborgebrauch auf einen automatisiert arbeitendenden Bioanalysator für die Prozessmesstechnik beschrieben: In dem Laborassay wird die Rezeptorschicht durch Adsorption von Antikörpern gegen den Bovinen Coronavirus (BCV) auf der hydrophoben Substratoberfläche einer Mikrotiterplatte hergestellt. Im Anschluss wird die Messprobe, die auf BCV-Bestandteile untersucht werden soll, aufgetragen und nachfolgend gespült, so dass nicht an die Rezeptoren gebundenes BCV entfernt wird. Nachfolgend wird ein zweiter Antikörper gegen ein anderes Epitop des BCV aufgebracht, erneut gespült und danach ein Konjugat eines Meerrettichperoxidase (HRP)-konjugierten dritten Antikörpers aufgebracht. Dieser dritte Antikörper bindet an den zweiten, so dass dieser indirekt mit HRP markiert wird. Nach wiederholtem Spülen erfolgt nachfolgend die Bestimmung des Messwerts durch einen abschließenden Schritt der Zugabe des chromophoren Substrats 2, 2'-Azino-di-(3-ethylbenzthiazolin)-6-sulfonsäure (ABTS) und Wasserstoffperoxid und photometrische Bestimmung der Absorption dieser Lösung.

Ein Verfahren zur Übertragung der Bestimmung des Gehalts an BCV einer Flüssigkeitsprobe mittels des beschriebenen einmal benutzbaren Affinitäts-Bioassays auf eine automatisierte und wiederholt durchführbare *in situ*-Bestimmung des BCV-Gehalts einer Flüssigkeitsprobe mittels eines Bioanalysators ist nachfolgend beschrieben. In einem ersten Schritt (Schritt a) ist eine geeignete Anbindungsstruktur zum Aufbau der Anbindungsschicht bzw. Anbindungsschichten der Sensormatrix auszuwählen. Da es sich bei dem als Rezeptor R350 dienenden Antikörper gegen BCV (anti-BCV) um einen nicht-biotinylierten Antikörper der Klasse IgG handelt, ist die anhand von Fig. 1 b) beschriebene Schichtabfolge Thiol-PEG-Biotin - Neutravidin - Biotin-Anti-IgG - anti-BCV, wobei Anti-BCV als Rezeptor R350 dient, gut geeignet. Als alternatives Bestimmungsverfahren (Schritt b) kann bei Verwendung eines HRP-Moleküls als Marker tragendes Molekül R500 neben der chromophoren ABTS-Reaktion mit nachfolgender photometrischer Bestimmung auch die Chemolumineszenzreaktion zwischen Luminol und Wasserstoffperoxid verwendet werden. Diese hat in einem automatisierten Bioanalysator den Vorteil, dass als Signalwandler lediglich ein Photodetektor, wie beispielsweise eine Photodiode, erforderlich ist.

In einem weiteren Schritt wird ein für die in dem automatisierten Bioanalysator für Flüssigkeitsleitungen oder sonstige, mit der Flüssigkeitsprobe oder den anderen verwendeten Reagenzien in Berührung kommenden Teile verwendeten Materialien ein geeignetes Blocking-Reagenz ausgewählt werden, um die unspezifische Adsorption der Komponenten zu reduzieren.

Nachdem auf diese Art und Weise die Anbindungsschicht bzw. Anbindungsschichten definiert und ein alternatives Bestimmungsverfahren ausgewählt wurde, ergibt sich eine aus wenigen, universellen Standardvarianten ausgewählte Anbindungsschichtfolge der Sensormatrix, gebildet aus:
- thiophiles Substrat (Substrat 3)
- Thiol-PEG-Biotin (erste Anbindungsmoleküle R200 mit Biotin als funktioneller Gruppe zur indirekten Anbindung des Rezeptors R350 über zweite Anbindungsmoleküle R310)
- Neutravidin (an die Anbindungsmoleküle R200 bindende Moleküle R300, an die zweite Anbindungsmoleküle R310 gebunden werden)
- Biotin-Anti-IgG (zweite Anbindungsmoleküle R310).

An diese Anbindungsschichtabfolge schließt sich dann für den Nachweis eines BCV-Gehalts in einer Flüssigkeitsprobe die Schichtabfolge des aus dem Laborverfahren bekannten Affinitätsassays an:
- Antikörper gegen BCV vom Typ IgG (an die zweiten Anbindungsmoleküle gebundener Rezeptor R350)
- BCV (Analyt R400, wobei die Belegungsdichte von der Konzentration des Analyten in der Flüssigkeitsprobe abhängt)
- Zweiter Antikörper gegen BCV (zur indirekten Anbindung des Marker tragenden Moleküls R500)
- Dritter, HRP-markierter Antikörper (R500) gegen den zweiten Antikörper zum Chemolumineszenz-Nachweis mittels Luminol/H₂O₂.

In einem nachfolgenden Schritt müssen sämtliche verwendeten Komponenten auf Kreuzreaktivität überprüft werden (Schritt c), um eine Verfälschung der Messsignale hierdurch auszuschließen. Hierfür kann beispielsweise die Rezeptorschicht sukzessive unter Auslassung jeweils einer Schicht aufgebaut und das Messsignal bestimmt werden, das bei Auftreten einer Kreuzreaktivität einen Wert deutlich über Null beträgt. Weiterhin ist die Dichte der einzelnen Schichten, vorzugsweise der primären Anbindungsschicht, durch Co-Anbindung einer bezüglich der nachfolgenden Schicht nichtfunktionalisierten bzw. nicht reaktiven weiteren Komponente einzustellen.

Weist der automatisierte Bioanalysator ein mikrofluidisches Kanalsystem zum Flüssigkeitstransport mit einem Messkanal, in dem die Sensormatrix gebildet und der Analyt-Nachweis durchgeführt wird, auf, sind zudem die geeigneten Volumenströme, die Konzentrationen von zur Herstellung der Sensormatrix verwendeten Präparationslösungen mit den die Sensormatrix bildenden chemischen Spezies, und die Zeiträume, während derer die einzelnen Präparationslösungen und gegebenenfalls Spüllösungen durch den Messkanal geleitet werden, zu ermitteln (Schritt d). Hierbei werden vorzugsweise die Konzentrationen hoch, die Volumenströme der Präparationslösungen ebenfalls hoch und die Zeiträume lang gewählt. Hierdurch wird sichergestellt, dass die einzelnen Komponenten der Sensormatrix unter den gegebenen Bedingungen eine - entsprechend des chemischen Gleichgewichts bei dieser Konzentration - die jeweils darunterliegende Schicht nahezu vollständig bedeckende Schicht bilden. Dabei wird jeweils die zu untersuchende Komponente bezüglich ihrer Konzentration in der Präparationslösung, dem Volumenstrom und der Zeitspanne, während derer die Präparationslösung durch den Messkanal geleitet wird, variiert. Die unter den jeweils gewählten Bedingungen aufgebaute Sensormatrix wird mit einer Messlösung mit einer bekannten Analytkonzentration beaufschlagt und mittels des gewählten Nachweisverfahrens anhand des Signalwandlers des Bioanalysators ein Messsignal erfasst. Aus den so erhaltenen Messsignalen ergibt sich eine Matrix, aus der die jeweils optimalen Verfahrensparameter Konzentration, Volumenstrom und Zeitspanne des Durchleitens durch den Messkanal für jede Schicht der Sensormatrix ermittelt werden kann. So kann beispielsweise eine Kombination gewählt werden, die ein möglichst kurzes Durchleiten der Präparationslösung durch den Messkanal ermöglicht und trotzdem ein Messsignal von 95% des Messsignals bei einer sehr viel längeren Dauer des Durchleitens der Präparationslösung ermöglicht. Durch dieses systematische Vorgehen kann die Anpassung der genannten Verfahrensparameter auf ein mikrofluidisches Durchflusssystem in schneller Weise erfolgen.

In einem weiteren Schritt sind die Verfahrensparameter zur Durchführung der Freilegung des Substrats in analoger Weise zu bestimmen (Schritt e). Hierzu wird zunächst festgelegt, ob eine Reinigung durch chemische Mittel, beispielsweise durch Durchleiten einer Reinigungslösung durch den Messkanal zur im Wesentlichen vollständigen Freilegung des Substrats ausreicht, oder ob eine elektrochemische Reinigung durchgeführt werden soll, bei der neben dem Durchleiten der Reinigungslösung auch das Potential des Substrats bezüglich einer Gegenelektrode bzw. Referenzelektrode derart variiert wird, dass, ggfs. unterstützt durch Wasserstoff- bzw. Sauerstoffbildung durch Elektrolyse der Reinigungslösung, eine zumindest teilweise Zersetzung der Sensormatrix und gegebenenfalls daran gebundener Moleküle erfolgt. Mit einer Reihe von Versuchen können die Verfahrensparameter Konzentration/Zusammensetzung der Reinigungslösung, Potential des Substrats oder Stromstärke des zwischen Substrat und Gegenelektrode fließenden Stroms, Zeitspanne des Durchleitens der Reinigungslösung durch den Messkanal, gegebenenfalls Zeitspanne des Durchleitens von Hilfsflüssigkeiten durch den Messkanal, Verlauf des Substratpotentials bzw. des zwischen dem Substrat und der Gegenelektrode fließenden Stroms variiert werden und anhand eines Messsignals, das den Grad der Freilegung des Substrats repräsentiert, der Regenerierungserfolg bestimmt werden. Aus einer entsprechenden Matrix, in der der Reinigungserfolg bei verschiedenen Verfahrensparametern aufgetragen ist, können die optimalen Verfahrensparameter bestimmt werden.

Mit Proben aus dem entsprechenden Einsatzgebiet sind bei Bedarf Messungen durchzuführen, um gegebenenfalls Matrixeffekte, die sich aus dem verwendeten Messmedium ergeben, zu identifizieren (Schritt f). Sollten diese auftreten, können geeignete, im Einzelfall zu ermittelnde Gegenmaßnahmen wie beispielsweise Verdünnung der Probe oder Berücksichtigung der Messwertabweichungen bei der nachfolgenden Kalibrierung des Verfahrens, ergriffen werden. Nachdem der auf die automatisierte *in situ* Bestimmung mittels eines Bioanalysators übertragene Affinitätsassay kalibriert ist, erfolgt eine entsprechende Validierung (Schritt g). Dies schließt die Überprüfung auf Lagerstabilität der Präparationslösungen und sonstiger eingesetzter Reagenzien explizit mit ein.

Der Vorteil des voranstehend beschriebenen Verfahrens besteht darin, dass in effizienter Art und Weise im Laboralltag gebräuchliche, beispielsweise als Einmal-Laborverfahren durchgeführte Affinitätsassays für die Qualitätskontrolle, zum Beispiel in einer biotechnologischen Produktionsanlage, auf einen automatisierten Bioanalysator übertragen werden können. Somit ist es beispielsweise möglich, den Gehalt einer zu analysierenden Substanz, beispielsweise eines Proteins oder wie im obigen Beispiels, eines Impfstoffes, vollautomatisch, wiederholt und online zu analysieren. Die Verwendung einer oder weniger Standard-Schichtsysteme zur Anbindung einer Vielzahl verschiedener, nahezu beliebiger Rezeptoren und/oder die Verwendung eines oder weniger Marker oder Markermoleküle bieten eine Plattform-Lösung, auf die in der beschriebenen Weise bekannte Laborverfahren mit verhältnismäßig geringem Aufwand übertragen werden können. ist, erfolgt eine entsprechende Validierung (Schritt g). Dies schließt die Überprüfung auf Lagerstabilität der Präparationslösungen und sonstiger eingesetzter Reagenzien explizit mit ein.

Der Vorteil des voranstehend beschriebenen Verfahrens besteht darin, dass in effizienter Art und Weise im Laboralltag gebräuchliche, beispielsweise als Einmal-Laborverfahren durchgeführte Affinitätsassays für die Qualitätskontrolle, zum Beispiel in einer biotechnologischen Produktionsanlage, auf einen automatisierten Bioanalysator übertragen werden können. Somit ist es beispielsweise möglich, den Gehalt einer zu analysierenden Substanz, beispielsweise eines Proteins oder wie im obigen Beispiels, eines Impfstoffes, vollautomatisch, wiederholt und online zu analysieren. Die Verwendung einer oder weniger Standard-Schichtsysteme zur Anbindung einer Vielzahl verschiedener, nahezu beliebiger Rezeptoren und/oder die Verwendung eines oder weniger Marker oder Markermoleküle bieten eine Plattform-Lösung, auf die in der beschriebenen Weise bekannte Laborverfahren mit verhältnismäßig geringem Aufwand übertragen werden können.

## Patentansprüche

1. Verfahren zur automatisierten *in situ*-Bestimmung eines Analytgehalts einer Flüssigkeitsprobe mittels eines Bioanalysators, welcher eine Mikrofluidikeinrichtung mit mindestens einem Messkanal und Mittel zum Durchleiten von einer oder mehreren Flüssigkeiten durch den Messkanal umfasst, wobei der Messkanal mindestens ein elektrisch leitfähiges, Substrat aufweist,
umfassend die zur Durchführung einer Messung mindestens innerhalb eines Messbereichs wiederholt durchführbare Schrittabfolge:
(i) Präparieren einer Sensormatrix, welche eine Vielzahl von Rezeptoren aufweist, die den Analyten und/oder ein weiteres Zielmolekül, vorzugsweise selektiv und spezifisch, insbesondere aufgrund einer affinen Wechselwirkung, binden oder eine chemische Umsetzung des Analyten oder des weiteren Zielmoleküls bewirken,
umfassend einen Schritt des Durchleitens einer Präparationslösung von mindestens einer ersten chemischen Spezies, welche mindestens eine an das Substrat bindende und mindestens eine weitere funktionelle Gruppe umfasst, durch den Messkanal, wobei eine Vielzahl der ersten chemischen Spezies über die an das Substrat bindende funktionelle Gruppe an das Substrat gebunden werden,
wobei die weitere funktionelle Gruppe der an das Substrat gebundenen Vielzahl der ersten chemischen Spezies zur nachfolgenden Anbindung eines Rezeptors dient,
wobei die an das Substrat gebundene Vielzahl von ersten chemischen Spezies eine erste Anbindungsschicht bilden, und die weiteren funktionellen Gruppen der ersten chemischen Spezies Bindungsstellen bilden, an die nachfolgend die Rezeptoren durch spezifische Anbindung, insbesondere über mindestens eine nicht-kovalente Bindung, an die weiteren funktionellen Gruppen direkt oder über eine oder mehrere weitere Anbindungsschichten angebunden werden, so dass die Sensormatrix aus einer oder mehreren Anbindungsschichten und einer abschließenden, an der obersten Anbindungsschicht gebundenen Rezeptorschicht gebildet wird;
(ii) Durchleiten der Flüssigkeitsprobe oder einer durch Behandeln der Flüssigkeitsprobe mit mindestens einem Reagenz erhaltenen Messflüssigkeit durch den Messkanal, wobei in der Flüssigkeitsprobe oder der Messflüssigkeit enthaltener Analyt und/oder weitere, in der Flüssigkeitsprobe oder der Messflüssigkeit enthaltene Zielmoleküle, vorzugsweise selektiv und spezifisch, an die Rezeptoren binden oder von diesen chemisch umgesetzt werden,
und Bestimmen einer mit der Menge der von den Rezeptoren gebundenen oder umgesetzten Zielmoleküle oder der Menge des von den Rezeptoren gebundenen oder umgesetzten Analyten korrelierenden Messgröße und daraus Ableiten des Analytgehalts der Flüssigkeitsprobe;
(iii) Freilegen des mindestens einen Substrats, wobei die Sensormatrix und gegebenenfalls daran gebundene Moleküle, insbesondere Analytmoleküle, weitere Zielmoleküle oder sonstige Moleküle, von dem Substrat abgelöst und zumindest teilweise zersetzt werden,
wobei das Freilegen des Substrats durch kontinuierliches Durchleiten eines Elektrolyten durch den Messkanal und Erzeugen eines elektrischen Stromflusses zwischen dem Substrat und einer über den Elektrolyten mit dem Substrat in elektrisch leitfähigem Kontakt stehenden Gegenelektrode erfolgt um das Substrat wieder freizulegen,
wobei das Potential des Substrats während des Durchleitens des Elektrolyten zwischen negativen Werten im Bereich zwischen -0,5 V und -1,25 V und positiven Werten im Bereich zwischen +1,5 V und +2,25 V gegenüber einer Ag/AgCl-Referenzelektrode variiert wird, so dass die Sensormatrix abwechselnd oxidativen und reduktiven Bedingungen und entstehendem Wasserstoff bzw. Sauerstoff ausgesetzt ist.

2. Verfahren nach Anspruch 1,
wobei die an das Substrat gebundene Vielzahl von ersten chemischen Spezies eine erste Anbindungsschicht bildet, und die weiteren funktionellen Gruppen der ersten chemischen Spezies Bindungsstellen zur nachfolgenden Anbindung einer oder mehrerer weiterer Anbindungsschichten bilden, wobei eine oberste Anbindungsschicht eine Vielzahl von Bindungsstellen zur nachfolgenden Anbindung der Rezeptoren zur Verfügung stellt.

3. Verfahren nach Anspruch 1 oder 2,
wobei die zur nachfolgenden Anbindung der Rezeptoren zur Verfügung gestellten Bindungsstellen ausgewählt sind aus einer Gruppe umfassend wenige, jeweils zur Anbindung einer Vielzahl unterschiedlicher Rezeptoren geeignete Bindungsstellen, insbesondere Bindungsstellen zur Anbindung von Biotin und/oder, insbesondere durch Anbindung eines biotinylierten, Immunglobulin-bindenden Moleküls an eine Bindungsstelle zur Anbindung von Biotin zur Verfügung gestellte, Immunglobulin-bindende Bindungsstellen.

4. Verfahren nach einem der Ansprüche 1 bis 3,
wobei die mit der Menge der von den Rezeptoren gebundenen oder umgesetzten Zielmoleküle oder der Menge des von den Rezeptoren gebundenen oder umgesetzten Analyten korrelierenden Messgröße eine optische Messgröße ist, welche insbesondere anhand einer Lumineszenzmessung, einer Reflexionsmessung oder einer Absorptionsmessung ermittelt wird.

5. Verfahren nach einem der Ansprüche 1 bis 4,
wobei der Analyt oder das weitere Zielmolekül direkt oder indirekt mit einem, insbesondere aus einer Gruppe umfassend Enzyme, Fluorophore und metallische oder halbleitende Nanopartikel ausgewählter, Marker markiert ist oder markiert wird, welcher zur Einbringung einer mit der Menge der von den Rezeptoren gebundenen oder umgesetzten Zielmoleküle oder der Menge des von den Rezeptoren gebundenen oder umgesetzten Analyten korrelierenden Messgröße beeinflussenden Eigenschaft dient.

6. Verfahren nach Anspruch 5,
wobei der Marker über mindestens eine affine Wechselwirkung, vorzugsweise mittels eines Antikörpers und/oder eines Protein-Tags, direkt oder indirekt an das zu markierende Zielmolekül, insbesondere einen Kompetitor, angebunden ist oder nach der Anbindung des zu markierenden Zielmoleküls an die Sensormatrix an dieses angebunden wird.

7. Verfahren nach Anspruch 6,
wobei der Marker über die mindestens eine affine Wechselwirkung mittels eines, insbesondere aus einer Gruppe umfassend hochaffine Protein-Tags, insbesondere den HA-Tag, den His-Tag, und fluoreszierende Proteine, insbesondere GFP, ausgewählten, Protein-Tags, direkt oder indirekt an das zu markierende Zielmolekül angebunden ist oder nach der Anbindung des zu markierenden Zielmoleküls an die Sensormatrix an dieses angebunden wird, wobei der Protein-Tag ausgewählt ist aus einer Gruppe umfassend wenige, über die jeweils gleiche affine Wechselwirkung (d.h. über die gleiche Schnittstelle) direkt oder indirekt markierbare Tags.

8. Verfahren nach Anspruch 7,
wobei der Tag und/oder das zu markierende weitere Zielmolekül ein rekombinant hergestelltes Protein oder Peptid ist.

9. Verfahren nach einem der Ansprüche 1 bis 8,
wobei es sich bei dem mindestens einen Substrat um ein elektrisch leitfähiges Substrat handelt, und wobei das Freilegen, des Substrats durch Erzeugen eines elektrischen Stromflusses zwischen dem Substrat und einer über einen Elektrolyten mit dem Substrat in elektrisch leitfähigem Kontakt stehenden Gegenelektrode erfolgt, um das Substrat, insbesondere im Wesentlichen unabhängig von der vorhandenen Belegung des Substrats, wieder freizulegen.

10. Verfahren nach Anspruch 9,
wobei die Gegenelektrode als weiteres Substrat ausgestaltet ist, an das die an das Substrat bindende funktionelle Gruppe der ersten chemischen Spezies bindet, im Messkanal, insbesondere dem Substrat gegenüberliegend oder koaxial zum Substrat ausgerichtet, angeordnet ist.

11. Verfahren nach einem der Ansprüche 1 bis 10,
wobei es sich bei dem mindestens einen Substrat um ein thiophiles, elektrisch leitfähiges Substrat und bei der an das Substrat bindenden funktionellen Gruppe der mindestens einen ersten chemischen Spezies um eine Thiol- oder Disulfidgruppe handelt, wobei durch Bindung der Schwefelatome der Thiol- oder Disulfidgruppen an das Substrat eine Schicht der mindestens einen ersten chemischen Spezies gebildet wird.

12. Verwendung eines Bioanalysators in dem Verfahren nach einem der Ansprüche 1 bis 11, umfassend eine Mikrofluidikeinrichtung mit mindestens einem Messkanal und Mittel zum Durchleiten von Flüssigkeit durch den Messkanal,
wobei der Messkanal mindestens ein Substrat umfasst, auf dem mindestens innerhalb eines Messbereichs mindestens zeitweise eine Sensormatrix mit einer Vielzahl von Rezeptoren angeordnet ist, welche in einer Flüssigkeitsprobe oder einer durch Behandeln der Flüssigkeitsprobe mit mindestens einem Reagenz erhaltenen Messflüssigkeit vorhandene Zielmoleküle oder Analyte vorzugsweise selektiv und spezifisch binden oder chemisch umsetzen,
wobei die Sensormatrix gebildet ist durch eine oder mehrere an das Substrat angebundene Anbindungsschichten und eine abschließende, an der obersten Anbindungsschicht gebundene Rezeptorschicht, wobei eine erste Anbindungsschicht durch eine Vielzahl von eine erste, an das Substrat gebundene funktionelle Gruppe aufweisenden ersten chemischen Spezies gebildet ist, und wobei die ersten chemischen Spezies eine weitere funktionelle Gruppe aufweisen, an welche die Rezeptoren durch spezifische Anbindung direkt oder indirekt über eine oder mehrere weitere Anbindungsschichten angebunden sind, so dass die Sensormatrix aus einer oder mehreren Anbindungsschichten und einer abschließenden, an der obersten Anbindungsschicht gebundenen Rezeptorschicht gebildet wird; wobei der Bioanalysator mindestens einen Signalwandler umfasst, welcher ein von einer Menge von an die Rezeptoren gebundenem oder von diesen umgesetztem Analyten oder ein von einer Menge von an die Rezeptoren gebundenen oder umgesetzten Zielmolekülen abhängiges Messsignal ausgibt, und
wobei das Substrat elektrisch leitfähig ist und innerhalb der Mikrofluidikeinrichtung eine zumindest während des Freilegens mit dem Substrat in leitendem Kontakt stehende, insbesondere innerhalb des Messkanals dem ersten Substrat gegenüberliegend oder koaxial zum Substrat ausgerichtet angeordnete und bevorzugt als weiteres Substrat dienende, Gegenelektrode angeordnet ist, wobei der Bioanalysator weiterhin Mittel zum Erzeugen eines Stromflusses zwischen dem Substrat und der Gegenelektrode umfasst, und wobei der Bioanalysator weiterhin eine, insbesondere innerhalb der Mikrofluidikeinrichtung gebildete Referenzelektrode umfasst, welche über einen, insbesondere diaphragmenlosen, Stromschlüssel, zumindest während des Freilegens des Substrats in elektrisch leitender Verbindung mit dem mindestens einen Substrat steht.

13. Verfahren zur Anpassung eines einmal verwendbaren Laborverfahrens zur Bestimmung eines Analytgehalts einer Flüssigkeitsprobe mittels eines Affinitäts-Bioassays zur, insbesondere vielfach wiederholten, Anwendung in einem Bioanalysator nach dem Verfahren gemäß einem der Ansprüche 1 bis 11, umfassend die Schritte:
(a) Wahl einer geeigneten Anbindungsstruktur zum Aufbau einer oder mehrerer Anbindungsschichten zur Anbindung eines Rezeptors des Affinitäts-Bioassays, und Einstellung einer Dichte der Anbindungsstruktur;
(b) optional Auswahl eines alternativen Bestimmungsverfahrens und/oder Bereitstellung eines alternativen weiteren Zielmoleküls und/oder eines Verfahrens zur direkten oder indirekten Einführung einer Markierung;
(c) Überprüfung aller Komponenten des vorzugsweise vielfach verwendbaren Affinitäts-Bioassays auf gegenseitige Kreuzreaktivität;
(d) Ermittlung geeigneter Verfahrensparameter des Bioanalysators, insbesondere von Volumenströmen, Konzentrationen und Zeitspannen der Durchleitung von Flüssigkeiten zur Durchführung einer Messung mit dem vielfach verwendbaren Affinitäts-Bioassay;
(e) Überprüfen und gegebenenfalls Einstellen der Verfahrensparameter des Bioanalysators zur Durchführung einer Freilegung des Substrats;
(f) optional Überprüfung auf Matrixeffekte der Flüssigkeitsprobe oder der Messflüssigkeit;
(g) Erstellung einer Kalibrierkurve und Validierung des Verfahrens.

14. Verfahren nach Anspruch 13,
wobei der Affinitäts-Bioassay nicht zur Bestimmung von Desoxyribonucleinsäure (DNA) und Ribonucleinsäure (RNA) dient.

## Claims

1. Method for the automated *in situ* determination of an analyte content of a liquid sample using a bioanalyzer comprising a microfluidic unit with at least one measuring channel and means for conducting one or more liquids through the measuring channel,
wherein the measuring channel has at least one electrically conductive substrate,
said method comprising the following sequence of steps, which can be performed repeatedly, to measure at least within a measuring region:
(i) preparation of a sensor matrix, which has a plurality of receptors that bind the analyte and/or an additional target molecule, preferably selectively and specifically, particularly due to an affinity interaction, or bring about the chemical conversion of the analyte or the additional target molecule, comprising a step wherein a preparation solution of at least a first chemical species, which comprises at least one functional group binding to the substrate and at least one other functional group, is conducted through the measuring channel, wherein a plurality of the first chemical species is bound to the substrate via the functional group binding to the substrate,
wherein the additional functional group of the plurality of the first chemical species bound to the substrate is used for the subsequent binding of a receptor,
wherein the plurality of first chemical species bound to the substrate forms a first binding layer, and the other functional groups of the first chemical species form binding sites where the receptors are subsequently bound, by specific binding, particularly via at least one non-covalent bond, to the other functional groups directly or via one or more additional binding layers, such that the sensor matrix is made from one or more binding layers and a final receptor layer, which is bound to the top binding layer;
(ii) the conduction of the liquid sample, or a liquid under measurement obtained by treating the liquid sample with at least one reagent, through the measuring channel, wherein analyte contained in the liquid sample or in the liquid under measurement, and/or other target molecules contained in the liquid sample or in the liquid under measurement, bind to the receptors, preferably selectively and specifically, or are converted chemically by said receptors,
and the determination of a measured variable, which correlates to the amount of the target molecules bound or converted by the receptors, or the amount of analyte bound or converted by the receptors, and derivation of the analyte content of the liquid sample on the basis of said measured variable;
(iii) clearing of the at least one substrate, wherein the sensor matrix and, where applicable, molecules bound thereto, particularly analyte molecules, additional target molecules or other molecules, are released from the substrate and at least partially decomposed,
wherein the substrate is cleared by continuously passing an electrolyte through the measuring channel and generating an electrical flow of current between the substrate and a counter-electrode which is in contact with the substrate in an electrically conductive manner via the electrolyte in order to clear the substrate,
wherein, while the electrolyte is passed through the measuring channel, the potential of the substrate varies between negative values in the range between -0.5 V and -1.25 V and positive values in the range between +1.5 V and +2.25 V in relation to an Ag/AgCI reference electrode such that the sensor matrix is alternately exposed to oxidative and reductive conditions and resulting hydrogen or oxygen.

2. Method as claimed in Claim 1,
wherein the plurality of first chemical species bonded to the substrate forms a first binding layer, and the other functional groups of the first chemical species form binding sites for the subsequent binding of one or more additional binding layers, wherein a top binding layer makes a multitude of binding sites available for the subsequent binding of receptors.

3. Method as claimed in Claim 1 or 2,
wherein the binding sites made available for the subsequent binding of receptors are selected from a group comprising a few binding sites that are suitable for binding a multitude of different receptors, particularly binding sites for the binding of biotin and/or immunoglobulin-binding binding sites that are made available to bind biotin particularly by binding a biotinylated immunoglobulin-binding molecule to a binding site.

4. Method as claimed in one of the Claims 1 to 3,
wherein the measured variable that correlates to the amount of target molecules bound or converted by the receptors or the amount of the analyte bound or converted by the receptors is an optical measured variable, which is determined, in particular, by means of a luminescence measurement, a reflection measurement or an absorption measurement.

5. Method as claimed in one of the Claims 1 to 4,
wherein the analyte or the additional target molecule is labeled directly or indirectly with a marker, particularly a marker selected from a group comprising enzymes, fluorophores and metallic or semi-conductive nanoparticles, said marker being used to introduce a property that influences a measured variable that correlates to the amount of target molecules bound or converted by the receptors or to the amount of analyte bound or converted by the receptors.

6. Method as claimed in Claim 5
wherein, via at least one affinity interaction, the marker is directly or indirectly bound to the target molecule to be labeled, particularly a competitor, preferably by means of an antibody and/or a protein tag, or is bound to the target molecule to be labeled after said molecule is bound to the sensor matrix.

7. Method as claimed in Claim 6,
wherein, via the at least one affinity interaction, the marker is bound either directly or indirectly to the target molecule to be labeled by means of a protein tag, particularly one selected from a group comprising high-affinity protein tags, particularly the HA tag, the His tag and fluorescent proteins, particularly GFP, or is bound to the target molecule to be labeled after said molecule is bound to the sensor matrix, wherein the protein tag is selected from a group comprising a few tags that can be labeled directly or indirectly by means of the same affinity interaction (i.e. via the same interface).

8. Method as claimed in Claim 7,
wherein the tag and/or the additional target molecule to be labeled is a recombinant protein or peptide.

9. Method as claimed in one of the Claims 1 to 8,
wherein the at least one substrate is an electrically conductive substrate, and wherein the clearing of the substrate takes place by generating a flow of electricity between the substrate and a counter-electrode in contact with the substrate in an electrically conductive manner by means of an electrolyte in order to clear the substrate, particularly independently, for the most part, of the extent to which the substrate is currently covered.

10. Method as claimed in Claim 9,
wherein the counter-electrode is designed as an additional substrate to which the functional group of the first chemical species binding to the substrate is bound, and is arranged in the measuring channel, particularly such that it is aligned opposite to the substrate or in a coaxial manner in relation to the substrate.

11. Method as claimed in one of the Claims 1 to 10,
wherein the at least one substrate is a thiophilic, electrically conductive substrate and wherein the functional group of the at least one first chemical species binding to the substrate is a thiol or disulfide group, wherein a layer of the at least one first chemical species is formed by the binding of the sulfur atoms of the thiol or disulfide groups to the substrate.

12. Use of a bioanalyzer in the method as claimed in one of the Claims 1 to 11, comprising a microfluidic unit with at least one measuring channel and means to conduct liquid through the measuring channel,
wherein the measuring channel comprises at least one substrate on which a sensor matrix with a plurality of receptors is at least temporarily arranged at least within a measuring region, wherein said receptors preferably selectively and specifically bind or chemically convert target molecules or analyte present in a liquid sample or a liquid under measurement which is obtained by treating the liquid sample with at least one reagent,
wherein the sensor matrix is formed by one or more binding layers bound to the substrate and a final receptor layer bound to the top binding layer, wherein a first binding layer is formed by a plurality of first chemical species having a first functional group bound to the substrate, and
wherein the first chemical species have an additional functional group to which the receptors are bound, directly or indirectly, by specific binding via one or more additional binding layers such that the sensor matrix is formed from one or more binding layers and a final receptor layer bound to the top binding layer;
wherein the bioanalyzer comprises at least one signal converter which provides a measuring signal that depends on an amount of analyte bound to the receptors or converted by said receptors, or on an amount of target molecules bound to or converted by the receptors, and
wherein the substrate is electrically conductive and a counter-electrode is arranged in the microfluidic unit, said counter-electrode being in contact in a conductive manner with the substrate at least during the clearing of the substrate, and particularly being arranged opposite to the first substrate or coaxially in relation to the substrate inside the measuring channel, and preferably acting as an additional substrate,
wherein the bioanalyzer further comprises means to generate a flow of current between the substrate and the counter-electrode, and wherein the bioanalyzer further comprises a reference electrode, particularly formed within the microfluidic unit, which has an electrically conductive connection to the at least one substrate at least during the clearing of the substrate via a salt bridge, particularly a diaphragm-free salt bridge.

13. Method to adapt a single-use laboratory method for determining an analyte content of a liquid sample using an affinity bioassay for use, particularly repeated multi-use, in a bioanalyzer according to the method as claimed in one of the Claims 1 to 11, comprising the following steps:
(a) selection of a suitable binding structure to produce one or more binding layers to bind a receptor of the affinity bioassay, and setting of a density of the binding structure;
(b) optionally, selection of an alternative determination method and/or provision of an alternative additional target molecule and/or a method for the direct or indirect introduction of a labeling;
(c) checking of all the components of the preferably multi-use affinity bioassay for mutual cross-reactivity;
(d) determination of suitable process parameters of the bioanalyzer, particularly volume flows, concentrations and intervals for the conduction of liquids to perform a measurement with the multi-use affinity bioassay;
(e) checking and where applicable setting of the process parameters of the bioanalyzer to perform the clearing of the substrate;
(f) optionally, checking for matrix effects of the liquid sample or the liquid under measurement;
(g) creation of a calibration curve and validation of the method.

14. Method as claimed in Claim 13,
wherein the affinity bioassay is not intended for the determination of deoxyribonucleic acid (DNA) and ribonucleic acid (RNA).

## Revendications

1. Procédé destiné à la détermination *in situ* automatisée d'une teneur en analyte dans un échantillon de liquide au moyen d'un bioanalyseur, lequel comprend un dispositif microfluidique avec au moins un canal de mesure et des moyens destinés à l'acheminement d'un ou de plusieurs liquides à travers le canal de mesure, le canal de mesure présentant au moins un substrat électriquement conducteur,
lequel procédé comprend une séquence d'étapes pouvant être exécutée de façon répétitive et destinée à la réalisation d'une mesure au moins à l'intérieur d'une gamme de mesure :
(i) Préparation d'une matrice de capteurs, qui comporte un grand nombre de récepteurs liant l'analyte et/ou une autre molécule cible, de préférence de façon sélective et spécifique, notamment en raison d'une interaction affine, ou provoquant une transformation chimique de l'analyte ou d'une autre molécule cible,
comprenant une étape d'acheminement d'une solution de préparation d'au moins une première espèce chimique, laquelle comprend au moins un groupe fonctionnel lié au substrat et au moins un autre groupe fonctionnel, à travers le canal de mesure, un grand nombre de premières espèces chimiques étant liées au substrat via le groupe fonctionnel lié au substrat,
l'autre groupe fonctionnel du grand nombre de premières espèces liées au substrat servant à la liaison subséquente d'un récepteur ;
le grand nombre de premières espèces chimiques liées au substrat formant une première couche de liaison, et les autres groupes fonctionnels des premières espèces chimiques formant des sites de liaison, auxquels les récepteurs sont liés ensuite par une liaison spécifique, notamment au moyen d'au moins une liaison non covalente, aux autres groupes fonctionnels, directement ou via une ou plusieurs couches de liaison supplémentaires, si bien que la matrice de capteurs est formée d'une ou de plusieurs couches de liaison et d'une couche de récepteurs finale, liée à la couche de liaison supérieure ;
(ii) Acheminement de l'échantillon de liquide ou d'un liquide de mesure obtenu par traitement de l'échantillon de liquide avec au moins un réactif, à travers le canal de mesure, l'analyte contenu dans l'échantillon de liquide ou le liquide de mesure et/ou d'autres molécules cibles contenues dans l'échantillon de liquide ou le liquide de mesure se liant, de préférence de façon sélective et spécifique, aux récepteurs ou étant convertis chimiquement par ces derniers,
et détermination d'une grandeur de mesure corrélée avec la quantité de molécules cibles liées ou converties par les récepteurs ou avec la quantité d'analytes liés ou convertis par les récepteurs, et déduction de la teneur en analytes dans l'échantillon de liquide ;
(iii) Nettoyage de l'au moins un substrat, la matrice de capteurs et les molécules qui y sont éventuellement fixées, notamment des molécules d'analyte, des molécules cibles supplémentaires ou d'autres molécules étant séparées du substrat et au moins partiellement décomposées,
le nettoyage du substrat étant obtenu par acheminement continu d'un électrolyte à travers le canal de mesure et génération d'un flux de courant électrique entre le substrat et une contre-électrode en contact électriquement conducteur avec le substrat via l'électrolyte, afin de nettoyer de nouveau le substrat,
le potentiel du substrat étant varié pendant l'acheminement de l'électrolyte entre des valeurs négatives, dans une plage comprise entre -0,5 V et -1,25 V, et des valeurs positives, dans une plage comprise entre +1,5 V et +2,25 V par rapport à une électrode de référence Ag/AgCl, de telle sorte que la matrice de capteurs est exposée tour à tour à des conditions oxydatives et réductrices et à de l'hydrogène ou de l'oxygène produit.

2. Procédé selon la revendication 1,
pour lequel le grand nombre de premières espèces chimiques liées au substrat forment une première couche de liaison, et les autres groupes fonctionnels des premières espèces chimiques forment des sites de liaison pour la liaison subséquente d'une ou de plusieurs couches de liaison, la couche de liaison supérieure mettant à disposition un grand nombre de sites de liaison pour la liaison subséquente des récepteurs.

3. Procédé selon l'une des revendications 1 ou 2,
pour lequel les sites de liaison mis à disposition pour la liaison subséquente des récepteurs sont sélectionnés à partir d'un groupe comprenant un faible nombre de sites appropriés pour la liaison d'un grand nombre de récepteurs différents, notamment des sites pour la liaison de biotine et/ou, notamment par la liaison à un site de liaison d'une molécule biotinylée se liant aux immunoglobulines, des sites de liaison se liant aux immunoglobulines mis à disposition pour la liaison de biotine.

4. Procédé selon l'une des revendications 1 à 3,
pour lequel la grandeur de mesure corrélée avec la quantité de molécules cibles liée ou convertie par les récepteurs ou avec la quantité d'analyte liée ou convertie par les récepteurs est une grande de mesure optique, laquelle est déterminée notamment au moyen d'une mesure de luminescence, une mesure de réflexion ou une mesure d'adsorption.

5. Procédé selon l'une des revendications 1 à 4,
pour lequel l'analyte ou la molécule cible supplémentaire est marqué directement ou indirectement avec un marqueur sélectionné notamment à partir d'un groupe comprenant des enzymes, des fluorophores et des nanoparticules métalliques ou semi-conductrices, lequel marqueur sert à l'introduction d'une propriété influençant la grandeur de mesure corrélée avec la quantité de molécules cibles liée ou convertie par les récepteurs ou avec la quantité d'analyte liée ou convertie par les récepteurs.

6. Procédé selon la revendication 5,
pour lequel le marqueur est lié au moins par une interaction affine, de préférence au moyen d'un anticorps et/ou d'une étiquette de protéine, directement ou indirectement à la molécule cible à marquer, notamment un compétiteur, et lié à celle-ci après la liaison de la molécule cible à marquer à la matrice de capteurs.

7. Procédé selon la revendication 6,
pour lequel le marqueur est lié, au moins par une interaction affine au moyen d'une étiquette de protéine, notamment sélectionné à partir d'un groupe comprenant des étiquettes de protéine à haute affinité, notamment l'étiquette HA, l'étiquette His et des protéines fluorescentes, notamment des GFP, directement ou indirectement à la molécule cible à marquer ou, après la liaison à la matrice de capteurs de la molécule cible à marquer, est lié à celle-ci, l'étiquette de protéine étant sélectionnée à partir d'un groupe comprenant un faible nombre d'étiquettes marquables directement ou indirectement par la même interaction affine (c'est-à-dire à travers la même interface).

8. Procédé selon la revendication 7,
pour lequel l'étiquette et/ou la molécule cible supplémentaire à marquer est une protéine ou un peptide produit de manière recombinante.

9. Procédé selon l'une des revendications 1 à 8,
pour lequel s'agit, concernant le substrat, d'un substrat électriquement conducteur, et pour lequel le nettoyage du substrat est réalisé par la génération d'un flux de courant électrique entre le substrat et une contre-électrode en contact électriquement conducteur avec le substrat via un électrolyte, afin de nettoyer de nouveau le substrat, notamment pour l'essentiel indépendamment du degré de recouvrement du substrat.

10. Procédé selon la revendication 9,
pour lequel la contre-électrode est conçue en tant que substrat supplémentaire dans le canal de mesure, notamment située à l'opposé du substrat ou alignée de façon coaxiale par rapport au substrat, auquel substrat supplémentaire est lié le groupe fonctionnel de la première espèce chimique, lié au substrat

11. Procédé selon l'une des revendications 1 à 10,
pour lequel, concernant l'au moins un substrat, il s'agit d'un substrat thiophile électriquement conducteur et, concernant le groupe fonctionnel de l'au moins une première espèce chimique, lié au substrat, il s'agit d'un groupe thiol ou bisulfure, une couche de l'au moins une première espèce chimique étant formée par la liaison des atomes de soufre des groupes thiol ou bisulfure avec le substrat.

12. Utilisation d'un bioanalyseur avec un procédé selon l'une des revendications 1 à 11, comprenant un dispositif microfluidique avec au moins un canal de mesure, ainsi que des moyens destinés à l'acheminement du liquide à travers le canal de mesure,
le canal de mesure comprenant au moins un substrat, sur lequel est disposée au moins à l'intérieur d'une gamme de mesure, au moins temporairement, une matrice de capteurs avec un grand nombre de récepteurs, qui lient de préférence de façon sélective et spécifique, ou convertissent chimiquement, les molécules cibles ou les analytes présents dans un échantillon de liquide ou un liquide de mesure obtenu par traitement de l'échantillon de liquide au moyen d'au moins un réactif,
la matrice de capteurs étant formée d'une ou de plusieurs couches de liaison liées au substrat et d'une couche de récepteurs finale, liée à la couche de liaison supérieure, une première couche de liaison étant formée par un grand nombre de première espèces chimiques présentant un premier groupe fonctionnel lié au substrat, et les premières espèces chimiques présentant un groupe fonctionnel supplémentaire, auquel les récepteurs sont liés par une liaison spécifique, directement ou indirectement par le biais d'une ou de plusieurs couches de liaison, si bien que la matrice de capteurs est formée d'une ou de plusieurs couches de liaison et d'une couche de récepteurs finale, liée à la couche de liaison supérieure,
pour lequel le bioanalyseur comprend au moins un convertisseur de signal, lequel émet un signal de mesure dépendant de la quantité d'analytes liée aux récepteurs ou convertie par ces derniers, ou un signal de mesure dépendant de la quantité de molécules cibles liée aux récepteurs ou convertie par ces derniers, et
pour lequel le substrat est électriquement conducteur et une contre-électrode est disposée à l'intérieur du dispositif microfluidique, notamment dans le canal de mesure, située à l'opposé du substrat ou alignée de façon coaxiale par rapport au substrat, au moins pendant le nettoyage, laquelle contre-électrode est en contact conducteur avec le substrat et laquelle contre-électrode sert de préférence de substrat supplémentaire,
pour lequel le bioanalyseur comprend en outre des moyens destinés à la génération d'un flux de courant entre le substrat et la contre-électrode, et pour lequel le bioanalyseur comprend en outre une électrode de référence notamment formée à l'intérieur du dispositif microfluidique, laquelle électrode est en liaison électriquement conductrice avec l'au moins un substrat par l'intermédiaire d'un pont électrolytique, notamment sans diaphragme, au moins pendant le nettoyage du substrat.

13. Procédé destiné à l'adaptation d'un procédé de laboratoire à usage unique, destiné à la détermination d'une teneur en analyte dans un échantillon de liquide au moyen d'un bioessai d'affinité pour l'utilisation, notamment maintes fois répétée, dans un bioanalyseur d'après le procédé selon l'une des revendications 1 à 11, comprenant les étapes suivantes :
(a) Sélection d'une structure de liaison appropriée destinée à la réalisation d'une ou de plusieurs couches destinées à la liaison d'un récepteur du bioessai d'affinité, et le réglage d'une densité de la structure de liaison ;
(b) Sélection optionnelle d'un procédé de détermination alternatif et/ou mise à disposition d'une molécule cible supplémentaire alternative et/ou d'un procédé destiné à l'introduction directe ou indirecte d'un marquage ;
(c) Vérification de l'ensemble des composants du bioessai d'affinité, de préférence à usage multiple, par rapport à une réaction croisée ;
(d) Détermination de paramètres de procédé appropriés du bioanalyseur, notamment de débits volumiques, concentrations et périodes de temps de l'acheminement de liquide en vue de l'exécution d'une mesure à l'aide du bioessai d'affinité à usage multiple ;
(e) Vérification et, le cas échéant, réglage des paramètres de procédé du bioanalyseur en vue de la réalisation d'un nettoyage du substrat ;
(f) Vérification optionnelle par rapport aux effets de matrice de l'échantillon de liquide ou du liquide de mesure ;
(g) Création d'une courbe d'étalonnage et validation du procédé.

14. Procédé selon la revendication 13,
pour lequel le bioessai d'affinité ne sert pas à la détermination d'acide désoxyribonucléique (DNA) et d'acide ribonucléique (RNA).
